# EUROPEAN PATENT APPLICATION

(11) **EP 3 647 788 A1**
(43) Date of publication of application: **06.05.2020**
(21) Application number: 18825005.4
(22) Date of filing: 29.06.2018
(51) Int. Cl.: G01N 33/68, C07K 14/47, C07K 14/705, C12N 9/64, G01N 33/53, G01N 33/574

(54) **BIOMARKER FOR DETECTING COLORECTAL CANCER**

(30) Priority: 30.06.2017 JP 2017129941
(71) Applicant: National Institutes of Biomedical Innovation, Health and Nutrition, Ibaraki-shi, Osaka 567-0085 (JP); Denka Seiken Co., Ltd., Chuo-ku Tokyo 103-8338 (JP)
(72) Inventor: TOMONAGA, Takeshi, Ibaraki-shi Osaka 567-0085 (JP); SHIROMIZU, Takashi, Ibaraki-shi Osaka 567-0085 (JP)
(74) Representative: Wohlfahrt, Jan Günther
(86) International application number: PCT/JP2018/024806
(87) International publication number: WO 2019/004430

(57) **Abstract**

Provided is a biomarker for detecting colorectal cancer in an early stage. A colorectal cancer biomarker for detecting colorectal cancer, wherein the biomarker consists of at least one protein of the following 22 proteins with numbers 1 to 22, or at least one peptide of the partial peptides of the proteins with numbers 1 to 22: 1. Annexin A11; 2. Annexin A3; 3. Annexin A4; 4. Tanascin-N; 5. Transferrin receptor protein 1; 6. Glucose transporter 1; 7. Complement component C9; 8. CD88 antigen; 9. 78 kDa glucose-regulated protein; 10. α-1-acid glycoprotein; 11. Matrix metalloproteinase-9; 12. Angiopoietin-1; 13. CD67 antigen; 14. Mucin-5B; 15. Adapter protein GRB2; 16. Annexin A5; 17. Olfactomedin-4; 18. Neutral amino acid transporter B(0); 19. Tripeptidyl-peptidase 1; 20. Heat shock-related 70 kDa protein 2; 21. Proteasome subunit α type-5; or 22. Neutrophil gelatinase-associated lipocalin.

## Description

### Technical Field

The present invention relates to a novel protein or peptide biomarker that can be used in detection of colorectal cancer.

### Background Art

Colorectal cancer (CRC) is one of the most common cancers in the world, and thus, the development of a biomarker effective for CRC is essential for the improvement of human survival rate. However, the currently used methods for diagnosing colorectal cancer have involved biomarkers with extremely low accuracy, such as faecal occult blood or CEA, and novel biomarkers capable of diagnosing colorectal cancer in an early stage with high accuracy have not been discovered. Hence, it has been desired to develop a novel blood biomarker.

As a result of large-scale omics-based studies, a large number of cancer-related factors and cancer biomarker candidates have been discovered. Nevertheless, clinically applied blood biomarkers have not yet been present. As evidence to support this fact, the number of biomarkers recently approved by United States Food and Drug Administration (FDA) is extremely low (2 biomarkers or less every year). One of the causes of this stagnation is considered to be a serious problem in the strategy of biomarker development.

Conventionally, in many clinical tests, detection of a protein biomarker has been carried out according to antibody-based quantitative analyses (in which the enzyme-linked immunosorbent assay (ELISA) has been mainly applied). These methods largely depend on the quality of an antibody, and thus, the methods are problematic in terms of accuracy, in particular, specificity. Moreover, according to these methods, it is difficult to simultaneously evaluate marker candidates for multiple items.

As a result of recent progression of mass spectrometers (MS), it has become possible that clinical test methods will largely change. As a reason therefor, a selected reaction monitoring/multiple reaction monitoring method (SRM/MRM), which is a representative target proteomics method, cannot only quantify a biomarker protein with high accuracy, but also, can simultaneously quantify markers for multiple items. According to the previous report, Whiteaker et al. had quantified breast cancer biomarker candidate proteins for multiple items present in the plasma of a patient according to the SRM/MRM method (see Non Patent Literature 1). Other than this, several cases of examining various cancer biomarker candidate proteins according to the SRM/MRM method, including the report by the present inventors' group, have been reported (see Non Patent Literatures 2 to 4).

Thus, the target proteomics using the SRM/MRM method could be a strong tool for discovering biomarkers.

### Citation List

### Non Patent Literature

Non Patent Literature 1: Whiteaker, J. et al., Nat. BIotechnol. 29, 625-634 (2011)
Non Patent Literature 2: Kume, H. et al., Mol. Cell. Proteomics 13, 1471-1484 (2014)
Non Patent Literature 3: Muraoka, S. et al., J. Proteome Res. 11, 4201-4210 (2012)
Non Patent Literature 4: Narumi, R. et al., J. Proteome Res. 11, 5311-5322 (2012)

### Summary of Invention

### Technical Problem

It is an object of the present invention to provide a biomarker for detecting colorectal cancer in an early stage.

### Solution to Problem

The present inventors have first searched for CRC biomarker candidate proteins by the document retrieval of PubMed. In order to detect such biomarker candidate proteins in blood, the inventors have focused on extracellular vesicles (EV) present in blood. This is because EV has recently attracted attention as a mediator for intercellular communication, and also because EV is said to be associated with the onset or progression of various diseases and is considered to comprise many biomarker candidates. When a trace amount protein is to be detected in blood using a mass spectrometer, detection of such a trace amount of marker candidate protein may be inhibited by the presence of a large amount of contaminant protein in blood, such as albumin, in some cases. However, since such a large amount of contaminant protein is removed when EV is purified from the blood, it becomes possible to detect a trace amount of biomarker candidate protein in the EV. Accordingly, such a protein contained in EV has been considered to be a promising biomarker candidate.

The present inventors have narrowed down the biomarker candidate proteins discovered by the document retrieval to proteins likely to be present in blood EV, and have then quantified those proteins according to the SRM/MRM method. As a result, the inventors have specified several biomarker candidates for use in the early-stage diagnosis of CRC, thereby completing the present invention.

Specifically, the aspects of the present invention are as follows.
[1] A colorectal cancer biomarker for detecting colorectal cancer, wherein the biomarker consists of at least one protein of the following 22 proteins with numbers 1 to 22, or at least one peptide of the partial peptides of the proteins with numbers 1 to 22:
   1. Annexin A11 (ANXA 11) (SEQ ID NO: 1);
   2. Annexin A3 (ANXA 3) (SEQ ID NO: 2);
   3. Annexin A4 (ANXA 4) (SEQ ID NO: 3);
   4. Tanascin-N (TNN) (SEQ ID NO: 4);
   5. Transferrin receptor protein 1 (TFRC) (SEQ ID NO: 5);
   6. Glucose transporter 1(GLUT-1) (SLC2A1) (SEQ ID NO: 6);
   7. Complement component C9 (C9) (SEQ ID NO: 7);
   8. CD88 antigen (C5AR1) (SEQ ID NO: 8);
   9. 78 kDa glucose-regulated protein (HSPA5) (SEQ ID NO: 9);
   10. α-1-acid glycoprotein (ORM1) (SEQ ID NO: 10);
   11. Matrix metalloproteinase-9 (MMP9) (SEQ ID NO: 11);
   12. Angiopoietin-1 (ANGPT1) (SEQ ID NO: 12);
   13. CD67 antigen (CEACAM8) (SEQ ID NO: 13);
   14. Mucin-5B (MUC5B) (SEQ ID NO: 14);
   15. Adapter protein GRB2 (GRB2) (SEQ ID NO: 15);
   16. Annexin A5(Annexin A5)(ANXA 5) (SEQ ID NO: 16);
   17. Olfactomedin-4 (OLFM4) (SEQ ID NO: 17);
   18. Neutral amino acid transporter B(0) (SLC1A5) (SEQ ID NO: 18);
   19. Tripeptidyl-peptidase 1 (TPP1) (SEQ ID NO: 19);
   20. Heat shock-related 70 kDa protein 2 (HSPA2) (SEQ ID NO: 20);
   21. Proteasome subunit α type-5 (PSMA5) (SEQ ID NO: 21); or
   22. Neutrophil gelatinase-associated lipocalin (LCN2) (SEQ ID NO: 22).
[2] The colorectal cancer biomarker for detecting colorectal cancer according to the above [1], which consists of a combination of two or more proteins of the 22 proteins with numbers 1 to 22 according to the above [1], or two or more peptides of the partial peptides of the proteins with numbers 1 to 22.
[3] The colorectal cancer biomarker for detecting colorectal cancer according to the above [1] or [2], wherein the partial peptides of the proteins with numbers 1 to 22 according to the above [1] are peptides consisting of the amino acid sequences as set forth in SEQ ID NOS: 23 to 59.
[4] The colorectal cancer biomarker for detecting colorectal cancer according to the above [1], which consists of at least one protein of the following 12 proteins, or at least one peptide of the partial peptides of the 12 proteins:
   1. Annexin A11 (SEQ ID NO: 1);
   2. Annexin A3 (SEQ ID NO: 2);
   3. Annexin A4 (SEQ ID NO: 3);
   4. Tanascin-N (SEQ ID NO: 4);
   5. Transferrin receptor protein 1 (SEQ ID NO: 5);
   6. Glucose transporter 1 (SEQ ID NO: 6);
   8. CD88 antigen (SEQ ID NO: 8);
   11. Matrix metalloproteinase-9 (SEQ ID NO: 11);
   16. Annexin A5 (SEQ ID NO: 16);
   17. Olfactomedin-4 (SEQ ID NO: 17);
   19. Tripeptidyl-peptidase 1 (SEQ ID NO: 19); or
   22. Neutrophil gelatinase-associated lipocalin (SEQ ID NO: 22).
[5] The colorectal cancer biomarker for detecting colorectal cancer according to the above [4], which consists of a combination of two or more proteins of the 12 proteins according to the above [4], or two or more peptides of the partial peptides of the 12 proteins.
[6] The colorectal cancer biomarker for detecting colorectal cancer according to the above [4] or [5], wherein the partial peptides of the 12 proteins according to the above [4] are peptides consisting of the amino acid sequences as set forth in SEQ ID NOS: 23 to 34, 37, 38, 43, 44, 53, 54, 56 and 59.
[7] The colorectal cancer biomarker for detecting colorectal cancer according to the above [1], which consists of Annexin A4 or Annexin A11, or a partial peptide thereof.
[8] The colorectal cancer biomarker for detecting colorectal cancer according to the above [7], which consists of a combination of Annexin A4 and Annexin A11, or partial peptides of Annexin A4 and partial peptides of Annexin A11.
[9] The colorectal cancer biomarker for detecting colorectal cancer according to the above [7] or [8], wherein the partial peptides of Annexin A4 and Annexin A11 are peptides consisting of the amino acid sequences as set forth in SEQ ID NOS: 23, 24, 27 and 28.
[10] A colorectal cancer biomarker for detecting colorectal cancer, which consists of a combination of the colorectal cancer biomarker according to any one of the above [1] to [9] and CEA.
[11] A method of detecting colorectal cancer, comprising measuring the colorectal cancer biomarker according to any one of the above [1] to [10] in a biological sample.
[12] A method of detecting colorectal cancer, comprising measuring the colorectal cancer biomarker according to any one of the above [1] to [10] in a biological sample, and then determining that the subject is affected with colorectal cancer when the biomarker is present in a higher concentration in the biological sample than in a healthy subject.
[13] The method of detecting colorectal cancer according to the above [11] or [12], wherein the biological sample is an extracellular vesicle (EV) in blood.
[14] The method of detecting colorectal cancer according to any one of the above [11] to [13], wherein the detection is carried out by an immunoassay or a mass spectrometry.
[15] A kit of detecting colorectal cancer, comprising an antibody reacting against the colorectal cancer biomarker according to any one of the above [1] to [10] in the biological sample.

The present application claims priority from Japanese Patent Application No. 2017-129941; the disclosure of which is hereby incorporated by reference.

### Advantageous Effects of Invention

According to the biomarker of the present invention, colorectal cancer can be detected with high sensitivity and high specificity, and in particular, early-stage colorectal cancer can be detected. Furthermore, by combining the present biomarkers with one another, colorectal cancer can be detected with higher sensitivity and higher specificity.

### Brief Description of Drawings

[Figure 1] Figure 1 is a view showing a strategy for searching for a colorectal cancer marker in an extracellular vesicle (EV). In the figure, the number of candidate proteins selected in each step is shown.
[Figure 2] Figure 2 is a view showing a method of shotgun proteomics analysis for CRC biomarker candidate proteins in EV. Figure 2a shows a step of preparation of EV and MS analysis, and Figure 2b shows the results of Venn diagram analysis performed on biomarker candidate proteins and EV proteins, which had been identified by the shotgun proteome analysis. Among the EV proteins identified from serum or cell culture supernatant, 356 colorectal cancer biomarker candidate proteins were identified to be EV proteins.
[Figure 3] Figure 3 is a table showing SRM target proteins (46) and peptides (71).
[Figure 4] Figure 4 is a table showing a list of proteins verified with high accuracy (AUC > 0.7).
[Figure 5-1] Figure 5-1 is a view showing the results of relative quantification performed on serum peptides among 3 groups (N, C and Cm) according to SRM analysis. N indicates non-cancerous control patients, C indicates non-metastatic cancer patients, and Cm indicates metastatic cancer patients. The dot plot graph shows the peak area ratio of endogenous peptides to SI peptides (*; p < 0.05, **; p < 0.01, N.S; no significant difference). The longitudinal axis indicates Area Ratio.
[Figure 5-2] Figure 5-2 is a view showing the results of relative quantification performed on peptides among 3 groups (N, C and Cm) according to SRM analysis (continuation of Figure 5-1).
[Figure 5-3] Figure 5-3 is a view showing the results of relative quantification performed on peptides among 3 groups (N, C and Cm) according to SRM analysis (continuation of Figure 5-2).
[Figure 5-4] Figure 5-4 is a view showing the results of relative quantification performed on peptides among 3 groups (N, C and Cm) according to SRM analysis (continuation of Figure 5-3).
[Figure 5-5] Figure 5-5 is a view showing the results of relative quantification performed on peptides among 3 groups (N, C and Cm) according to SRM analysis (continuation of Figure 5-4).

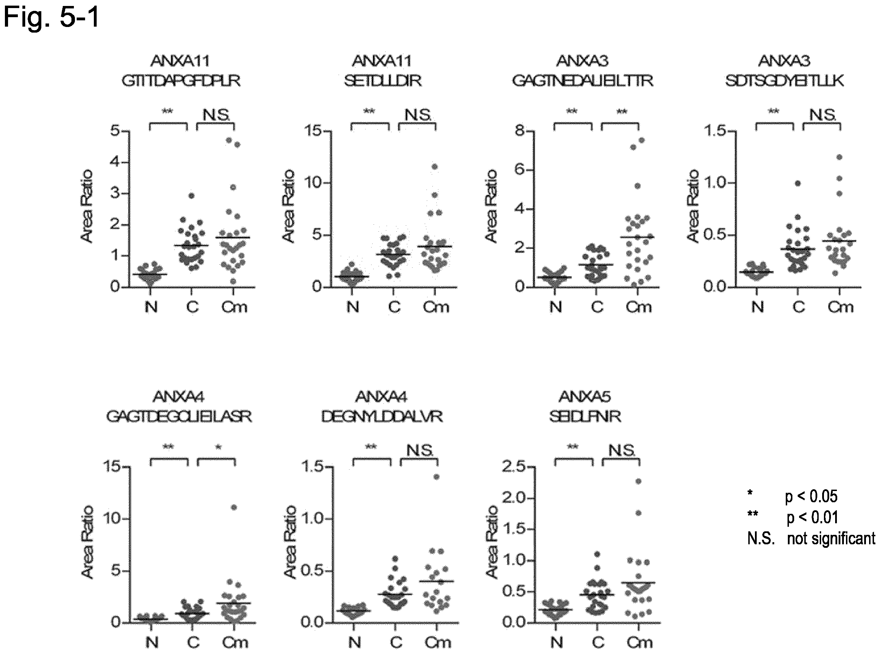

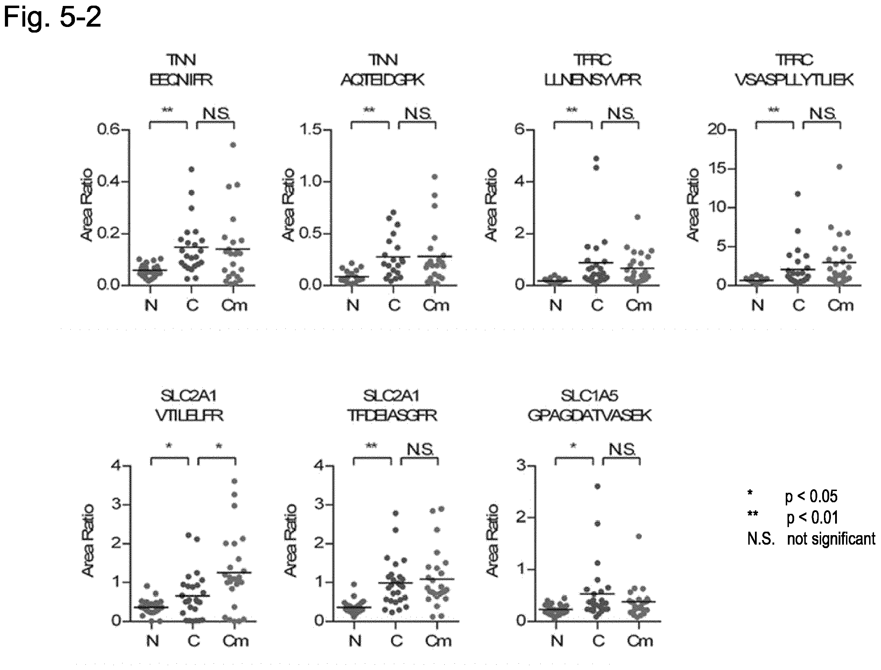

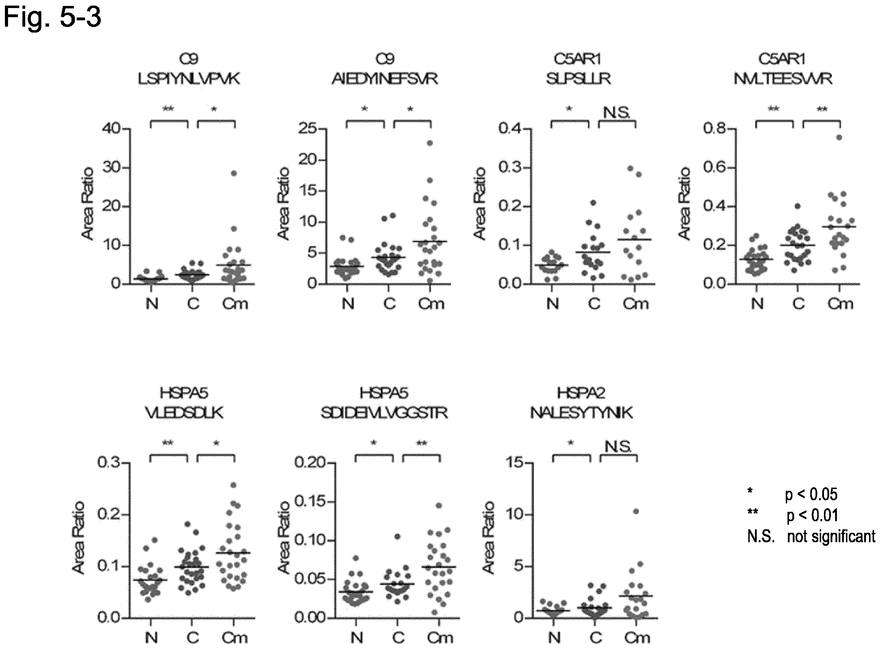

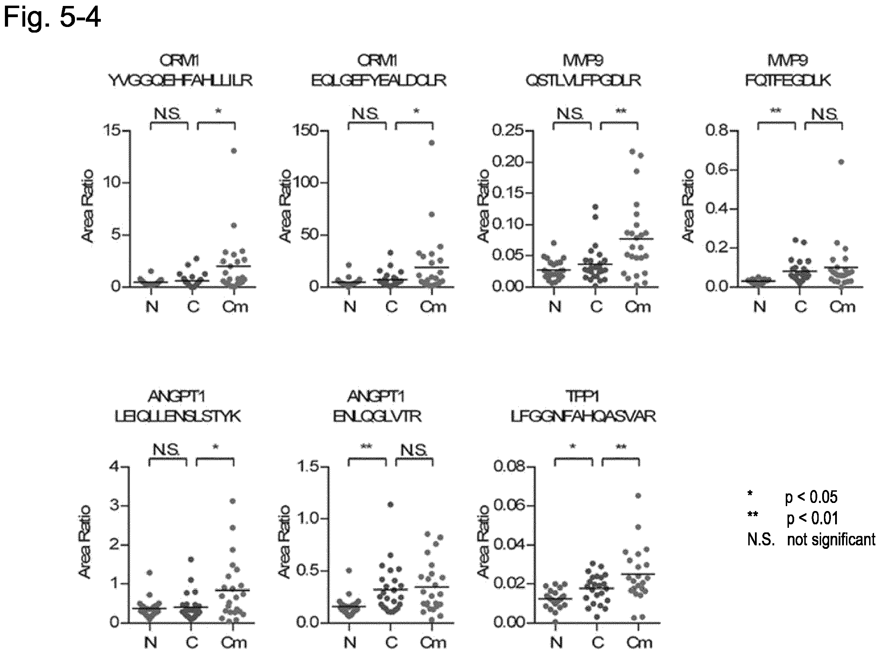

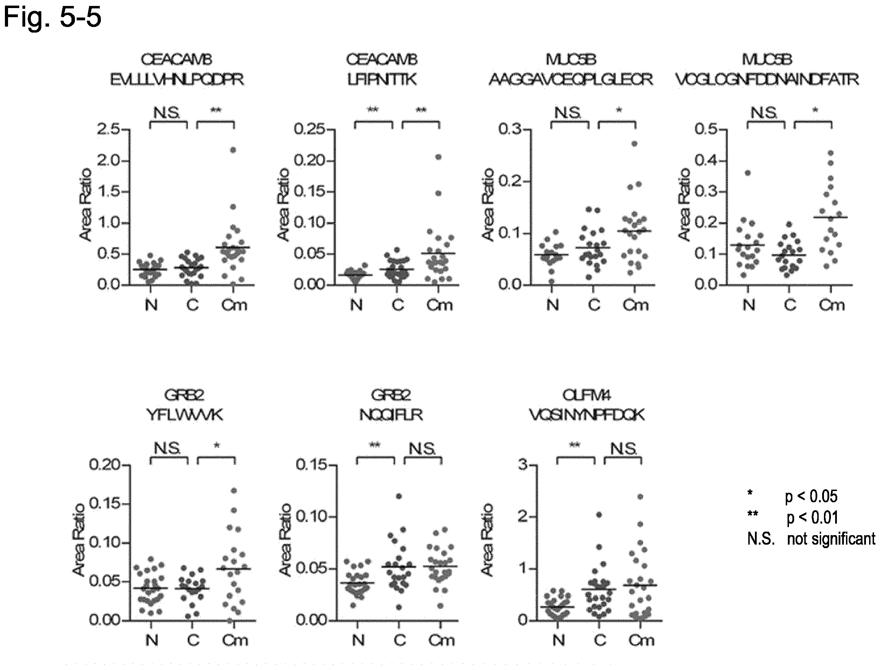

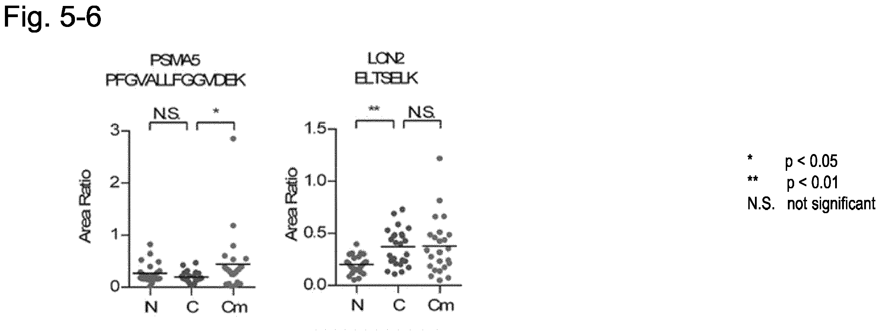

[Figure 5-6] Figure 5-6 is a view showing the results of relative quantification performed on peptides among 3 groups (N, C and Cm) according to SRM analysis (continuation of Figure 5-5).

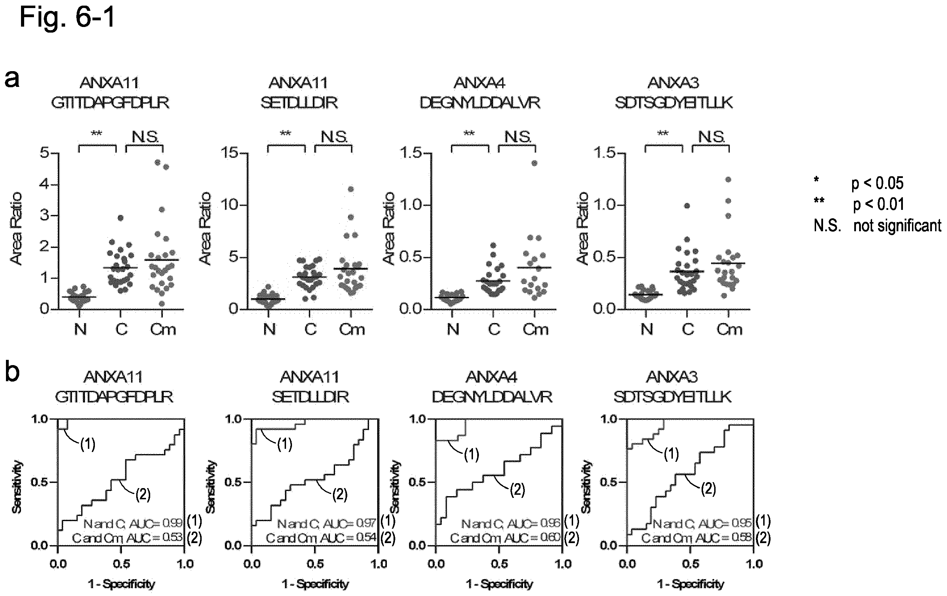

[Figure 6-1] Figure 6-1 is a view showing the results of the statistical analysis of target peptides (Part 1). Figure 6-1a shows the results of relative quantification performed on peptides among 3 groups (N, C and Cm) according to SRM analysis. N indicates non-cancerous control, C indicates non-metastatic cancer, and Cm indicates metastatic cancer. The dot plot graph shows the peak area ratio of endogenous peptides to SI peptides (*; p < 0.05, **; p < 0.01, N.S; no significant difference). The longitudinal axis indicates Area Ratio. Figure 6-1b shows the results (1) of ROC curve analysis for discriminating between N and C, and the results (2) of ROC curve analysis for discriminating between C and Cm. The area under the curve (AUC) for discrimination is shown in each graph. The longitudinal axis indicates sensitivity, and the horizontal axis indicates 1-specificity.

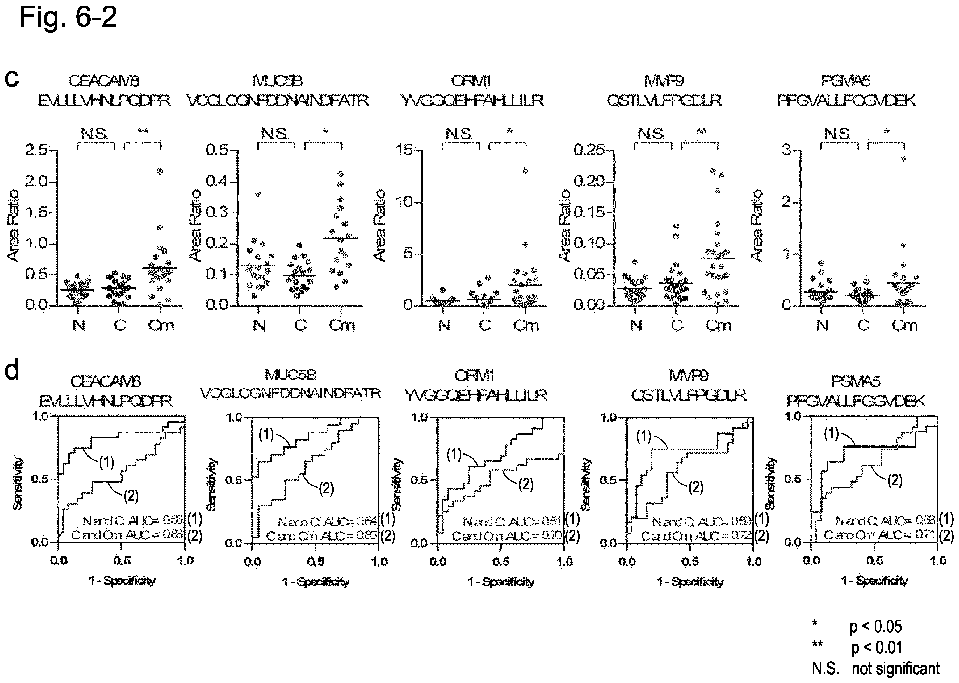

[Figure 6-2] Figure 6-2 is a view showing the results of the statistical analysis of target peptides (Part 2). Figure 6-2c shows the results of relative quantification performed on peptides among 3 groups (N, C and Cm) according to SRM analysis. N indicates non-cancerous control, C indicates non-metastatic cancer, and Cm indicates metastatic cancer. The dot plot graph shows the peak area ratio of endogenous peptides to SI peptides (*; p < 0.05, **; p < 0.01, N.S; no significant difference). The longitudinal axis indicates Area Ratio. Figure 6-2d shows the results (1) of ROC curve analysis for discriminating between N and C and the results (2) of ROC curve analysis for discriminating between C and Cm. The area under the curve (AUC) for discrimination is shown in each graph. The longitudinal axis indicates sensitivity, and the horizontal axis indicates 1-specificity.

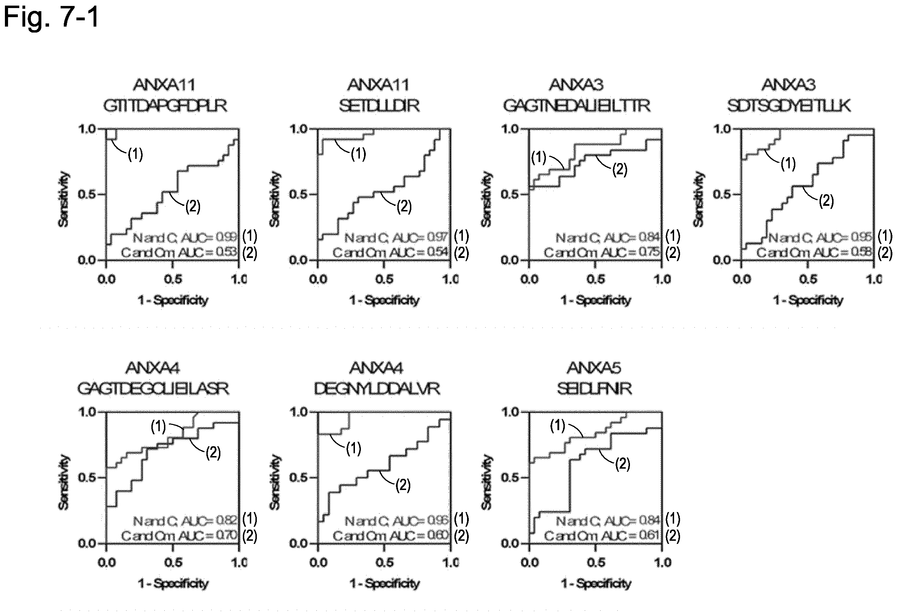

[Figure 7-1] Figure 7-1 is a view showing ROC curve (1) for discriminating between N and C, and ROC curve (2) for discriminating between C and Cm (Part 1). The area under the curve (AUC) for discrimination is shown in each graph. The longitudinal axis indicates sensitivity, and the horizontal axis indicates 1-specificity.

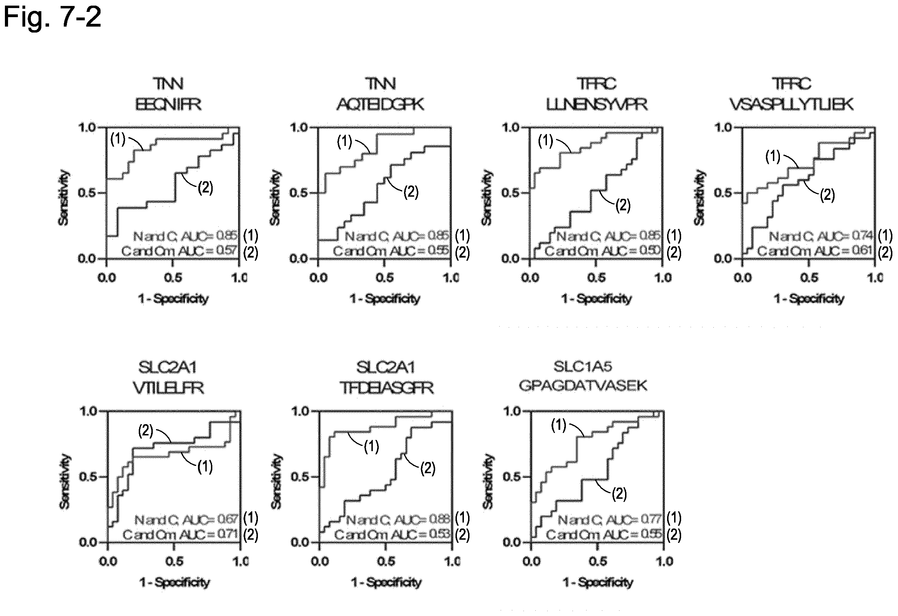

[Figure 7-2] Figure 7-2 is a view showing ROC curve (1) for discriminating between N and C, and ROC curve (2) for discriminating between C and Cm (continuation of Figure 7-1).

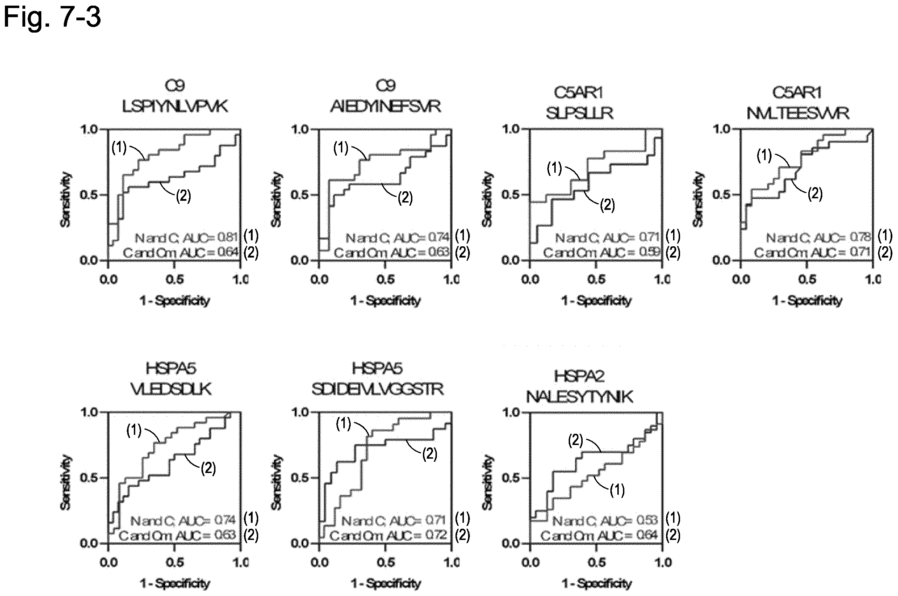

[Figure 7-3] Figure 7-3 is a view showing ROC curve (1) for discriminating between N and C, and ROC curve (2) for discriminating between C and Cm (continuation of Figure 7-2).

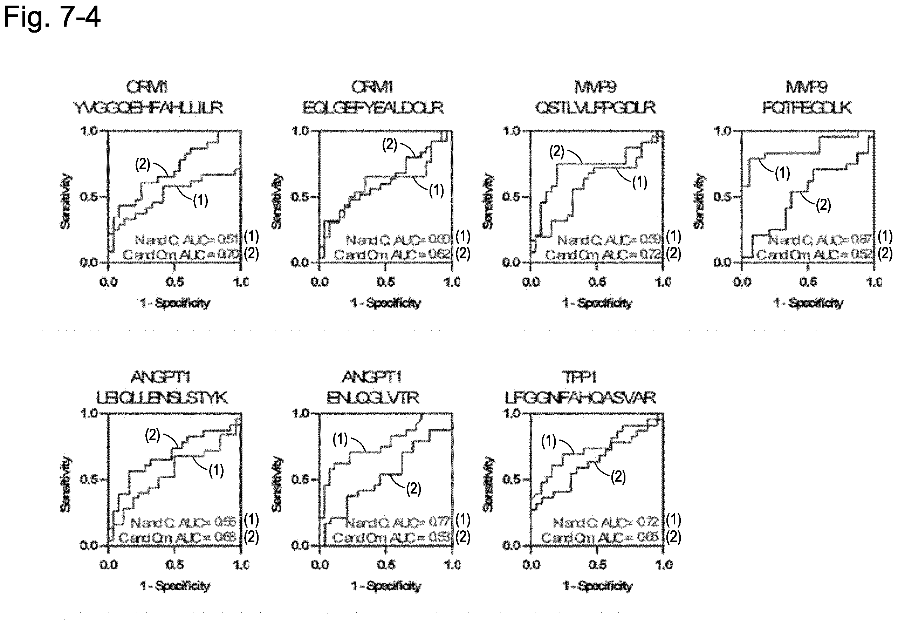

[Figure 7-4] Figure 7-4 is a view showing ROC curve (1) for discriminating between N and C, and ROC curve (2) for discriminating between C and Cm (continuation of Figure 7-3).

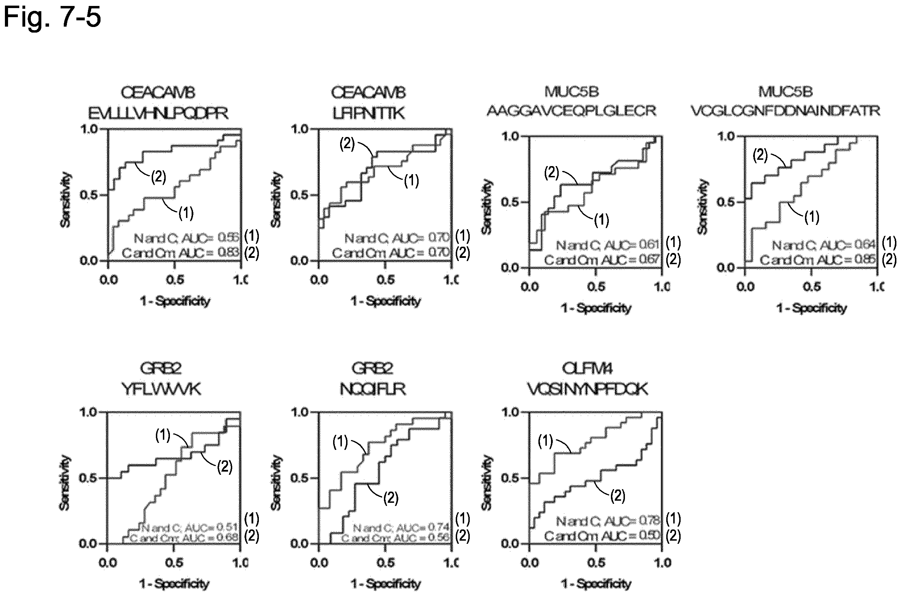

[Figure 7-5] Figure 7-5 is a view showing ROC curve (1) for discriminating between N and C, and ROC curve (2) for discriminating between C and Cm (continuation of Figure 7-4).

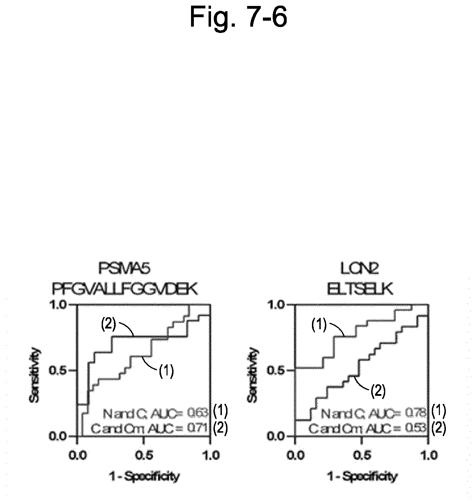

[Figure 7-6] Figure 7-6 is a view showing ROC curve (1) for discriminating between N and C, and ROC curve (2) for discriminating between C and Cm (continuation of Figure 7-5).

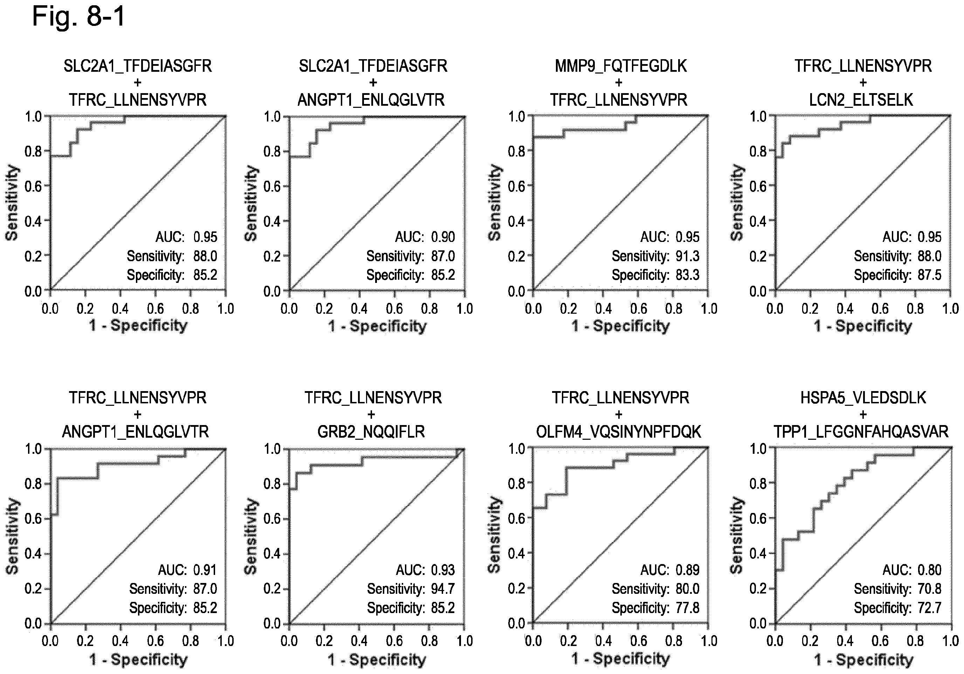

[Figure 8-1] Figure 8-1 is a view showing the results of ROC curve analysis performed on the combinations of target peptides (Part 1). The diagnostic sensitivity obtained by the combination of peptides was evaluated between N and C. The area under the curve (AUC), sensitivity and specificity are shown in each graph.

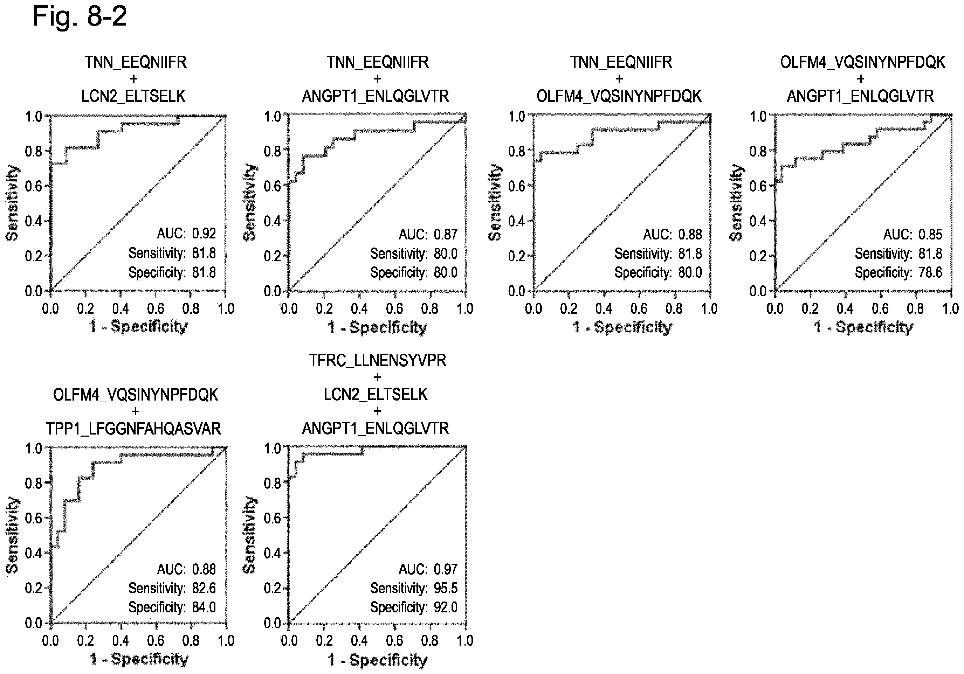

[Figure 8-2] Figure 8-2 is a view showing the results of ROC curve analysis performed on the combinations of target peptides (Part 2). The diagnostic sensitivity obtained by the combination of peptides was evaluated between N and C. The area under the curve (AUC), sensitivity and specificity are shown in each graph.

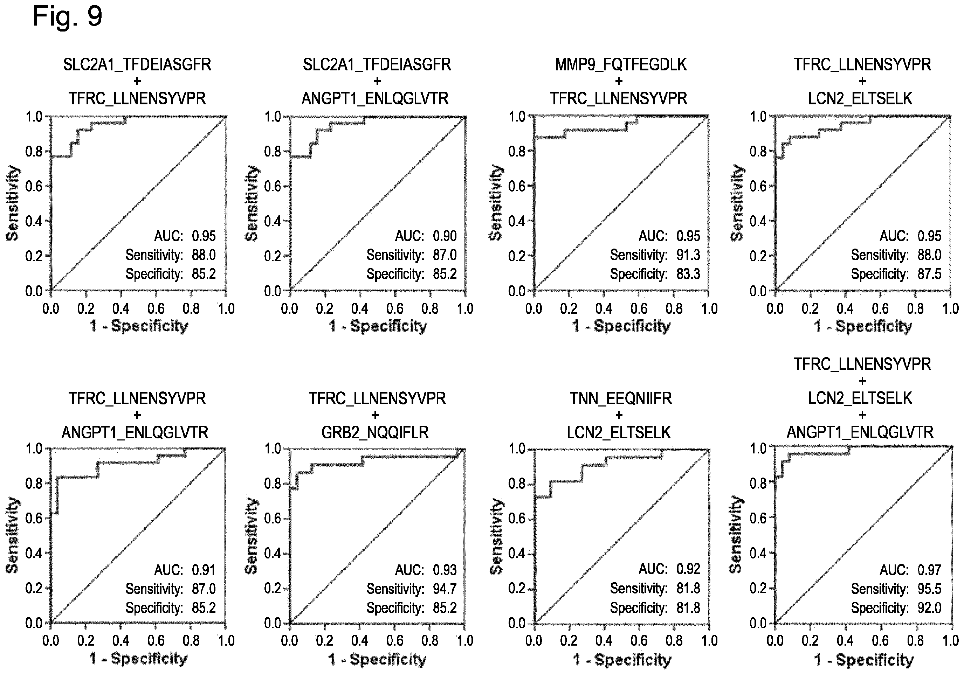

[Figure 9] Figure 9 is a view showing the results of ROC curve analysis performed on the combinations of target peptides. The diagnostic sensitivity obtained by the combination of peptides was evaluated between N and C. The area under the curve (AUC), sensitivity and specificity are shown in each graph. Figure 9 shows combinations having high accuracy (AUC > 0.9). Other combinations are shown in Figure 8-1 and Figure 8-2.

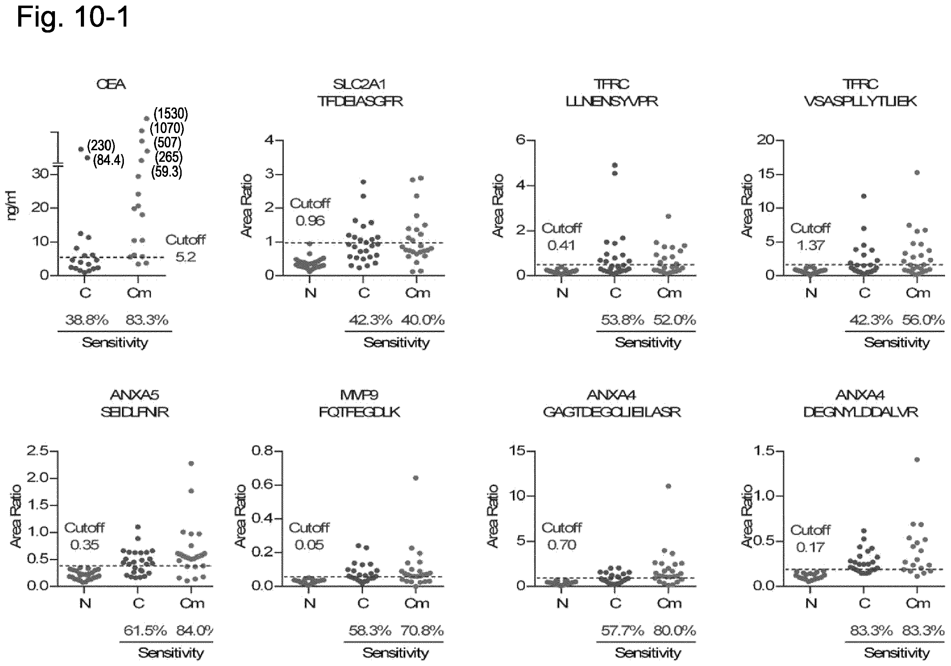

[Figure 10-1] Figure 10-1 is a view showing the results of a comparison between the target peptides and CEA in terms of sensitivity. When the cutoff value is set to be in the maximum peak area of N, the sensitivity is calculated to be the ratio of the sample (wherein the dotted line indicates a specificity of 100%). The longitudinal axis indicates Area Ratio.

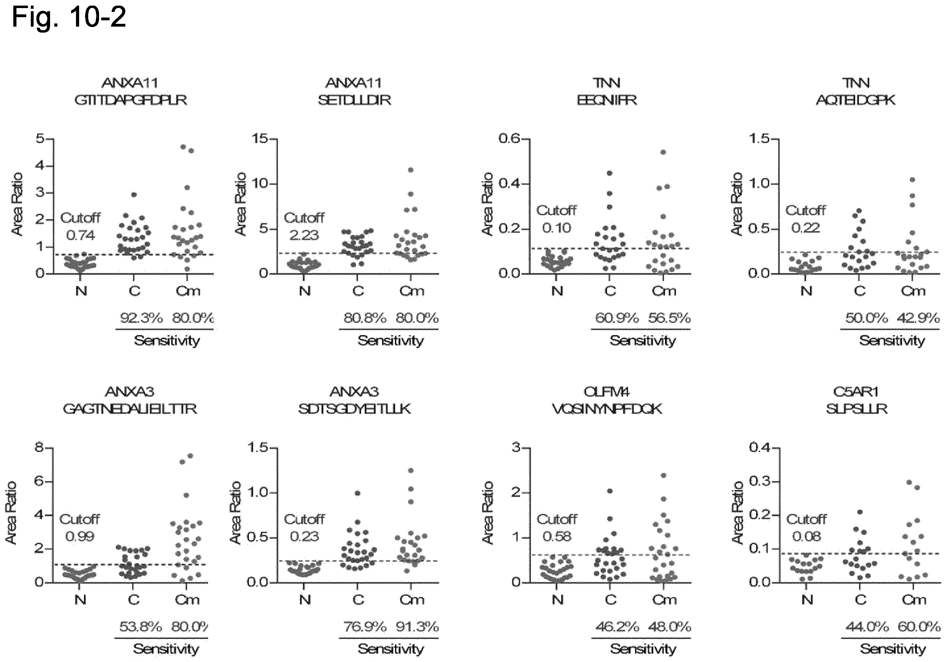

[Figure 10-2] Figure 10-2 is a view showing the results of a comparison between the target peptides and CEA in terms of sensitivity (continuation of Figure 10-1).

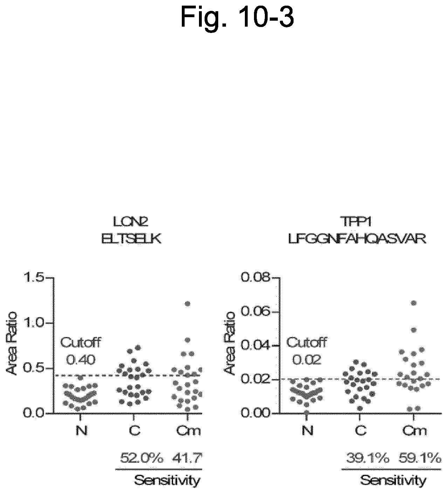

[Figure 10-3] Figure 10-3 is a view showing the results of a comparison between the target peptides and CEA in terms of sensitivity (continuation of Figure 10-2).

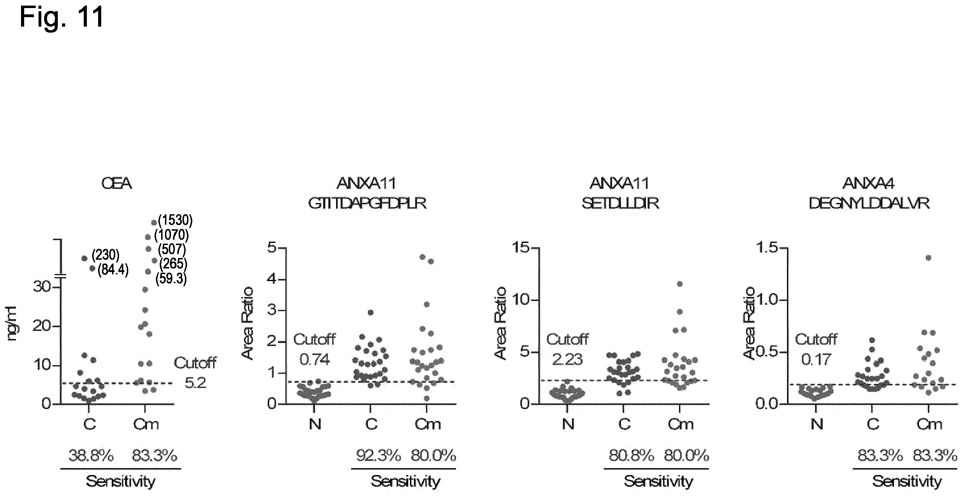

[Figure 11] Figure 11 is a view showing the results of a comparison between the target peptides and CEA in terms of sensitivity. When the cutoff value (Cutoff) is set to be in the maximum peak area of N, the sensitivity is calculated to be the ratio of the sample (wherein the dotted line indicates a specificity of 100%). Figure 11 shows top three peptides having high sensitivity in discrimination between N and C. The longitudinal axis of the graphs regarding the three peptides indicates Area Ratio. Other peptides having higher sensitivity than CEA are shown in Figures 10-1 to 10-3.

### Description of Embodiments

Hereinafter, the present invention will be described in detail.

The present invention relates to a protein used as a biomarker for detecting colorectal cancer (CRC), or a partial peptide thereof. In addition, the present invention relates to a method of detecting colorectal cancer, using the biomarker. Moreover, the present invention relates to a method for obtaining auxiliary data for use in diagnosing colorectal cancer using the biomarker. By using the biomarker of the present invention, it is possible to detect colorectal cancer in an early stage.

The biomarker used in the present invention can be searched, for example, according to proteome analysis technology. For example, the biomarker used in the present can be searched by the following process.

First, using the tissues of colorectal cancer patients, proteins that become biomarker candidates are searched according to quantitative shotgun proteomics. In this searching, comparative quantification is carried out among the colorectal tissues or colorectal benign tumor tissues of a healthy subject, the tissues of a non-metastatic colorectal cancer case, and the tissues of a metastatic colorectal cancer case, so that proteins found to fluctuate among the aforementioned tissues can be narrowed as candidate proteins. Moreover, such biomarker candidate proteins can also be searched by using proteins that have been reported to be associated with the onset or progression of colorectal cancer according to previous studies. A fluctuation in the expression levels among the tissues can be verified according to a target proteomics method involving a SRM/MRM (selected reaction monitoring/multiple reaction monitoring) method (Gillette MA et al., Nat Methods. 2013; 10: 28-34), by which, with respect to such biomarker candidates, parent ions having a specific mass are destroyed, and specific ions in the generated daughter ions are then detected, so that a peptide derived from a target protein can be detected with high sensitivity in a complicated sample. According to this verification, final candidates of colorectal cancer biomarkers can be selected. Subsequently, whether or not these biomarker candidates can be detected and quantified in a biological sample such as blood is confirmed. During this operation, the biomarker candidates are preferably detected and quantified in an extracellular vesicle (EV) fraction in blood.

In the method of the present invention, the following proteins may be considered to be biomarkers for detection of colorectal cancer:
1. Annexin A11 (ANXA 11) (SEQ ID NO: 1);
2. Annexin A3 (ANXA 3) (SEQ ID NO: 2);
3. Annexin A4 (ANXA 4) (SEQ ID NO: 3);
4. Tanascin-N (TNN) (SEQ ID NO: 4);
5. Transferrin receptor protein 1 (TFRC) (SEQ ID NO: 5);
6. Glucose transporter 1(GLUT-1) (SLC2A1) (SEQ ID NO: 6);
7. Complement component C9 (C9) (SEQ ID NO: 7);
8. CD88 antigen (C5AR1) (SEQ ID NO: 8);
9. 78 kDa glucose-regulated protein (HSPA5) (SEQ ID NO: 9);
10. α-1-acid glycoprotein (ORM1) (SEQ ID NO: 10);
11. Matrix metalloproteinase-9 (MMP9) (SEQ ID NO: 11);
12. Angiopoietin-1 (ANGPT1) (SEQ ID NO: 12);
13. CD67 antigen (CEACAM8) (SEQ ID NO: 13);
14. Mucin-5B (MUC5B) (SEQ ID NO: 14);
15. Adapter protein GRB2 (GRB2) (SEQ ID NO: 15);
16. Annexin A5(Annexin A5)(ANXA 5) (SEQ ID NO: 16);
17. Olfactomedin-4 (OLFM4) (SEQ ID NO: 17);
18. Neutral amino acid transporter B(0) (SLC1A5) (SEQ ID NO: 18);
19. Tripeptidyl-peptidase 1 (TPP1) (SEQ ID NO: 19);
20. Heat shock-related 70 kDa protein 2 (HSPA2) (SEQ ID NO: 20);
21. Proteasome subunit α type-5 (PSMA5) (SEQ ID NO: 21); and
22. Neutrophil gelatinase-associated lipocalin (LCN2) (SEQ ID NO: 22).

Moreover, examples of the partial peptides of the above-described proteins that can be used as biomarkers for detection of colorectal cancer may include:
a peptide consisting of the amino acid sequence as set forth in SEQ ID NO: 23 or 24 (a partial peptide of Annexin A11),
a peptide consisting of the amino acid sequence as set forth in SEQ ID NO: 25 or 26 (a partial peptide of Annexin A3),
a peptide consisting of the amino acid sequence as set forth in SEQ ID NO: 27 or 28 (a partial peptide of Annexin A4),
a peptide consisting of the amino acid sequence as set forth in SEQ ID NO: 29 or 30 (a partial peptide of Tanascin-N),
a peptide consisting of the amino acid sequence as set forth in SEQ ID NO: 31 or 32 (a partial peptide of Transferrin receptor protein 1),
a peptide consisting of the amino acid sequence as set forth in SEQ ID NO: 33 or 34 (a partial peptide of Glucose transporter 1),
a peptide consisting of the amino acid sequence as set forth in SEQ ID NO: 35 or 36 (a partial peptide of Complement component C9),
a peptide consisting of the amino acid sequence as set forth in SEQ ID NO: 37 or 38 (a partial peptide of CD88 antigen),
a peptide consisting of the amino acid sequence as set forth in SEQ ID NO: 39 or 40 (a partial peptide of 78 kDa glucose-regulated protein),
a peptide consisting of the amino acid sequence as set forth in SEQ ID NO: 41 or 42 (a partial peptide of α-1-acid glycoprotein),
a peptide consisting of the amino acid sequence as set forth in SEQ ID NO: 43 or 44 (a partial peptide of Matrix metalloproteinase-9),
a peptide consisting of the amino acid sequence as set forth in SEQ ID NO: 45 or 46 (a partial peptide of Angiopoietin-1),
a peptide consisting of the amino acid sequence as set forth in SEQ ID NO: 47 or 48 (a partial peptide of CD67 antigen),
a peptide consisting of the amino acid sequence as set forth in SEQ ID NO: 49 or 50 (a partial peptide of Mucin-5B),
a peptide consisting of the amino acid sequence as set forth in SEQ ID NO: 51 or 52 (a partial peptide of Adapter protein GRB2),
a peptide consisting of the amino acid sequence as set forth in SEQ ID NO: 53 (a partial peptide of Annexin A5),
a peptide consisting of the amino acid sequence as set forth in SEQ ID NO: 54 (a partial peptide of Olfactomedin-4),
a peptide consisting of the amino acid sequence as set forth in SEQ ID NO: 55 (a partial peptide of Neutral amino acid transporter B(0)),
a peptide consisting of the amino acid sequence as set forth in SEQ ID NO: 56 (a partial peptide of Tripeptidyl-peptidase 1),
a peptide consisting of the amino acid sequence as set forth in SEQ ID NO: 57 (a partial peptide of Heat shock-related 70 kDa protein 2),
a peptide consisting of the amino acid sequence as set forth in SEQ ID NO: 58 (a partial peptide of Proteasome subunit α type-5), and
a peptide consisting of the amino acid sequence as set forth in SEQ ID NO: 59 (a partial peptide of Neutrophil gelatinase-associated lipocalin).

In the present invention, the above-described proteins, or partial peptides of the proteins are used as biomarkers. The above-described proteins or partial peptides thereof may also include proteins each consisting of an amino acid sequence comprising a deletion, substitution or addition of one or several amino acids in the amino acid sequences as set forth in SEQ ID NOS: 1 to 59, or peptides thereof. These proteins or peptides can also be used as biomarkers in the method of the present invention. Herein, the phrase "one or several" means "1 or 3," "1 or 2," or "1."

The term "peptide" generally means amino acid residues having a molecular weight of 10,000 or less, which are connected with one another via a peptide bond. The number of amino acid residues is from several to approximately 50 or less. In the present description, the protein or a partial peptide thereof can be used as a biomarker. When the partial peptide is used as a biomarker, it is a peptide having a partial amino acid sequence that is a part of the amino acid sequence of a protein, and the molecular weight thereof is not limited. It is preferably a peptide having a molecular weight of 10,000 or less. There is a case where the partial peptide is generated in the expression and synthesis process involving transcription and/or translation. There is also a case where, after synthesis of a protein, the protein is subjected to digestive degradation *in vivo*, so that the partial peptide is generated as a digestive degradation product peptide.

As a result of the ROC analysis, it has been revealed that, among these proteins and peptides, Glucose transporter 1, Transferrin receptor protein 1, Annexin A5, Matrix metalloproteinase-9, Annexin A4, Annexin A11, Tanascin-N, Annexin A3, Olfactomedin-4, CD88 antigen, Neutrophil gelatinase-associated lipocalin or tripeptidyl-peptidase 1, or their partial peptides can detect colorectal cancer with favorable detection sensitivity and specificity. Thus, the aforementioned proteins, peptides or their partial peptides are preferably used as biomarkers for detection of colorectal cancer. Furthermore, Annexin A4 or Annexin A11, or their partial peptides are more preferably used as biomarkers for detection of colorectal cancer.

In the present invention, among the above-described 22 proteins and partial peptides, at least one protein and/or at least one partial peptide can be used as a biomarker. However, preferably 2 types or more, more preferably 3 types or more, 4 types or more, 5 types or more, 6 types or more, 7 types or more, 8 types or more, 9 types or more, 10 types or more, 11 types or more, 12 types or more, 13 types or more, 14 types or more, 15 types or more, 16 types or more, 17 types or more, 18 types or more, 19 types or more, 20 types or more, 21 types or more, or 22 types proteins or partial peptides thereof can be used to detect colorectal cancer with higher accuracy. In this case, it may be a combination of different proteins, or may also be a combination of the partial peptide of a certain protein and the partial peptide of a protein other than the certain protein, or a combination of partial peptides. Otherwise, a plurality of intact proteins alone may be used as biomarkers. Thus, by using a plurality of proteins or partial peptides as biomarkers, colorectal cancer can be more accurately detected, and also, the progression of colorectal cancer can be precisely judged.

Examples of the combination of proteins may include a combination of Glucose transporter 1 and Transferrin receptor protein 1, a combination of Glucose transporter 1 and Angiopoietin-1, a combination of Matrix metalloproteinase-9 and Transferrin receptor protein 1, a combination of Transferrin receptor protein 1 and Neutrophil gelatinase-associated lipocalin, a combination of Transferrin receptor protein 1 and Angiopoietin-1, a combination of Transferrin receptor protein 1 and Adapter protein, a combination of 78 kDa glucose-regulated protein and Tripeptidyl-peptidase 1, a combination of Tanascin-N and Neutrophil gelatinase-associated lipocalin, a combination of Transferrin receptor protein 1, Neutrophil gelatinase-associated lipocalin and Angiopoietin-1, a combination of Transferrin receptor protein 1 and Olfactomedin-4, a combination of Tanascin-N and Angiopoietin-1, a combination of Olfactomedin-4 and Angiopoietin-1, a combination of Tanascin-N and Olfactomedin-4, and a combination of Olfactomedin-4 and Tripeptidyl-peptidase 1. Combination of the partial peptides of the aforementioned respective proteins may also be adequate.

Further, the above-described protein or a partial peptide thereof is combined with CEA (Carcinoembryonic antigen) that has conventionally been known as a biomarker for colorectal cancer, and the combination is used as a biomarker for detection of colorectal cancer, so that colorectal cancer can be detected with higher sensitivity and higher specificity than in the case of a single use thereof.

The expression of the above-described protein or a partial peptide thereof increases in colorectal cancer patients. Accordingly, the protein or a partial peptide thereof in blood may be quantified. Herein, the blood includes serum and plasma. Moreover, the protein is present in an extracellular vesicle (EV). As such, EV present in blood is isolated, and is then concentrated, as necessary, and thereafter, the protein or a partial peptide thereof may be detected in the EV. EV can be separated as an EV fraction, for example, according to ultracentrifugation. Otherwise, EV can be isolated using a marker specific to the EV. Examples of such an EV marker may include CD9, CD63, and CD81, and EV can be separated by utilizing magnetic beads to which antibodies reacting against these markers bind, etc. Another example of the EV marker may be phosphatidylserine (PS), and EV can be separated by utilizing magnetic beads to which phosphatidylserine-binding proteins having affinity for phosphatidylserine bind, etc. Examples of the phosphatidylserine-binding protein may include a T-cell immunoglobulin and mucin domain-containing molecule 4 (Tim4), Annexin A5, Annexin V, and milk fat globule-EGF factor protein 8 (MFG-E8).

By the method of the present invention, protein/partial peptide profile in a biological sample is examined, but the biological sample used herein is not limited. Examples of the biological sample may include liquid samples that can be collected from living bodies, including body fluids such as whole blood, serum, plasma, urine, and saliva. Besides, a protein, a partial protein, and a partial peptide in such a biological sample may be further degraded depending on the preserved state of the biological sample. Thus, the biological sample is preferably used without repeating freezing and thawing operations. Moreover, a protease inhibitor and the like may be added to the biological sample. Otherwise, exosome may be separated with the above-described marker as an indicator, using FACS or a flow cytometer.

A protein or a peptide may be extracted from the isolated exosome, and the protein or the peptide may be then measured.

A protein or a partial peptide can be detected according to various methods. Examples of such various methods are given below, but are not limited thereto.

For example, a protein or a partial peptide thereof to be detected can be measured according to an immunoassay using an antibody reacting against such a protein or a partial peptide thereof to be detected. Examples of the immunoassay may include solid phase immunoassays (RIA, EIA, FIA, CLIA, etc.), a dot blotting method, a latex agglutination method (LA: Latex Agglutination-Turbidimetric Immunoassay), and an immunochromatography method. The antibody can be immobilized on a substrate and can be then used.

Among these methods, from the viewpoint of quantitativity, an ELISA (Enzyme-Linked ImmunoSorbent Assay) method that is one type of EIA (Enzyme Immunoassay) method is preferable. According to the ELISA method, a specimen is added into a well of a microtiter plate, on which an antibody is immobilized, to perform an antigen-antibody reaction, and thereafter, an enzyme-labeled antibody is further added thereto to perform an antigen-antibody reaction, followed by washing. Thereafter, the reaction mixture is allowed to react with and/or develop color with an enzyme substrate, the absorbance is measured, and a marker protein or a partial peptide thereof in the sample is detected, and thereafter, the concentration of the protein or a partial peptide thereof in the specimen can be calculated from the measured value. Otherwise, using a fluorescently-labeled antibody, an antigen-antibody reaction may be performed, and the fluorescence may be then measured. The antigen-antibody reaction can be carried out at 4°C to 45°C, more preferably at 20°C to 40°C, and further preferably at 25°C to 38°C. In addition, the reaction time is approximately 10 minutes to 18 hours, more preferably approximately 10 minutes to 1 hour, and further preferably approximately 30 minutes to 1 hour.

The antibody reacting against a marker protein or a partial peptide thereof used in immunoassays may be either a monoclonal antibody or a polyclonal antibody, and further, binding active fragments of monoclonal antibodies, such as Fab, F(ab'), and F(ab')₂ can also be used.

The present invention also includes a test reagent or kit for detecting colorectal cancer, comprising antibodies reacting against one or more of the above-described 22 types of proteins or partial peptides thereof.

A biomarker protein for detection of colorectal cancer or a partial peptide thereof can also be analyzed according to mass spectrometry using a mass spectrometer. Such mass spectrometry is particularly suitable for detection of a partial peptide.

Moreover, a biomarker protein or a partial peptide thereof can also be detected using a mass spectrometer. In particular, for detection of a partial peptide of a biomarker protein, the use of a mass spectrometer is suitable. The mass spectrometer comprises a sample introduction part, an ionization chamber, an analysis part, a detection part, a record part, and the like. Examples of the ionization method that may be applied herein may include an electron ionization (EI) method, a chemical ionization (CI) method, a field desorption (FD) method, a secondary ion mass spectrometry (SIMS) method, a fast atom bombardment (FAB) method, a matrix-assisted laser desorption ionization (MALDI) method, and an electrospray ionization (ESI) method. Moreover, for the analysis part, a double-focusing mass spectrometer, a quadrupole mass spectrometer, a time-of-flight mass spectrometer, a Fourier transform mass spectrometer, an ion cyclotron mass spectrometer, or the like is used. For performing a precise analysis, a tandem mass spectrometer (MS/MS), in which two mass spectrometers are connected with each other, can also be used.

The mass spectrometer may be used alone, or may also be connected with separation equipment such as liquid chromatography, measurement equipment, and the like, and thus, a liquid chromatograph-mass spectrometry (LC/MS or LC/MS/MS), in which mass spectrometry is combined with high performance liquid chromatography, can be used in the analysis. Mass spectrometry can be carried out by selected reaction monitoring (SRM) or multiple reaction monitoring (MRM) using a triple quadrupole mass spectrometer, according to an LC/MS (LC/MS/MS) System that is broadly used for quantitative detection of a peptide in the present technical field. According to SRM/MRM, many factors can be simultaneously measured, and thus, several hundreds of proteins or peptides can be simultaneously measured by a single measurement.

When a large amount of the above described biomarker protein or a partial peptide thereof is present in a sample collected from a subject, namely, when the subject is positive, the subject can be determined to be affected with colorectal cancer.

In the present invention, a sample collected from a healthy subject may be simultaneously measured as a negative control. In this case, when a subject is affected with colorectal cancer, the concentration of a biomarker protein or a partial peptide thereof in the specimen of the subject increases in comparison to the healthy subject. Hence, when the concentration of the biomarker protein or a partial peptide thereof in the subject is higher than that in the healthy subject, the biomarker protein or a partial peptide thereof is determined to be positive, and thus, the subject can be determined to be affected with colorectal cancer. With regard to whether or not the biomarker protein or a partial peptide thereof in a sample is positive, a cutoff value has previously been determined with respect to the ratio between the peak area value of a biomarker peptide that has previously been detected according to the SRM/MRM method and the peak area value of a stable isotope-labeled peptide used as an internal standard, or with respect to an antibody measured value, and the cutoff value is used as a reference. When the measured value exceeds the cutoff value, the biomarker protein or a partial peptide thereof can be determined to be positive.

The cutoff value can be determined, for example, by ROC (receiver operating characteristic curve) analysis. Moreover, the diagnostic accuracy (sensitivity and specificity) of the method of the present invention can be determined by the ROC analysis. According to the ROC analysis, the biomarker protein or a partial peptide thereof in a sample collected from a colorectal cancer patient and the biomarker protein or a partial peptide thereof in a sample collected from a healthy subject are measured, and sensitivity and false positive rate (1-specificty) are then calculated for each cutoff value. Then, the horizontal axis is set to be 1-specificity, the longitudinal axis is set to be sensitivity, and the values are plotted on the coordinates.

When diagnostic accuracy is analyzed based on the measurement results of the method of the present invention according to the ROC analysis, the area under the curve (AUC) is high (0.9 or more); the sensitivity is 70% or more, preferably 80% or more, more preferably 85% or more, and further preferably 90% or more; and the specificity is 70% or more, preferably 75% or more, and more preferably 80% or more. According to the method of the present invention, early-stage colorectal cancer can be detected with extremely high accuracy.

### Examples

The present invention will be specifically described in the following examples. However, these examples are not intended to limit the scope of the present invention.

### Methods

### Serums of colorectal cancer patients and healthy subjects, and colorectal cancer cultured cells

Serum was collected from 51 colorectal cancer patients and 26 healthy subjects (collected at School of Medicine, Chiba University). Each specimen serum sample was preserved at -80°C before use in analysis.

Four colorectal cancer cell lines, namely, HCT116 (ATCC; CCL-247), DLD-1 (ATCC; CCL-221), SW480 (ATCC; CCL-228), and SW620 (ATCC; CCL-227) were cultured in an RPMI-1640 medium (Gibco Laboratories) supplemented with 10% fetal bovine serum (FBS) and antibiotics.

The cells of each line were cultured in a 5% CO₂ incubator, while the temperature was maintained at 37°C, until the cells have grown and have become sub-confluent. Thereafter, the cultured cells were washed with a FBS-free medium, and a fresh FBS-free medium was then added thereto. The thus obtained cells were further cultured in the incubator for 48 hours. Thereafter, the conditioned supernatant medium was gathered and was used as a sample for use in isolation of EV (extracellular vesicles).

### Isolation of EV (extracellular vesicles)

Isolation of EV was carried out according to an ultracentrifugation method. A sucrose buffer was placed on the bottom of a centrifuge tube, so that low-density contaminants can be efficiently separated upon the recovery of EV. As a treatment performed before ultracentrifugation, 100 µl of the serum was centrifuged at 300 g for 10 minutes to remove large contaminants. Thereafter, the recovered serum was passed through a 0.22-µm spin filter (Agilent Technologies, Santa Clara, CA), and was then centrifuged with a 30% sucrose/D₂O buffer at 100,000 g for 90 minutes. The buffer was recovered, and was further subjected to an ultracentrifuge at 100,000 g for 70 minutes twice, and an EV fraction deposited on the centrifuge tube was then recovered.

### Extraction of protein from EV and digestion thereof

Extraction of a protein and the subsequent digestion thereof were carried out in accordance with phase transfer surfactant (PTS) protocols (Masuda T et al. J. Proteome Res. 7, 731-740 (2008)). The EV fraction protein was lysed using an MPEX PTS reagent kit (GL Science, Japan), and dithiothreitol was then added thereto to a final concentration of 5 mM. The thus obtained mixture was subjected to a reduction reaction at room temperature for 30 minutes, and iodoacetamide was further added to the reaction mixture to a final concentration of 20 mM, followed by an alkylation reaction.

Thereafter, 1% (w/w) trypsin (proteomics grade; Roche Mannheim, Germany) was added to the sample, and the obtained mixture was digested at 37°C overnight. After completion of the digestion, ethyl acetate in an amount equal to the liquid amount and trifluoroacetic acid (final concentration: 1%) were added to the resultant, and the obtained mixture was then stirred using Vortex, so that the surfactant contained in the liquid was separated into an organic layer. After completion of the centrifugation, a water phase containing the peptide was recovered, and desalination was then carried out using Stage Tips (Rappsilber J. et al., Nat. Protoc. 2, 1896-1906 (2007)).

### Liquid chromatography (LC)-mass spectrometer (MS/MS) and data analysis of proteome

The digested peptide was fractionated into 7 fractions, using a C18-SCX StageTip chromatography column. Thereafter, analysis was carried out using a mass spectrometer (Q-Exactive (Thermo Scientific, Bremen, Germany)) connected with LC (UltiMate3000 Nanoflow HPLC system (Dionex, Sunnyvale, CA)). The sample was introduced into the mass spectrometer by using the present inventors' own making analysis column, in which a 1.9-µm C 18-AQ resin was enclosed in a needle with an inner diameter of 75 µm and a length of 300 mm. The mobile phase of LC was composed of buffer A (0.1% formic acid and 2% acetonitrile) and buffer B (0.1% formic acid and 90% acetonitrile). The digested peptide was dissolved in the buffer A, and was then loaded on a trap column (0.075 x 20 mm, Acclaim PepMap RSLC Nano-Trap Column; Thermo Scientific). Regarding Nano LC, the solution was supplied at a rate of 280 nL/min, and the mobile phase was developed with a gradient from 5% to 35% buffer B for 120 minutes. Regarding mass spectrometry, the measurement was carried out with Full MS scan (350 to 1800m/z, ion integration: 3 x 10⁶, resolution: 70,000) and MS/MS scan (Top10 precursor ion, injection time: 120 ms, resolution: 35,000, MS/MS ion selection threshold : 5 x 10⁴ counts, separation width: 3.0 Da). The data file was analyzed using MaxQuant software (Ver 1.5.1.2). Andromeda was used as a search engine, and a peak list was searched against UniProt human protein database. The precursor mass error range was set to be 7 ppm, and the fragment ion mass error range was set to be 0.01 Da. The identified protein and peptide were determined with a false positive rate of less than 1% (FDR 1% >) with respect to the reverse database.

### LC-SRM/MRM analysis

LC-SRM/MRM analysis was carried out according to the previously reported method (Kume, H. et al., Mol. Cell. Proteomics 13, 1471-1484 (2014): Muraoka, S. et al., J. Proteome Res. 11, 4201-4210 (2012): Narumi, R. et al., J. Proteome Res. 11, 5311-5322 (2012)). The digested peptide was dissolved in a 2% acetonitrile solution containing 0.1% trifluoroacetic acid (TFA), and thereafter, it was analysis by using a triple quadrupole mass spectrometer TSQ-Vantage (Thermo Fisher Scientific, Bremen, Germany) connected with NanoFlow LC Paradigm MS2 (Michrom BioResources, Auburn, CA). The sample was introduced into the mass spectrometer by using the present inventors' own making analysis column, in which a 1.9-µm C18-AQ resin was enclosed in a needle with an inner diameter of 75 µm and a length of 100 mm. The mobile phase of LC was composed of buffer A (0.1% formic acid and 2% acetonitrile) and buffer B (0.1% formic acid and 90% acetonitrile). The digested peptide was dissolved in the buffer A, and was then loaded on a trap column (0.075 x 20 mm, Acclaim PepMap RSLC Nano-Trap Column; Thermo Scientific). Regarding Nano LC, the solution was supplied at a rate of 280 nL/min, and the mobile phase was developed with a gradient from 5% to 35% buffer B for 60 minutes. The analysis was carried out at an SRM mode under the following conditions (Q1 Peak Width 0.7 FWHM; Cycle time: 1 sec; and Collision Gass Pressure 1.8 mTorr). The collision energy was optimized for every SRM transition, and the intensity of each transition was measured at a schedule mode of a peak time width of 5 minutes.

### Quantification of target peptide using SI-peptide

With regard to quantification of a target peptide according to SRM, a stable isotope-labeled peptide (SI-peptide) having the same sequence as that of the target peptide was mixed as an internal standard with EV prepared from each specimen serum, and thereafter, the amount was calculated based on the peak area ratio of the endogenous peptide/the SI-peptide detected according to the SRM analysis. The spike amount of the SI-peptide having the same sequence as that of the target peptide was adjusted to be close to that of the endogenous peptide by performing a pre-analysis.

### Statistical analysis of peak area ratio according to SRM analysis

In order to evaluate the target peptide as a colorectal biomarker based on the peak area ratio of each specimen calculated according to the SRM analysis, the statistical analysis of the SRM data was carried out. For the statistical analysis, SPSS software (Ver. 23) (SPSS Inc., Chicago, IL) was used. First, regarding the evaluation of a single target peptide as a biomarker, the value of AUC (Area under the Curve) was calculated according to the ROC analysis (ROC: Receiver Operating Characteristic). Furthermore, a plurality of target peptides were combined with one another, and the thus combined target peptides were also evaluated as multiple markers. For the combination of the target peptides, a logistic regression model was prepared using SPSS software, and was then evaluated. A combination of peptides, for which the model was effective, was also evaluated with the value of AUC calculated according to the ROC analysis.

### Results

### Searching for CRC-related biomarker candidate proteins

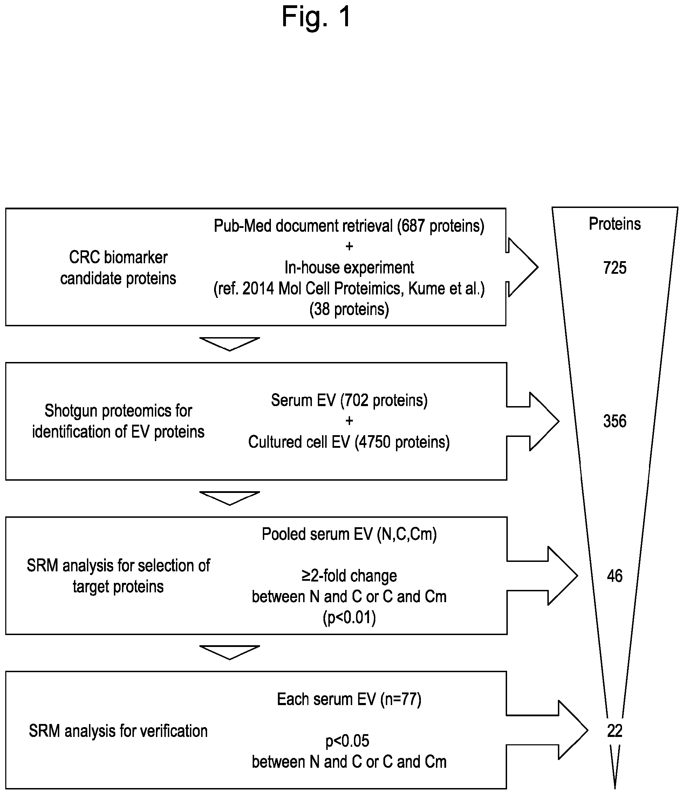

Figure 1 shows a strategy for searching for the biomarker candidates of the present case. A list of CRC biomarker candidate proteins was acquired from PubMed Database regarding medical and biological publications. As a retrieval style, "cancer" AND "colorectal" AND "expression" was used, and document retrieval was performed on the database from 2003 to 2014, and as a result, 687 colorectal cancer-related proteins were listed up as CRC biomarker candidates. The candidate proteins were selected in accordance with the following criteria (1) to (4).
(1) The expression of a protein in human CRC tissues or blood has been confirmed according to Western blot, ELISA, or immunohistochemistry.
(2) An increase in the expression of a target protein has been confirmed in cancer. (Since a protein with an increased expression is detected more easily than a protein with a decreased expression, it is suitable as a biomarker.)
(3) The molecular function of a target protein relevant to the expression and progression of cancer has been experimentally confirmed by RNAi or the overexpression of molecules.
(4) Proteins specified only by large-scale analysis (e.g., omics analysis) have been excluded.

Moreover, the present inventors had previously specified 44 CRC biomarker candidate proteins by performing targeted proteomics on clinical specimens (Kume, H. et al., Mol. Cell. Proteomics 13, 1471-1484 (2014)). These 44 proteins were gathered with the above-described biomarker candidates, and then, overlapped proteins were excluded therefrom. The thus obtained total 725 proteins were selected as CRC biomarker candidates.

### Selection of biomarker candidates contained in EV according to shotgun proteomics analysis

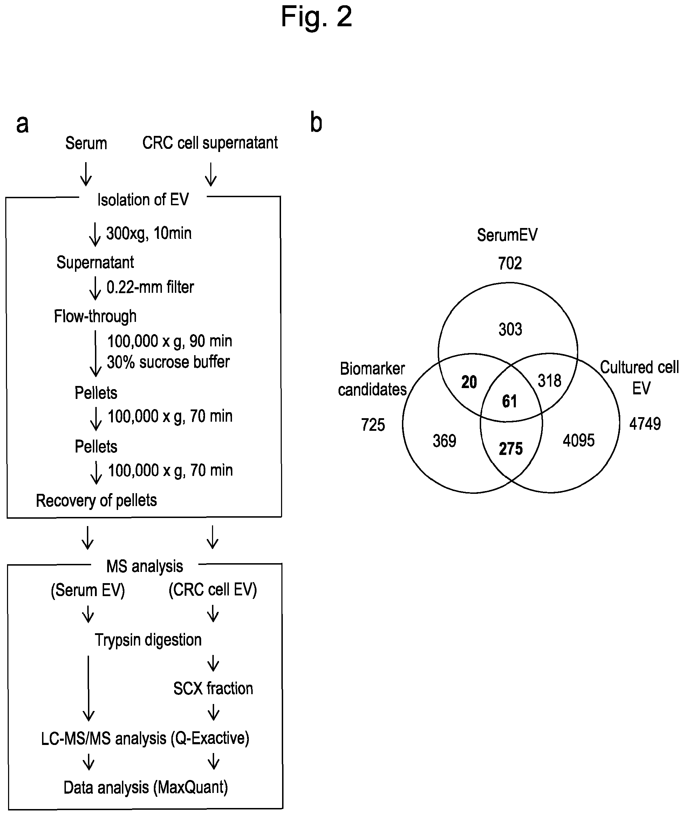

In order to select proteins present in EV from the selected biomarker candidate proteins, EV prepared from serum and cultured cell supernatant was used, and the qualitative analysis of EV proteins was carried out according to shotgun proteomics analysis. A work flow of the shotgun proteomics is shown in Figure 2a.

Preparation of EV from serum and cell supernatant was carried out according to an ultracentrifugation method. Serum EV was collected from 8 healthy subjects, 8 non-metastatic cancer patients, and 8 metastatic cancer patients. A cultured cell supernatant was collected from each of 4 types of CRC cells (HCT116, DLD-1, SW480, and SW620). The extracted proteins were digested according to a phase transfer solubilizer (PTS) method, and were then fractionated using a C-18 Stage SCX Tip column (Masuda, T et al., J. Proteome Res. 7, 731-740 (2008); Adachi, J. et al. Anal. Chem. 88, 7899-7903 (2016)). The fraction samples were analyzed according to LC-MS/MS, and then, as a result of Mascot database searching, 702 proteins (serum EV) and 4749 proteins (culture supernatant EV) were identified. By comparing these EV identified proteins with the biomarker candidate proteins, 356 proteins were identified as EV protein-containing biomarker candidates (Figure 2b).

### Selection of SRM target peptides from biomarker candidate proteins identified in EV

In order to verify the effectiveness of the candidate proteins as biomarkers, EV fractions collected from CRC patient serum were subjected to the SRM analysis. SRM candidate peptides were selected from the peptides specified by shotgun proteomics. The candidate peptides were selected in accordance with the following criteria (1) to (3).
(1) The identified proteins had peptide sequences specific to proteins. When there were multiple peptides, targets having high intensity were selected according to shotgun analysis.
(2) Peptides having a cleavage mistake with trypsin or an amino acid modification that was not suitable for the SRM analysis (e.g., oxidized methionine, etc.) were excluded.
(3) In the case of too long peptides (> 20 amino acids), it was difficult to synthesize a stable isotope-labeled peptide (SI-peptide). Thus, such too long peptides were excluded from target peptides.

Among all of the identified peptides, 3316 peptides (i.e., 346 proteins) satisfied these criteria.

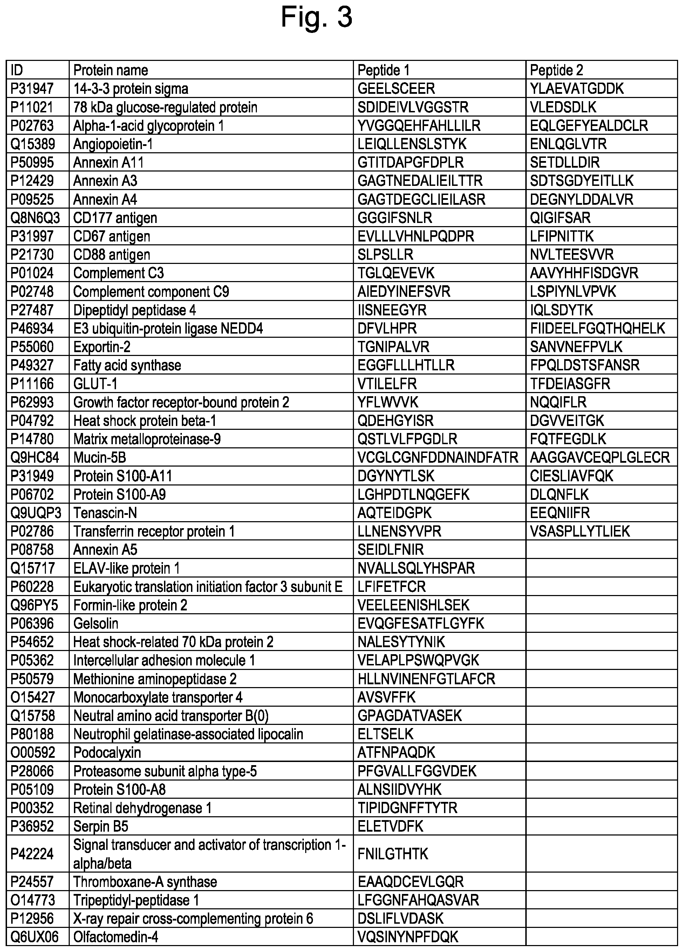

Subsequently, in order to select SRM target peptides from these candidates, EV fractions were prepared from the pooled serum of healthy subjects (N) as non-cancerous controls (N = 26), the pooled serum of non-metastatic cancer patients (C) (N = 26), and the pooled serum of metastatic cancer patients (Cm) (N = 25), and were then used in the SRM analysis. From the results of the shotgun analysis, 3 or 4 transitions (a pair of a precursor ion and a product ion) were selected for a single peptide. In the SRM analysis, the measurement was repeated three times for each pool, and the peptide peak area was quantified using Skyline software. A target peptide, the peak area of which was significantly (2-fold or more) increased (p < 0.01) between the pooled samples (between N and C, or between C and Cm), was selected as a marker candidate peptide. Regarding a protein in which two or more candidates peptides were detected, two peptides having high peak intensity according to the SRM analysis were selected. Considering these criteria, 71 peptides (46 proteins) were selected as target peptides serving as biomarker candidates (Figure 3).

### Quantification of target peptides in serum EV fraction according to SRM

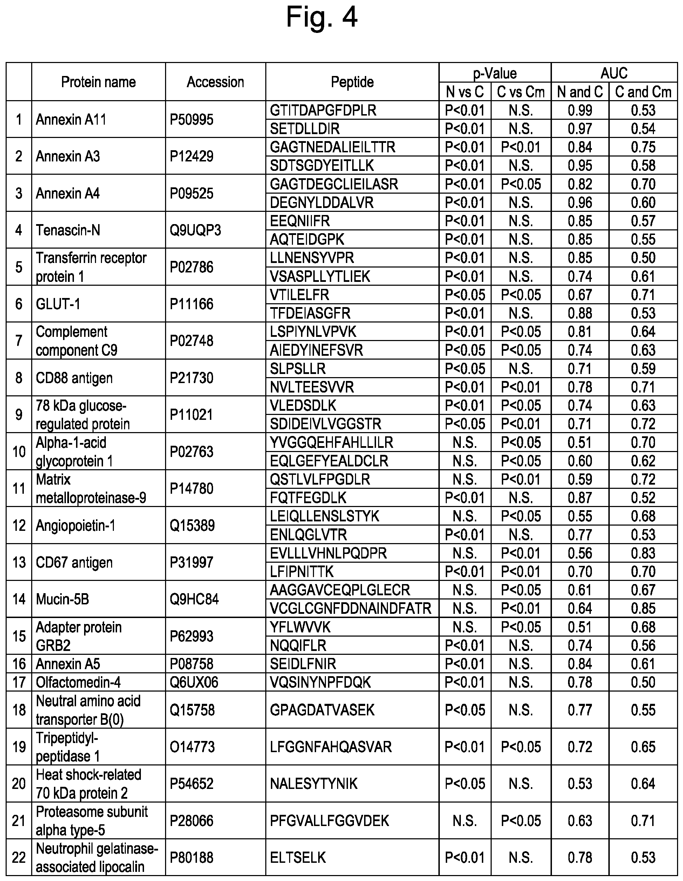

37 Biomarker candidate peptides in the EV fractions prepared from the serums of individual patients were subjected to quantitative analysis according to SRM. Individual EV fractions were prepared from the patient serums of three groups (healthy subjects (N), n = 26; non-metastatic cancer patients (C), n = 26; and metastatic cancer patients (Cm), n = 25). An equal amount of SI-peptide was added as an internal standard to each specimen, and the peak area ratio of the SI-peptide to the endogenous target peptide was calculated using Skyline software. Regarding each target peptide, the quantitative values were compared among the groups N, C, and Cm according to a t-test. As a result, a significant increase in the quantitative values was observed between N and C, or between C and Cm (p < 0.05) (Figure 4 and Figures 5-1, 5-2, 5-3, 5-4, 5-5 and 5-6).

Evaluation of target peptides as biomarkers according to statistical analysis

In order to evaluate the candidate peptides as biomarkers, 37 peptides were subjected to ROC (Receiver Operating Characteristic) analysis. Regarding individual candidate peptides, the distinguishing between N and C, and between C and Cm was evaluated. Four peptides (3 proteins) were found to be proteins having extremely high sensitivity (AUC > 0.9, Figures 6-1a and b). Also, 22 peptides were found to have high sensitivity that was sufficient for distinguishing between N and C (0.7-0.9 AUC, Figures 7-1, 7-2, 7-3, 7-4, 7-5, and 7-6). On the other hand, 11 peptides had high sensitivity that was sufficient for distinguishing between C and Cm (Figures 6-2c and d).

Subsequently, whether or not multiple markers obtained by combining candidate peptides with one another had high sensitivity was examined according to a logistic regression analysis. Significant combinations of peptides were evaluated using SPSS software. The results of the logistic analysis using two or more peptides were evaluated based on the AUC values of the ROC curves of the multiple markers. Regarding 14 combinations of candidate peptides, their AUC values were higher than the AUC value of a marker alone (Figures 8-1 and 8-2), and in particular, regarding 8 combinations, the AUC value was 0.9 < (Figure 9). The highest AUC value (0.97) was obtained by a combination of three peptides (Transferrin receptor protein 1, Neutrophil gelatinase-associated lipocalin, and Angiopoietin-1). These results suggest that a combination of multiple markers be effective for improving the accuracy of the diagnosis of colorectal cancer.

### Comparison between broadly used tumor marker (carcinoembryonic antigen CEA) and target peptide in terms of sensitivity

Today, CEA is one of most broadly used blood markers for colorectal cancer. However, the sensitivity of CEA is not sufficient for discovering early-stage patients, and the sensitivity of CEA to patients with stage 2 has been reported to be approximately 30%. Hence, newly established 37 biomarker candidates were compared with CEA. Since CEA specificity was almost 100%, the cutoff point of the SRM quantitative value of each target peptide was set to be a specificity of 100%. The sensitivity of CEA to specimen group C used in verification was 38.8%. On the other hand, among the 37 target peptides, 17 peptides (12 proteins; namely, Annexin A3, Annexin A4, Annexin A5, Annexin A11, Tenascin-N, Transferrin receptor protein 1, GLUT-1, Matrix metalloproteinase-9, Olfactomedin-4, CD88 antigen, Tripeptidyl-peptidase 1, and Neutrophil gelatinase-associated lipocalin) exhibited sensitivity that was significantly higher than the sensitivity of CEA (Figures 10-1, 10-2, and 10-3). In particular, the three peptides of Annexin A4 and A11 exhibited sensitivity of 80% < (Figure 11).

### Industrial Applicability

According to the present invention, colorectal cancer can be detected in an early stage.

### Sequence Listing Free Text

### SEQ ID NOS: 23 to 59 Synthetic peptides

All publications, patents and patent applications cited in the present description are incorporated herein by reference in their entirety.

## Claims

1. A colorectal cancer biomarker for detecting colorectal cancer, wherein the biomarker consists of at least one protein of the following 22 proteins with numbers 1 to 22, or at least one peptide of the partial peptides of the proteins with numbers 1 to 22:
1. Annexin A11 (ANXA 11) (SEQ ID NO: 1);
2. Annexin A3 (ANXA 3) (SEQ ID NO: 2);
3. Annexin A4 (ANXA 4) (SEQ ID NO: 3);
4. Tanascin-N (TNN) (SEQ ID NO: 4);
5. Transferrin receptor protein 1 (TFRC) (SEQ ID NO: 5);
6. Glucose transporter 1(GLUT-1) (SLC2A1) (SEQ ID NO: 6);
7. Complement component C9 (C9) (SEQ ID NO: 7);
8. CD88 antigen (C5AR1) (SEQ ID NO: 8);
9. 78 kDa glucose-regulated protein (HSPA5) (SEQ ID NO: 9);
10. α-1-acid glycoprotein (ORM1) (SEQ ID NO: 10);
11. Matrix metalloproteinase-9 (MMP9) (SEQ ID NO: 11);
12. Angiopoietin-1 (ANGPT1) (SEQ ID NO: 12);
13. CD67 antigen (CEACAM8) (SEQ ID NO: 13);
14. Mucin-5B (MUC5B) (SEQ ID NO: 14);
15. Adapter protein GRB2 (GRB2) (SEQ ID NO: 15);
16. Annexin A5 (Annexin A5) (ANXA 5) (SEQ ID NO: 16);
17. Olfactomedin-4 (OLFM4) (SEQ ID NO: 17);
18. Neutral amino acid transporter B(0) (SLC1A5) (SEQ ID NO: 18);
19. Tripeptidyl-peptidase 1 (TPP1) (SEQ ID NO: 19);
20. Heat shock-related 70 kDa protein 2 (HSPA2) (SEQ ID NO: 20);
21. Proteasome subunit α type-5 (PSMA5) (SEQ ID NO: 21); or
22. Neutrophil gelatinase-associated lipocalin (LCN2) (SEQ ID NO: 22).

2. The colorectal cancer biomarker for detecting colorectal cancer according to claim 1, which consists of a combination of two or more proteins of the 22 proteins with numbers 1 to 22 according to claim 1, or two or more peptides of the partial peptides of the proteins with numbers 1 to 22.

3. The colorectal cancer biomarker for detecting colorectal cancer according to claim 1 or 2, wherein the partial peptides of the proteins with numbers 1 to 22 according to claim 1 are peptides consisting of the amino acid sequences as set forth in SEQ ID NOS: 23 to 59.

4. The colorectal cancer biomarker for detecting colorectal cancer according to claim 1, which consists of at least one protein of the following 12 proteins, or at least one peptide of the partial peptides of the 12 proteins:
1. Annexin A11 (SEQ ID NO: 1);
2. Annexin A3 (SEQ ID NO: 2);
3. Annexin A4 (SEQ ID NO: 3);
4. Tanascin-N (SEQ ID NO: 4);
5. Transferrin receptor protein 1 (SEQ ID NO: 5);
6. Glucose transporter 1 (SEQ ID NO: 6);
8. CD88 antigen (SEQ ID NO: 8);
11. Matrix metalloproteinase-9 (SEQ ID NO: 11);
16. Annexin A5 (SEQ ID NO: 16);
17. Olfactomedin-4 (SEQ ID NO: 17);
19. Tripeptidyl-peptidase 1 (SEQ ID NO: 19); or
22. Neutrophil gelatinase-associated lipocalin (SEQ ID NO: 22).

5. The colorectal cancer biomarker for detecting colorectal cancer according to claim 4, which consists of a combination of two or more proteins of the 12 proteins according to claim 4, or two or more peptides of the partial peptides of the 12 proteins.

6. The colorectal cancer biomarker for detecting colorectal cancer according to claim 4 or 5, wherein the partial peptides of the 12 proteins according to claim 4 are peptides consisting of the amino acid sequences as set forth in SEQ ID NOS: 23 to 34, 37, 38, 43, 44, 53, 54, 56 and 59.

7. The colorectal cancer biomarker for detecting colorectal cancer according to claim 1, which consists of Annexin A4 or Annexin A11, or a partial peptide thereof.

8. The colorectal cancer biomarker for detecting colorectal cancer according to claim 7, which consists of a combination of Annexin A4 and Annexin A11, or partial peptides of Annexin A4 and partial peptides of Annexin A11.

9. The colorectal cancer biomarker for detecting colorectal cancer according to claim 7 or 8, wherein the partial peptides of Annexin A4 and Annexin A11 are peptides consisting of the amino acid sequences as set forth in SEQ ID NOS: 23, 24, 27 and 28.

10. A colorectal cancer biomarker for detecting colorectal cancer, which consists of a combination of the colorectal cancer biomarker according to any one of claims 1 to 9 and CEA.

11. A method of detecting colorectal cancer, comprising measuring the colorectal cancer biomarker according to any one of claims 1 to 10 in a biological sample.

12. A method of detecting colorectal cancer, comprising measuring the colorectal cancer biomarker according to any one of claims 1 to 10 in a biological sample, and then determining that the subject is affected with colorectal cancer when the biomarker is present in a higher concentration in the biological sample than in a healthy subject.

13. The method of detecting colorectal cancer according to claim 11 or 12, wherein the biological sample is an extracellular vesicle (EV) in blood.

14. The method of detecting colorectal cancer according to any one of claims 11 to 13, wherein the detection is carried out by an immunoassay or a mass spectrometry.

15. A kit of detecting colorectal cancer, comprising an antibody reacting against the colorectal cancer biomarker according to any one of claims 1 to 10 in the biological sample.


SEQUENCE LISTING

<110>  National Institutes of Biomedical Innovation, Health and
       Nutrition
       DENKA SEIKEN Co., Ltd.

<120>  A biomarker for detecting colorectal cancer

<130>  PH-7502-PCT

<150>  JP 2017-129941
<151>  2017-06-30

<160>  59

<170>  PatentIn version 3.5

<210>  1
<211>  505
<212>  PRT
<213>  Homo sapiens

<400>  1

<210>  2
<211>  323
<212>  PRT
<213>  Homo sapiens

<400>  2

Met Ala Ser Ile Trp Val Gly His Arg Gly Thr Val Arg Asp Tyr Pro
1               5                   10                  15

Asp Phe Ser Pro Ser Val Asp Ala Glu Ala Ile Gln Lys Ala Ile Arg
            20                  25                  30

Gly Ile Gly Thr Asp Glu Lys Met Leu Ile Ser Ile Leu Thr Glu Arg
        35                  40                  45

Ser Asn Ala Gln Arg Gln Leu Ile Val Lys Glu Tyr Gln Ala Ala Tyr
    50                  55                  60

Gly Lys Glu Leu Lys Asp Asp Leu Lys Gly Asp Leu Ser Gly His Phe
65                  70                  75                  80

Glu His Leu Met Val Ala Leu Val Thr Pro Pro Ala Val Phe Asp Ala
                85                  90                  95

Lys Gln Leu Lys Lys Ser Met Lys Gly Ala Gly Thr Asn Glu Asp Ala
            100                 105                 110

Leu Ile Glu Ile Leu Thr Thr Arg Thr Ser Arg Gln Met Lys Asp Ile
        115                 120                 125

Ser Gln Ala Tyr Tyr Thr Val Tyr Lys Lys Ser Leu Gly Asp Asp Ile
    130                 135                 140

Ser Ser Glu Thr Ser Gly Asp Phe Arg Lys Ala Leu Leu Thr Leu Ala
145                 150                 155                 160

Asp Gly Arg Arg Asp Glu Ser Leu Lys Val Asp Glu His Leu Ala Lys
                165                 170                 175

Gln Asp Ala Gln Ile Leu Tyr Lys Ala Gly Glu Asn Arg Trp Gly Thr
            180                 185                 190

Asp Glu Asp Lys Phe Thr Glu Ile Leu Cys Leu Arg Ser Phe Pro Gln
        195                 200                 205

Leu Lys Leu Thr Phe Asp Glu Tyr Arg Asn Ile Ser Gln Lys Asp Ile
    210                 215                 220

Val Asp Ser Ile Lys Gly Glu Leu Ser Gly His Phe Glu Asp Leu Leu
225                 230                 235                 240

Leu Ala Ile Val Asn Cys Val Arg Asn Thr Pro Ala Phe Leu Ala Glu
                245                 250                 255

Arg Leu His Arg Ala Leu Lys Gly Ile Gly Thr Asp Glu Phe Thr Leu
            260                 265                 270

Asn Arg Ile Met Val Ser Arg Ser Glu Ile Asp Leu Leu Asp Ile Arg
        275                 280                 285

Thr Glu Phe Lys Lys His Tyr Gly Tyr Ser Leu Tyr Ser Ala Ile Lys
    290                 295                 300

Ser Asp Thr Ser Gly Asp Tyr Glu Ile Thr Leu Leu Lys Ile Cys Gly
305                 310                 315                 320

Gly Asp Asp

<210>  3
<211>  319
<212>  PRT
<213>  Homo sapiens

<400>  3

Met Ala Thr Lys Gly Gly Thr Val Lys Ala Ala Ser Gly Phe Asn Ala
1               5                   10                  15

Met Glu Asp Ala Gln Thr Leu Arg Lys Ala Met Lys Gly Leu Gly Thr
            20                  25                  30

Asp Glu Asp Ala Ile Ile Ser Val Leu Ala Tyr Arg Asn Thr Ala Gln
        35                  40                  45

Arg Gln Glu Ile Arg Thr Ala Tyr Lys Ser Thr Ile Gly Arg Asp Leu
    50                  55                  60

Ile Asp Asp Leu Lys Ser Glu Leu Ser Gly Asn Phe Glu Gln Val Ile
65                  70                  75                  80

Val Gly Met Met Thr Pro Thr Val Leu Tyr Asp Val Gln Glu Leu Arg
                85                  90                  95

Arg Ala Met Lys Gly Ala Gly Thr Asp Glu Gly Cys Leu Ile Glu Ile
            100                 105                 110

Leu Ala Ser Arg Thr Pro Glu Glu Ile Arg Arg Ile Ser Gln Thr Tyr
        115                 120                 125

Gln Gln Gln Tyr Gly Arg Ser Leu Glu Asp Asp Ile Arg Ser Asp Thr
    130                 135                 140

Ser Phe Met Phe Gln Arg Val Leu Val Ser Leu Ser Ala Gly Gly Arg
145                 150                 155                 160

Asp Glu Gly Asn Tyr Leu Asp Asp Ala Leu Val Arg Gln Asp Ala Gln
                165                 170                 175

Asp Leu Tyr Glu Ala Gly Glu Lys Lys Trp Gly Thr Asp Glu Val Lys
            180                 185                 190

Phe Leu Thr Val Leu Cys Ser Arg Asn Arg Asn His Leu Leu His Val
        195                 200                 205

Phe Asp Glu Tyr Lys Arg Ile Ser Gln Lys Asp Ile Glu Gln Ser Ile
    210                 215                 220

Lys Ser Glu Thr Ser Gly Ser Phe Glu Asp Ala Leu Leu Ala Ile Val
225                 230                 235                 240

Lys Cys Met Arg Asn Lys Ser Ala Tyr Phe Ala Glu Lys Leu Tyr Lys
                245                 250                 255

Ser Met Lys Gly Leu Gly Thr Asp Asp Asn Thr Leu Ile Arg Val Met

260                 265                 270

Val Ser Arg Ala Glu Ile Asp Met Leu Asp Ile Arg Ala His Phe Lys
        275                 280                 285

Arg Leu Tyr Gly Lys Ser Leu Tyr Ser Phe Ile Lys Gly Asp Thr Ser
    290                 295                 300

Gly Asp Tyr Arg Lys Val Leu Leu Val Leu Cys Gly Gly Asp Asp
305                 310                 315

<210>  4
<211>  1299
<212>  PRT
<213>  Homo sapiens

<400>  4

Met Ser Leu Gln Glu Met Phe Arg Phe Pro Met Gly Leu Leu Leu Gly
1               5                   10                  15

Ser Val Leu Leu Val Ala Ser Ala Pro Ala Thr Leu Glu Pro Pro Gly
            20                  25                  30

Cys Ser Asn Lys Glu Gln Gln Val Thr Val Ser His Thr Tyr Lys Ile
        35                  40                  45

Asp Val Pro Lys Ser Ala Leu Val Gln Val Asp Ala Asp Pro Gln Pro
    50                  55                  60

Leu Ser Asp Asp Gly Ala Ser Leu Leu Ala Leu Gly Glu Ala Arg Glu
65                  70                  75                  80

Glu Gln Asn Ile Ile Phe Arg His Asn Ile Arg Leu Gln Thr Pro Gln
                85                  90                  95

Lys Asp Cys Glu Leu Ala Gly Ser Val Gln Asp Leu Leu Ala Arg Val
            100                 105                 110

Lys Lys Leu Glu Glu Glu Met Val Glu Met Lys Glu Gln Cys Ser Ala
        115                 120                 125

Gln Arg Cys Cys Gln Gly Val Thr Asp Leu Ser Arg His Cys Ser Gly
    130                 135                 140

His Gly Thr Phe Ser Leu Glu Thr Cys Ser Cys His Cys Glu Glu Gly
145                 150                 155                 160

Arg Glu Gly Pro Ala Cys Glu Arg Leu Ala Cys Pro Gly Ala Cys Ser

                165                 170                 175

Gly His Gly Arg Cys Val Asp Gly Arg Cys Leu Cys His Glu Pro Tyr
            180                 185                 190

Val Gly Ala Asp Cys Gly Tyr Pro Ala Cys Pro Glu Asn Cys Ser Gly
        195                 200                 205

His Gly Glu Cys Val Arg Gly Val Cys Gln Cys His Glu Asp Phe Met
    210                 215                 220

Ser Glu Asp Cys Ser Glu Lys Arg Cys Pro Gly Asp Cys Ser Gly His
225                 230                 235                 240

Gly Phe Cys Asp Thr Gly Glu Cys Tyr Cys Glu Glu Gly Phe Thr Gly
                245                 250                 255

Leu Asp Cys Ala Gln Val Val Thr Pro Gln Gly Leu Gln Leu Leu Lys
            260                 265                 270

Asn Thr Glu Asp Ser Leu Leu Val Ser Trp Glu Pro Ser Ser Gln Val
        275                 280                 285

Asp His Tyr Leu Leu Ser Tyr Tyr Pro Leu Gly Lys Glu Leu Ser Gly
    290                 295                 300

Lys Gln Ile Gln Val Pro Lys Glu Gln His Ser Tyr Glu Ile Leu Gly
305                 310                 315                 320

Leu Leu Pro Gly Thr Lys Tyr Ile Val Thr Leu Arg Asn Val Lys Asn
                325                 330                 335

Glu Val Ser Ser Ser Pro Gln His Leu Leu Ala Thr Thr Asp Leu Ala
            340                 345                 350

Val Leu Gly Thr Ala Trp Val Thr Asp Glu Thr Glu Asn Ser Leu Asp
        355                 360                 365

Val Glu Trp Glu Asn Pro Ser Thr Glu Val Asp Tyr Tyr Lys Leu Arg
    370                 375                 380

Tyr Gly Pro Met Thr Gly Gln Glu Val Ala Glu Val Thr Val Pro Lys
385                 390                 395                 400

Ser Ser Asp Pro Lys Ser Arg Tyr Asp Ile Thr Gly Leu His Pro Gly
                405                 410                 415

Thr Glu Tyr Lys Ile Thr Val Val Pro Met Arg Gly Glu Leu Glu Gly
                420                 425                 430

Lys Pro Ile Leu Leu Asn Gly Arg Thr Glu Ile Asp Ser Pro Thr Asn
            435                 440                 445

Val Val Thr Asp Arg Val Thr Glu Asp Thr Ala Thr Val Ser Trp Asp
    450                 455                 460

Pro Val Gln Ala Val Ile Asp Lys Tyr Val Val Arg Tyr Thr Ser Ala
465                 470                 475                 480

Asp Gly Asp Thr Lys Glu Met Ala Val His Lys Asp Glu Ser Ser Thr
                485                 490                 495

Val Leu Thr Gly Leu Lys Pro Gly Glu Ala Tyr Lys Val Tyr Val Trp
            500                 505                 510

Ala Glu Arg Gly Asn Gln Gly Ser Lys Lys Ala Asp Thr Asn Ala Leu
        515                 520                 525

Thr Glu Ile Asp Ser Pro Ala Asn Leu Val Thr Asp Arg Val Thr Glu
    530                 535                 540

Asn Thr Ala Thr Ile Ser Trp Asp Pro Val Gln Ala Thr Ile Asp Lys
545                 550                 555                 560

Tyr Val Val Arg Tyr Thr Ser Ala Asp Asp Gln Glu Thr Arg Glu Val
                565                 570                 575

Leu Val Gly Lys Glu Gln Ser Ser Thr Val Leu Thr Gly Leu Arg Pro
            580                 585                 590

Gly Val Glu Tyr Thr Val His Val Trp Ala Gln Lys Gly Asp Arg Glu
        595                 600                 605

Ser Lys Lys Ala Asp Thr Asn Ala Pro Thr Asp Ile Asp Ser Pro Lys
    610                 615                 620

Asn Leu Val Thr Asp Arg Val Thr Glu Asn Met Ala Thr Val Ser Trp
625                 630                 635                 640

Asp Pro Val Gln Ala Ala Ile Asp Lys Tyr Val Val Arg Tyr Thr Ser
                645                 650                 655

Ala Gly Gly Glu Thr Arg Glu Val Pro Val Gly Lys Glu Gln Ser Ser
            660                 665                 670

Thr Val Leu Thr Gly Leu Arg Pro Gly Met Glu Tyr Met Val His Val
        675                 680                 685

Trp Ala Gln Lys Gly Asp Gln Glu Ser Lys Lys Ala Asp Thr Lys Ala
    690                 695                 700

Gln Thr Asp Ile Asp Ser Pro Gln Asn Leu Val Thr Asp Arg Val Thr
705                 710                 715                 720

Glu Asn Met Ala Thr Val Ser Trp Asp Pro Val Arg Ala Thr Ile Asp
                725                 730                 735

Arg Tyr Val Val Arg Tyr Thr Ser Ala Lys Asp Gly Glu Thr Arg Glu
            740                 745                 750

Val Pro Val Gly Lys Glu Gln Ser Ser Thr Val Leu Thr Gly Leu Arg
        755                 760                 765

Pro Gly Val Glu Tyr Thr Val His Val Trp Ala Gln Lys Gly Ala Gln
    770                 775                 780

Glu Ser Lys Lys Ala Asp Thr Lys Ala Gln Thr Asp Ile Asp Ser Pro
785                 790                 795                 800

Gln Asn Leu Val Thr Asp Trp Val Thr Glu Asn Thr Ala Thr Val Ser
                805                 810                 815

Trp Asp Pro Val Gln Ala Thr Ile Asp Arg Tyr Val Val His Tyr Thr
            820                 825                 830

Ser Ala Asn Gly Glu Thr Arg Glu Val Pro Val Gly Lys Glu Gln Ser
        835                 840                 845

Ser Thr Val Leu Thr Gly Leu Arg Pro Gly Met Glu Tyr Thr Val His
    850                 855                 860

Val Trp Ala Gln Lys Gly Asn Gln Glu Ser Lys Lys Ala Asp Thr Lys
865                 870                 875                 880

Ala Gln Thr Glu Ile Asp Gly Pro Lys Asn Leu Val Thr Asp Trp Val
                885                 890                 895

Thr Glu Asn Met Ala Thr Val Ser Trp Asp Pro Val Gln Ala Thr Ile
            900                 905                 910

Asp Lys Tyr Met Val Arg Tyr Thr Ser Ala Asp Gly Glu Thr Arg Glu
        915                 920                 925

Val Pro Val Gly Lys Glu His Ser Ser Thr Val Leu Thr Gly Leu Arg
    930                 935                 940

Pro Gly Met Glu Tyr Met Val His Val Trp Ala Gln Lys Gly Ala Gln
945                 950                 955                 960

Glu Ser Lys Lys Ala Asp Thr Lys Ala Gln Thr Glu Leu Asp Pro Pro
                965                 970                 975

Arg Asn Leu Arg Pro Ser Ala Val Thr Gln Ser Gly Gly Ile Leu Thr
            980                 985                 990

Trp Thr Pro Pro Ser Ala Gln Ile His Gly Tyr Ile Leu Thr Tyr Gln
        995                 1000                1005

Phe Pro Asp Gly Thr Val Lys Glu Met Gln Leu Gly Arg Glu Asp
    1010                1015                1020

Gln Arg Phe Ala Leu Gln Gly Leu Glu Gln Gly Ala Thr Tyr Pro
    1025                1030                1035

Val Ser Leu Val Ala Phe Lys Gly Gly Arg Arg Ser Arg Asn Val
    1040                1045                1050

Ser Thr Thr Leu Ser Thr Val Gly Ala Arg Phe Pro His Pro Ser
    1055                1060                1065

Asp Cys Ser Gln Val Gln Gln Asn Ser Asn Ala Ala Ser Gly Leu
    1070                1075                1080

Tyr Thr Ile Tyr Leu His Gly Asp Ala Ser Arg Pro Leu Gln Val
    1085                1090                1095

Tyr Cys Asp Met Glu Thr Asp Gly Gly Gly Trp Ile Val Phe Gln
    1100                1105                1110

Arg Arg Asn Thr Gly Gln Leu Asp Phe Phe Lys Arg Trp Arg Ser
    1115                1120                1125

Tyr Val Glu Gly Phe Gly Asp Pro Met Lys Glu Phe Trp Leu Gly
    1130                1135                1140

Leu Asp Lys Leu His Asn Leu Thr Thr Gly Thr Pro Ala Arg Tyr
    1145                1150                1155

Glu Val Arg Val Asp Leu Gln Thr Ala Asn Glu Ser Ala Tyr Ala

1160                1165                1170

Ile Tyr Asp Phe Phe Gln Val Ala Ser Ser Lys Glu Arg Tyr Lys
    1175                1180                1185

Leu Thr Val Gly Lys Tyr Arg Gly Thr Ala Gly Asp Ala Leu Thr
    1190                1195                1200

Tyr His Asn Gly Trp Lys Phe Thr Thr Phe Asp Arg Asp Asn Asp
    1205                1210                1215

Ile Ala Leu Ser Asn Cys Ala Leu Thr His His Gly Gly Trp Trp
    1220                1225                1230

Tyr Lys Asn Cys His Leu Ala Asn Pro Asn Gly Arg Tyr Gly Glu
    1235                1240                1245

Thr Lys His Ser Glu Gly Val Asn Trp Glu Pro Trp Lys Gly His
    1250                1255                1260

Glu Phe Ser Ile Pro Tyr Val Glu Leu Lys Ile Arg Pro His Gly
    1265                1270                1275

Tyr Ser Arg Glu Pro Val Leu Gly Arg Lys Lys Arg Thr Leu Arg
    1280                1285                1290

Gly Arg Leu Arg Thr Phe
    1295

<210>  5
<211>  760
<212>  PRT
<213>  Homo sapiens

<400>  5

Met Met Asp Gln Ala Arg Ser Ala Phe Ser Asn Leu Phe Gly Gly Glu
1               5                   10                  15

Pro Leu Ser Tyr Thr Arg Phe Ser Leu Ala Arg Gln Val Asp Gly Asp
            20                  25                  30

Asn Ser His Val Glu Met Lys Leu Ala Val Asp Glu Glu Glu Asn Ala
        35                  40                  45

Asp Asn Asn Thr Lys Ala Asn Val Thr Lys Pro Lys Arg Cys Ser Gly
    50                  55                  60

Ser Ile Cys Tyr Gly Thr Ile Ala Val Ile Val Phe Phe Leu Ile Gly

65                  70                  75                  80

Phe Met Ile Gly Tyr Leu Gly Tyr Cys Lys Gly Val Glu Pro Lys Thr
                85                  90                  95

Glu Cys Glu Arg Leu Ala Gly Thr Glu Ser Pro Val Arg Glu Glu Pro
            100                 105                 110

Gly Glu Asp Phe Pro Ala Ala Arg Arg Leu Tyr Trp Asp Asp Leu Lys
        115                 120                 125

Arg Lys Leu Ser Glu Lys Leu Asp Ser Thr Asp Phe Thr Gly Thr Ile
    130                 135                 140

Lys Leu Leu Asn Glu Asn Ser Tyr Val Pro Arg Glu Ala Gly Ser Gln
145                 150                 155                 160

Lys Asp Glu Asn Leu Ala Leu Tyr Val Glu Asn Gln Phe Arg Glu Phe
                165                 170                 175

Lys Leu Ser Lys Val Trp Arg Asp Gln His Phe Val Lys Ile Gln Val
            180                 185                 190

Lys Asp Ser Ala Gln Asn Ser Val Ile Ile Val Asp Lys Asn Gly Arg
        195                 200                 205

Leu Val Tyr Leu Val Glu Asn Pro Gly Gly Tyr Val Ala Tyr Ser Lys
    210                 215                 220

Ala Ala Thr Val Thr Gly Lys Leu Val His Ala Asn Phe Gly Thr Lys
225                 230                 235                 240

Lys Asp Phe Glu Asp Leu Tyr Thr Pro Val Asn Gly Ser Ile Val Ile
                245                 250                 255

Val Arg Ala Gly Lys Ile Thr Phe Ala Glu Lys Val Ala Asn Ala Glu
            260                 265                 270

Ser Leu Asn Ala Ile Gly Val Leu Ile Tyr Met Asp Gln Thr Lys Phe
        275                 280                 285

Pro Ile Val Asn Ala Glu Leu Ser Phe Phe Gly His Ala His Leu Gly
    290                 295                 300

Thr Gly Asp Pro Tyr Thr Pro Gly Phe Pro Ser Phe Asn His Thr Gln
305                 310                 315                 320

Phe Pro Pro Ser Arg Ser Ser Gly Leu Pro Asn Ile Pro Val Gln Thr
                325                 330                 335

Ile Ser Arg Ala Ala Ala Glu Lys Leu Phe Gly Asn Met Glu Gly Asp
            340                 345                 350

Cys Pro Ser Asp Trp Lys Thr Asp Ser Thr Cys Arg Met Val Thr Ser
        355                 360                 365

Glu Ser Lys Asn Val Lys Leu Thr Val Ser Asn Val Leu Lys Glu Ile
    370                 375                 380

Lys Ile Leu Asn Ile Phe Gly Val Ile Lys Gly Phe Val Glu Pro Asp
385                 390                 395                 400

His Tyr Val Val Val Gly Ala Gln Arg Asp Ala Trp Gly Pro Gly Ala
                405                 410                 415

Ala Lys Ser Gly Val Gly Thr Ala Leu Leu Leu Lys Leu Ala Gln Met
            420                 425                 430

Phe Ser Asp Met Val Leu Lys Asp Gly Phe Gln Pro Ser Arg Ser Ile
        435                 440                 445

Ile Phe Ala Ser Trp Ser Ala Gly Asp Phe Gly Ser Val Gly Ala Thr
    450                 455                 460

Glu Trp Leu Glu Gly Tyr Leu Ser Ser Leu His Leu Lys Ala Phe Thr
465                 470                 475                 480

Tyr Ile Asn Leu Asp Lys Ala Val Leu Gly Thr Ser Asn Phe Lys Val
                485                 490                 495

Ser Ala Ser Pro Leu Leu Tyr Thr Leu Ile Glu Lys Thr Met Gln Asn
            500                 505                 510

Val Lys His Pro Val Thr Gly Gln Phe Leu Tyr Gln Asp Ser Asn Trp
        515                 520                 525

Ala Ser Lys Val Glu Lys Leu Thr Leu Asp Asn Ala Ala Phe Pro Phe
    530                 535                 540

Leu Ala Tyr Ser Gly Ile Pro Ala Val Ser Phe Cys Phe Cys Glu Asp
545                 550                 555                 560

Thr Asp Tyr Pro Tyr Leu Gly Thr Thr Met Asp Thr Tyr Lys Glu Leu
                565                 570                 575

Ile Glu Arg Ile Pro Glu Leu Asn Lys Val Ala Arg Ala Ala Ala Glu
            580                 585                 590

Val Ala Gly Gln Phe Val Ile Lys Leu Thr His Asp Val Glu Leu Asn
        595                 600                 605

Leu Asp Tyr Glu Arg Tyr Asn Ser Gln Leu Leu Ser Phe Val Arg Asp
    610                 615                 620

Leu Asn Gln Tyr Arg Ala Asp Ile Lys Glu Met Gly Leu Ser Leu Gln
625                 630                 635                 640

Trp Leu Tyr Ser Ala Arg Gly Asp Phe Phe Arg Ala Thr Ser Arg Leu
                645                 650                 655

Thr Thr Asp Phe Gly Asn Ala Glu Lys Thr Asp Arg Phe Val Met Lys
            660                 665                 670

Lys Leu Asn Asp Arg Val Met Arg Val Glu Tyr His Phe Leu Ser Pro
        675                 680                 685

Tyr Val Ser Pro Lys Glu Ser Pro Phe Arg His Val Phe Trp Gly Ser
    690                 695                 700

Gly Ser His Thr Leu Pro Ala Leu Leu Glu Asn Leu Lys Leu Arg Lys
705                 710                 715                 720

Gln Asn Asn Gly Ala Phe Asn Glu Thr Leu Phe Arg Asn Gln Leu Ala
                725                 730                 735

Leu Ala Thr Trp Thr Ile Gln Gly Ala Ala Asn Ala Leu Ser Gly Asp
            740                 745                 750

Val Trp Asp Ile Asp Asn Glu Phe
        755                 760

<210> 6
<211> 492
<212> PRT
<213> Homo sapiens

<400> 6

<210>  7
<211>  559
<212>  PRT
<213>  Homo sapiens

<400>  7

Met Ser Ala Cys Arg Ser Phe Ala Val Ala Ile Cys Ile Leu Glu Ile
1               5                   10                  15

Ser Ile Leu Thr Ala Gln Tyr Thr Thr Ser Tyr Asp Pro Glu Leu Thr
            20                  25                  30

Glu Ser Ser Gly Ser Ala Ser His Ile Asp Cys Arg Met Ser Pro Trp
        35                  40                  45

Ser Glu Trp Ser Gln Cys Asp Pro Cys Leu Arg Gln Met Phe Arg Ser
    50                  55                  60

Arg Ser Ile Glu Val Phe Gly Gln Phe Asn Gly Lys Arg Cys Thr Asp
65                  70                  75                  80

Ala Val Gly Asp Arg Arg Gln Cys Val Pro Thr Glu Pro Cys Glu Asp
                85                  90                  95

Ala Glu Asp Asp Cys Gly Asn Asp Phe Gln Cys Ser Thr Gly Arg Cys
            100                 105                 110

Ile Lys Met Arg Leu Arg Cys Asn Gly Asp Asn Asp Cys Gly Asp Phe
        115                 120                 125

Ser Asp Glu Asp Asp Cys Glu Ser Glu Pro Arg Pro Pro Cys Arg Asp
    130                 135                 140

Arg Val Val Glu Glu Ser Glu Leu Ala Arg Thr Ala Gly Tyr Gly Ile
145                 150                 155                 160

Asn Ile Leu Gly Met Asp Pro Leu Ser Thr Pro Phe Asp Asn Glu Phe
                165                 170                 175

Tyr Asn Gly Leu Cys Asn Arg Asp Arg Asp Gly Asn Thr Leu Thr Tyr
            180                 185                 190

Tyr Arg Arg Pro Trp Asn Val Ala Ser Leu Ile Tyr Glu Thr Lys Gly
        195                 200                 205

Glu Lys Asn Phe Arg Thr Glu His Tyr Glu Glu Gln Ile Glu Ala Phe
    210                 215                 220

Lys Ser Ile Ile Gln Glu Lys Thr Ser Asn Phe Asn Ala Ala Ile Ser
225                 230                 235                 240

Leu Lys Phe Thr Pro Thr Glu Thr Asn Lys Ala Glu Gln Cys Cys Glu
                245                 250                 255

Glu Thr Ala Ser Ser Ile Ser Leu His Gly Lys Gly Ser Phe Arg Phe

            260                 265                 270

Ser Tyr Ser Lys Asn Glu Thr Tyr Gln Leu Phe Leu Ser Tyr Ser Ser
        275                 280                 285

Lys Lys Glu Lys Met Phe Leu His Val Lys Gly Glu Ile His Leu Gly
    290                 295                 300

Arg Phe Val Met Arg Asn Arg Asp Val Val Leu Thr Thr Thr Phe Val
305                 310                 315                 320

Asp Asp Ile Lys Ala Leu Pro Thr Thr Tyr Glu Lys Gly Glu Tyr Phe
                325                 330                 335

Ala Phe Leu Glu Thr Tyr Gly Thr His Tyr Ser Ser Ser Gly Ser Leu
            340                 345                 350

Gly Gly Leu Tyr Glu Leu Ile Tyr Val Leu Asp Lys Ala Ser Met Lys
        355                 360                 365

Arg Lys Gly Val Glu Leu Lys Asp Ile Lys Arg Cys Leu Gly Tyr His
    370                 375                 380

Leu Asp Val Ser Leu Ala Phe Ser Glu Ile Ser Val Gly Ala Glu Phe
385                 390                 395                 400

Asn Lys Asp Asp Cys Val Lys Arg Gly Glu Gly Arg Ala Val Asn Ile
                405                 410                 415

Thr Ser Glu Asn Leu Ile Asp Asp Val Val Ser Leu Ile Arg Gly Gly
            420                 425                 430

Thr Arg Lys Tyr Ala Phe Glu Leu Lys Glu Lys Leu Leu Arg Gly Thr
        435                 440                 445

Val Ile Asp Val Thr Asp Phe Val Asn Trp Ala Ser Ser Ile Asn Asp
    450                 455                 460

Ala Pro Val Leu Ile Ser Gln Lys Leu Ser Pro Ile Tyr Asn Leu Val
465                 470                 475                 480

Pro Val Lys Met Lys Asn Ala His Leu Lys Lys Gln Asn Leu Glu Arg
                485                 490                 495

Ala Ile Glu Asp Tyr Ile Asn Glu Phe Ser Val Arg Lys Cys His Thr
            500                 505                 510

Cys Gln Asn Gly Gly Thr Val Ile Leu Met Asp Gly Lys Cys Leu Cys
        515                 520                 525

Ala Cys Pro Phe Lys Phe Glu Gly Ile Ala Cys Glu Ile Ser Lys Gln
    530                 535                 540

Lys Ile Ser Glu Gly Leu Pro Ala Leu Glu Phe Pro Asn Glu Lys
545                 550                 555

<210>  8
<211>  350
<212>  PRT
<213>  Homo sapiens

<400>  8

Met Asp Ser Phe Asn Tyr Thr Thr Pro Asp Tyr Gly His Tyr Asp Asp
1               5                   10                  15

Lys Asp Thr Leu Asp Leu Asn Thr Pro Val Asp Lys Thr Ser Asn Thr
            20                  25                  30

Leu Arg Val Pro Asp Ile Leu Ala Leu Val Ile Phe Ala Val Val Phe
        35                  40                  45

Leu Val Gly Val Leu Gly Asn Ala Leu Val Val Trp Val Thr Ala Phe
    50                  55                  60

Glu Ala Lys Arg Thr Ile Asn Ala Ile Trp Phe Leu Asn Leu Ala Val
65                  70                  75                  80

Ala Asp Phe Leu Ser Cys Leu Ala Leu Pro Ile Leu Phe Thr Ser Ile
                85                  90                  95

Val Gln His His His Trp Pro Phe Gly Gly Ala Ala Cys Ser Ile Leu
            100                 105                 110

Pro Ser Leu Ile Leu Leu Asn Met Tyr Ala Ser Ile Leu Leu Leu Ala
        115                 120                 125

Thr Ile Ser Ala Asp Arg Phe Leu Leu Val Phe Lys Pro Ile Trp Cys
    130                 135                 140

Gln Asn Phe Arg Gly Ala Gly Leu Ala Trp Ile Ala Cys Ala Val Ala
145                 150                 155                 160

Trp Gly Leu Ala Leu Leu Leu Thr Ile Pro Ser Phe Leu Tyr Arg Val
                165                 170                 175

Val Arg Glu Glu Tyr Phe Pro Pro Lys Val Leu Cys Gly Val Asp Tyr
            180                 185                 190

Ser His Asp Lys Arg Arg Glu Arg Ala Val Ala Ile Val Arg Leu Val
        195                 200                 205

Leu Gly Phe Leu Trp Pro Leu Leu Thr Leu Thr Ile Cys Tyr Thr Phe
    210                 215                 220

Ile Leu Leu Arg Thr Trp Ser Arg Arg Ala Thr Arg Ser Thr Lys Thr
225                 230                 235                 240

Leu Lys Val Val Val Ala Val Val Ala Ser Phe Phe Ile Phe Trp Leu
                245                 250                 255

Pro Tyr Gln Val Thr Gly Ile Met Met Ser Phe Leu Glu Pro Ser Ser
            260                 265                 270

Pro Thr Phe Leu Leu Leu Lys Lys Leu Asp Ser Leu Cys Val Ser Phe
        275                 280                 285

Ala Tyr Ile Asn Cys Cys Ile Asn Pro Ile Ile Tyr Val Val Ala Gly
    290                 295                 300

Gln Gly Phe Gln Gly Arg Leu Arg Lys Ser Leu Pro Ser Leu Leu Arg
305                 310                 315                 320

Asn Val Leu Thr Glu Glu Ser Val Val Arg Glu Ser Lys Ser Phe Thr
                325                 330                 335

Arg Ser Thr Val Asp Thr Met Ala Gln Lys Thr Gln Ala Val
            340                 345                 350

<210> 9
<211> 654
<212> PRT
<213> Homo sapiens

<400> 9

Met Lys Leu Ser Leu Val Ala Ala Met Leu Leu Leu Leu Ser Ala Ala
1               5                   10                  15

Arg Ala Glu Glu Glu Asp Lys Lys Glu Asp Val Gly Thr Val Val Gly
            20                  25                  30

Ile Asp Leu Gly Thr Thr Tyr Ser Cys Val Gly Val Phe Lys Asn Gly
        35                  40                  45

Arg Val Glu Ile Ile Ala Asn Asp Gln Gly Asn Arg Ile Thr Pro Ser
    50                  55                  60

Tyr Val Ala Phe Thr Pro Glu Gly Glu Arg Leu Ile Gly Asp Ala Ala
65                  70                  75                  80

Lys Asn Gln Leu Thr Ser Asn Pro Glu Asn Thr Val Phe Asp Ala Lys
                85                  90                  95

Arg Leu Ile Gly Arg Thr Trp Asn Asp Pro Ser Val Gln Gln Asp Ile
            100                 105                 110

Lys Phe Leu Pro Phe Lys Val Val Glu Lys Lys Thr Lys Pro Tyr Ile
        115                 120                 125

Gln Val Asp Ile Gly Gly Gly Gln Thr Lys Thr Phe Ala Pro Glu Glu
    130                 135                 140

Ile Ser Ala Met Val Leu Thr Lys Met Lys Glu Thr Ala Glu Ala Tyr
145                 150                 155                 160

Leu Gly Lys Lys Val Thr His Ala Val Val Thr Val Pro Ala Tyr Phe
                165                 170                 175

Asn Asp Ala Gln Arg Gln Ala Thr Lys Asp Ala Gly Thr Ile Ala Gly
            180                 185                 190

Leu Asn Val Met Arg Ile Ile Asn Glu Pro Thr Ala Ala Ala Ile Ala
        195                 200                 205

Tyr Gly Leu Asp Lys Arg Glu Gly Glu Lys Asn Ile Leu Val Phe Asp
    210                 215                 220

Leu Gly Gly Gly Thr Phe Asp Val Ser Leu Leu Thr Ile Asp Asn Gly
225                 230                 235                 240

Val Phe Glu Val Val Ala Thr Asn Gly Asp Thr His Leu Gly Gly Glu
                245                 250                 255

Asp Phe Asp Gln Arg Val Met Glu His Phe Ile Lys Leu Tyr Lys Lys
            260                 265                 270

Lys Thr Gly Lys Asp Val Arg Lys Asp Asn Arg Ala Val Gln Lys Leu
        275                 280                 285

Arg Arg Glu Val Glu Lys Ala Lys Arg Ala Leu Ser Ser Gln His Gln
    290                 295                 300

Ala Arg Ile Glu Ile Glu Ser Phe Tyr Glu Gly Glu Asp Phe Ser Glu
305                 310                 315                 320

Thr Leu Thr Arg Ala Lys Phe Glu Glu Leu Asn Met Asp Leu Phe Arg
                325                 330                 335

Ser Thr Met Lys Pro Val Gln Lys Val Leu Glu Asp Ser Asp Leu Lys
            340                 345                 350

Lys Ser Asp Ile Asp Glu Ile Val Leu Val Gly Gly Ser Thr Arg Ile
        355                 360                 365

Pro Lys Ile Gln Gln Leu Val Lys Glu Phe Phe Asn Gly Lys Glu Pro
    370                 375                 380

Ser Arg Gly Ile Asn Pro Asp Glu Ala Val Ala Tyr Gly Ala Ala Val
385                 390                 395                 400

Gln Ala Gly Val Leu Ser Gly Asp Gln Asp Thr Gly Asp Leu Val Leu
                405                 410                 415

Leu Asp Val Cys Pro Leu Thr Leu Gly Ile Glu Thr Val Gly Gly Val
            420                 425                 430

Met Thr Lys Leu Ile Pro Arg Asn Thr Val Val Pro Thr Lys Lys Ser
        435                 440                 445

Gln Ile Phe Ser Thr Ala Ser Asp Asn Gln Pro Thr Val Thr Ile Lys
    450                 455                 460

Val Tyr Glu Gly Glu Arg Pro Leu Thr Lys Asp Asn His Leu Leu Gly
465                 470                 475                 480

Thr Phe Asp Leu Thr Gly Ile Pro Pro Ala Pro Arg Gly Val Pro Gln
                485                 490                 495

Ile Glu Val Thr Phe Glu Ile Asp Val Asn Gly Ile Leu Arg Val Thr
            500                 505                 510

Ala Glu Asp Lys Gly Thr Gly Asn Lys Asn Lys Ile Thr Ile Thr Asn
        515                 520                 525

Asp Gln Asn Arg Leu Thr Pro Glu Glu Ile Glu Arg Met Val Asn Asp
    530                 535                 540

Ala Glu Lys Phe Ala Glu Glu Asp Lys Lys Leu Lys Glu Arg Ile Asp
545                 550                 555                 560

Thr Arg Asn Glu Leu Glu Ser Tyr Ala Tyr Ser Leu Lys Asn Gln Ile
                565                 570                 575

Gly Asp Lys Glu Lys Leu Gly Gly Lys Leu Ser Ser Glu Asp Lys Glu
            580                 585                 590

Thr Met Glu Lys Ala Val Glu Glu Lys Ile Glu Trp Leu Glu Ser His
        595                 600                 605

Gln Asp Ala Asp Ile Glu Asp Phe Lys Ala Lys Lys Lys Glu Leu Glu
    610                 615                 620

Glu Ile Val Gln Pro Ile Ile Ser Lys Leu Tyr Gly Ser Ala Gly Pro
625                 630                 635                 640

Pro Pro Thr Gly Glu Glu Asp Thr Ala Glu Lys Asp Glu Leu
                645                 650

<210>  10
<211>  201
<212>  PRT
<213>  Homo sapiens

<400>  10

Met Ala Leu Ser Trp Val Leu Thr Val Leu Ser Leu Leu Pro Leu Leu
1               5                   10                  15

Glu Ala Gln Ile Pro Leu Cys Ala Asn Leu Val Pro Val Pro Ile Thr
            20                  25                  30

Asn Ala Thr Leu Asp Gln Ile Thr Gly Lys Trp Phe Tyr Ile Ala Ser
        35                  40                  45

Ala Phe Arg Asn Glu Glu Tyr Asn Lys Ser Val Gln Glu Ile Gln Ala
    50                  55                  60

Thr Phe Phe Tyr Phe Thr Pro Asn Lys Thr Glu Asp Thr Ile Phe Leu
65                  70                  75                  80

Arg Glu Tyr Gln Thr Arg Gln Asp Gln Cys Ile Tyr Asn Thr Thr Tyr
                85                  90                  95

Leu Asn Val Gln Arg Glu Asn Gly Thr Ile Ser Arg Tyr Val Gly Gly
            100                 105                 110

Gln Glu His Phe Ala His Leu Leu Ile Leu Arg Asp Thr Lys Thr Tyr
        115                 120                 125

Met Leu Ala Phe Asp Val Asn Asp Glu Lys Asn Trp Gly Leu Ser Val
    130                 135                 140

Tyr Ala Asp Lys Pro Glu Thr Thr Lys Glu Gln Leu Gly Glu Phe Tyr
145                 150                 155                 160

Glu Ala Leu Asp Cys Leu Arg Ile Pro Lys Ser Asp Val Val Tyr Thr
                165                 170                 175

Asp Trp Lys Lys Asp Lys Cys Glu Pro Leu Glu Lys Gln His Glu Lys
            180                 185                 190

Glu Arg Lys Gln Glu Glu Gly Glu Ser
        195                 200

<210> 11
<211> 707
<212> PRT
<213> Homo sapiens

<400> 11

<210> 12
<211> 498
<212> PRT
<213> Homo sapiens

<400> 12

Met Thr Val Phe Leu Ser Phe Ala Phe Leu Ala Ala Ile Leu Thr His
1               5                   10                  15

Ile Gly Cys Ser Asn Gln Arg Arg Ser Pro Glu Asn Ser Gly Arg Arg
            20                  25                  30

Tyr Asn Arg Ile Gln His Gly Gln Cys Ala Tyr Thr Phe Ile Leu Pro
        35                  40                  45

Glu His Asp Gly Asn Cys Arg Glu Ser Thr Thr Asp Gln Tyr Asn Thr
    50                  55                  60

Asn Ala Leu Gln Arg Asp Ala Pro His Val Glu Pro Asp Phe Ser Ser
65                  70                  75                  80

Gln Lys Leu Gln His Leu Glu His Val Met Glu Asn Tyr Thr Gln Trp
                85                  90                  95

Leu Gln Lys Leu Glu Asn Tyr Ile Val Glu Asn Met Lys Ser Glu Met
            100                 105                 110

Ala Gln Ile Gln Gln Asn Ala Val Gln Asn His Thr Ala Thr Met Leu
        115                 120                 125

Glu Ile Gly Thr Ser Leu Leu Ser Gln Thr Ala Glu Gln Thr Arg Lys
    130                 135                 140

Leu Thr Asp Val Glu Thr Gln Val Leu Asn Gln Thr Ser Arg Leu Glu
145                 150                 155                 160

Ile Gln Leu Leu Glu Asn Ser Leu Ser Thr Tyr Lys Leu Glu Lys Gln
                165                 170                 175

Leu Leu Gln Gln Thr Asn Glu Ile Leu Lys Ile His Glu Lys Asn Ser
            180                 185                 190

Leu Leu Glu His Lys Ile Leu Glu Met Glu Gly Lys His Lys Glu Glu
        195                 200                 205

Leu Asp Thr Leu Lys Glu Glu Lys Glu Asn Leu Gln Gly Leu Val Thr
    210                 215                 220

Arg Gln Thr Tyr Ile Ile Gln Glu Leu Glu Lys Gln Leu Asn Arg Ala
225                 230                 235                 240

Thr Thr Asn Asn Ser Val Leu Gln Lys Gln Gln Leu Glu Leu Met Asp
                245                 250                 255

Thr Val His Asn Leu Val Asn Leu Cys Thr Lys Glu Gly Val Leu Leu
            260                 265                 270

Lys Gly Gly Lys Arg Glu Glu Glu Lys Pro Phe Arg Asp Cys Ala Asp
        275                 280                 285

Val Tyr Gln Ala Gly Phe Asn Lys Ser Gly Ile Tyr Thr Ile Tyr Ile
    290                 295                 300

Asn Asn Met Pro Glu Pro Lys Lys Val Phe Cys Asn Met Asp Val Asn
305                 310                 315                 320

Gly Gly Gly Trp Thr Val Ile Gln His Arg Glu Asp Gly Ser Leu Asp
                325                 330                 335

Phe Gln Arg Gly Trp Lys Glu Tyr Lys Met Gly Phe Gly Asn Pro Ser
            340                 345                 350

Gly Glu Tyr Trp Leu Gly Asn Glu Phe Ile Phe Ala Ile Thr Ser Gln
        355                 360                 365

Arg Gln Tyr Met Leu Arg Ile Glu Leu Met Asp Trp Glu Gly Asn Arg
    370                 375                 380

Ala Tyr Ser Gln Tyr Asp Arg Phe His Ile Gly Asn Glu Lys Gln Asn
385                 390                 395                 400

Tyr Arg Leu Tyr Leu Lys Gly His Thr Gly Thr Ala Gly Lys Gln Ser
                405                 410                 415

Ser Leu Ile Leu His Gly Ala Asp Phe Ser Thr Lys Asp Ala Asp Asn
            420                 425                 430

Asp Asn Cys Met Cys Lys Cys Ala Leu Met Leu Thr Gly Gly Trp Trp
        435                 440                 445

Phe Asp Ala Cys Gly Pro Ser Asn Leu Asn Gly Met Phe Tyr Thr Ala
    450                 455                 460

Gly Gln Asn His Gly Lys Leu Asn Gly Ile Lys Trp His Tyr Phe Lys
465                 470                 475                 480

Gly Pro Ser Tyr Ser Leu Arg Ser Thr Thr Met Met Ile Arg Pro Leu
                485                 490                 495

Asp Phe

<210>  13
<211>  349
<212>  PRT
<213>  Homo sapiens

<400>  13

Met Gly Pro Ile Ser Ala Pro Ser Cys Arg Trp Arg Ile Pro Trp Gln
1               5                   10                  15

Gly Leu Leu Leu Thr Ala Ser Leu Phe Thr Phe Trp Asn Pro Pro Thr
            20                  25                  30

Thr Ala Gln Leu Thr Ile Glu Ala Val Pro Ser Asn Ala Ala Glu Gly
        35                  40                  45

Lys Glu Val Leu Leu Leu Val His Asn Leu Pro Gln Asp Pro Arg Gly
    50                  55                  60

Tyr Asn Trp Tyr Lys Gly Glu Thr Val Asp Ala Asn Arg Arg Ile Ile
65                  70                  75                  80

Gly Tyr Val Ile Ser Asn Gln Gln Ile Thr Pro Gly Pro Ala Tyr Ser
                85                  90                  95

Asn Arg Glu Thr Ile Tyr Pro Asn Ala Ser Leu Leu Met Arg Asn Val
            100                 105                 110

Thr Arg Asn Asp Thr Gly Ser Tyr Thr Leu Gln Val Ile Lys Leu Asn
        115                 120                 125

Leu Met Ser Glu Glu Val Thr Gly Gln Phe Ser Val His Pro Glu Thr
    130                 135                 140

Pro Lys Pro Ser Ile Ser Ser Asn Asn Ser Asn Pro Val Glu Asp Lys
145                 150                 155                 160

Asp Ala Val Ala Phe Thr Cys Glu Pro Glu Thr Gln Asn Thr Thr Tyr
                165                 170                 175

Leu Trp Trp Val Asn Gly Gln Ser Leu Pro Val Ser Pro Arg Leu Gln
            180                 185                 190

Leu Ser Asn Gly Asn Arg Thr Leu Thr Leu Leu Ser Val Thr Arg Asn
        195                 200                 205

Asp Val Gly Pro Tyr Glu Cys Glu Ile Gln Asn Pro Ala Ser Ala Asn
    210                 215                 220

Phe Ser Asp Pro Val Thr Leu Asn Val Leu Tyr Gly Pro Asp Ala Pro
225                 230                 235                 240

Thr Ile Ser Pro Ser Asp Thr Tyr Tyr His Ala Gly Val Asn Leu Asn
                245                 250                 255

Leu Ser Cys His Ala Ala Ser Asn Pro Pro Ser Gln Tyr Ser Trp Ser
            260                 265                 270

Val Asn Gly Thr Phe Gln Gln Tyr Thr Gln Lys Leu Phe Ile Pro Asn
        275                 280                 285

Ile Thr Thr Lys Asn Ser Gly Ser Tyr Ala Cys His Thr Thr Asn Ser
    290                 295                 300

Ala Thr Gly Arg Asn Arg Thr Thr Val Arg Met Ile Thr Val Ser Asp
305                 310                 315                 320

Ala Leu Val Gln Gly Ser Ser Pro Gly Leu Ser Ala Arg Ala Thr Val
                325                 330                 335

Ser Ile Met Ile Gly Val Leu Ala Arg Val Ala Leu Ile
            340                 345

<210>  14
<211>  5762

<212>  PRT
<213>  Homo sapiens

<400>  14

Met Gly Ala Pro Ser Ala Cys Arg Thr Leu Val Leu Ala Leu Ala Ala
1               5                   10                  15

Met Leu Val Val Pro Gln Ala Glu Thr Gln Gly Pro Val Glu Pro Ser
            20                  25                  30

Trp Glu Asn Ala Gly His Thr Met Asp Gly Gly Ala Pro Thr Ser Ser
        35                  40                  45

Pro Thr Arg Arg Val Ser Phe Val Pro Pro Val Thr Val Phe Pro Ser
    50                  55                  60

Leu Ser Pro Leu Asn Pro Ala His Asn Gly Arg Val Cys Ser Thr Trp
65                  70                  75                  80

Gly Asp Phe His Tyr Lys Thr Phe Asp Gly Asp Val Phe Arg Phe Pro
                85                  90                  95

Gly Leu Cys Asn Tyr Val Phe Ser Glu His Cys Arg Ala Ala Tyr Glu
            100                 105                 110

Asp Phe Asn Val Gln Leu Arg Arg Gly Leu Val Gly Ser Arg Pro Val
        115                 120                 125

Val Thr Arg Val Val Ile Lys Ala Gln Gly Leu Val Leu Glu Ala Ser
    130                 135                 140

Asn Gly Ser Val Leu Ile Asn Gly Gln Arg Glu Glu Leu Pro Tyr Ser
145                 150                 155                 160

Arg Thr Gly Leu Leu Val Glu Gln Ser Gly Asp Tyr Ile Lys Val Ser
                165                 170                 175

Ile Arg Leu Val Leu Thr Phe Leu Trp Asn Gly Glu Asp Ser Ala Leu
            180                 185                 190

Leu Glu Leu Asp Pro Lys Tyr Ala Asn Gln Thr Cys Gly Leu Cys Gly
        195                 200                 205

Asp Phe Asn Gly Leu Pro Ala Phe Asn Glu Phe Tyr Ala His Asn Ala
    210                 215                 220

Arg Leu Thr Pro Leu Gln Phe Gly Asn Leu Gln Lys Leu Asp Gly Pro
225                 230                 235                 240

Thr Glu Gln Cys Pro Asp Pro Leu Pro Leu Pro Ala Gly Asn Cys Thr
                    245                 250                 255

Asp Glu Glu Gly Ile Cys His Arg Thr Leu Leu Gly Pro Ala Phe Ala
                260                 265                 270

Glu Cys His Ala Leu Val Asp Ser Thr Ala Tyr Leu Ala Ala Cys Ala
            275                 280                 285

Gln Asp Leu Cys Arg Cys Pro Thr Cys Pro Cys Ala Thr Phe Val Glu
        290                 295                 300

Tyr Ser Arg Gln Cys Ala His Ala Gly Gly Gln Pro Arg Asn Trp Arg
305                 310                 315                 320

Cys Pro Glu Leu Cys Pro Arg Thr Cys Pro Leu Asn Met Gln His Gln
                    325                 330                 335

Glu Cys Gly Ser Pro Cys Thr Asp Thr Cys Ser Asn Pro Gln Arg Ala
                340                 345                 350

Gln Leu Cys Glu Asp His Cys Val Asp Gly Cys Phe Cys Pro Pro Gly
            355                 360                 365

Thr Val Leu Asp Asp Ile Thr His Ser Gly Cys Leu Pro Leu Gly Gln
        370                 375                 380

Cys Pro Cys Thr His Gly Gly Arg Thr Tyr Ser Pro Gly Thr Ser Phe
385                 390                 395                 400

Asn Thr Thr Cys Ser Ser Cys Thr Cys Ser Gly Gly Leu Trp Gln Cys
                    405                 410                 415

Gln Asp Leu Pro Cys Pro Gly Thr Cys Ser Val Gln Gly Gly Ala His
                420                 425                 430

Ile Ser Thr Tyr Asp Glu Lys Leu Tyr Asp Leu His Gly Asp Cys Ser
            435                 440                 445

Tyr Val Leu Ser Lys Lys Cys Ala Asp Ser Ser Phe Thr Val Leu Ala
        450                 455                 460

Glu Leu Arg Lys Cys Gly Leu Thr Asp Asn Glu Asn Cys Leu Lys Ala
465                 470                 475                 480

Val Thr Leu Ser Leu Asp Gly Gly Asp Thr Ala Ile Arg Val Gln Ala

                485                 490                 495

Asp Gly Gly Val Phe Leu Asn Ser Ile Tyr Thr Gln Leu Pro Leu Ser
            500                 505                 510

Ala Ala Asn Ile Thr Leu Phe Thr Pro Ser Ser Phe Phe Ile Val Val
        515                 520                 525

Gln Thr Gly Leu Gly Leu Gln Leu Leu Val Gln Leu Val Pro Leu Met
    530                 535                 540

Gln Val Phe Val Arg Leu Asp Pro Ala His Gln Gly Gln Met Cys Gly
545                 550                 555                 560

Leu Cys Gly Asn Phe Asn Gln Asn Gln Ala Asp Asp Phe Thr Ala Leu
                565                 570                 575

Ser Gly Val Val Glu Ala Thr Gly Ala Ala Phe Ala Asn Thr Trp Lys
            580                 585                 590

Ala Gln Ala Ala Cys Ala Asn Ala Arg Asn Ser Phe Glu Asp Pro Cys
        595                 600                 605

Ser Leu Ser Val Glu Asn Glu Asn Tyr Ala Arg His Trp Cys Ser Arg
    610                 615                 620

Leu Thr Asp Pro Asn Ser Ala Phe Ser Arg Cys His Ser Ile Ile Asn
625                 630                 635                 640

Pro Lys Pro Phe His Ser Asn Cys Met Phe Asp Thr Cys Asn Cys Glu
                645                 650                 655

Arg Ser Glu Asp Cys Leu Cys Ala Ala Leu Ser Ser Tyr Val His Ala
            660                 665                 670

Cys Ala Ala Lys Gly Val Gln Leu Ser Asp Trp Arg Asp Gly Val Cys
        675                 680                 685

Thr Lys Tyr Met Gln Asn Cys Pro Lys Ser Gln Arg Tyr Ala Tyr Val
    690                 695                 700

Val Asp Ala Cys Gln Pro Thr Cys Arg Gly Leu Ser Glu Ala Asp Val
705                 710                 715                 720

Thr Cys Ser Val Ser Phe Val Pro Val Asp Gly Cys Thr Cys Pro Ala
                725                 730                 735

Gly Thr Phe Leu Asn Asp Ala Gly Ala Cys Val Pro Ala Gln Glu Cys
        740                 745                 750

Pro Cys Tyr Ala His Gly Thr Val Leu Ala Pro Gly Glu Val Val His
    755                 760                 765

Asp Glu Gly Ala Val Cys Ser Cys Thr Gly Gly Lys Leu Ser Cys Leu
    770                 775                 780

Gly Ala Ser Leu Gln Lys Ser Thr Gly Cys Ala Ala Pro Met Val Tyr
785                 790                 795                 800

Leu Asp Cys Ser Asn Ser Ser Ala Gly Thr Pro Gly Ala Glu Cys Leu
                805                 810                 815

Arg Ser Cys His Thr Leu Asp Val Gly Cys Phe Ser Thr His Cys Val
            820                 825                 830

Ser Gly Cys Val Cys Pro Pro Gly Leu Val Ser Asp Gly Ser Gly Gly
        835                 840                 845

Cys Ile Ala Glu Glu Asp Cys Pro Cys Val His Asn Glu Ala Thr Tyr
    850                 855                 860

Lys Pro Gly Glu Thr Ile Arg Val Asp Cys Asn Thr Cys Thr Cys Arg
865                 870                 875                 880

Asn Arg Arg Trp Glu Cys Ser His Arg Leu Cys Leu Gly Thr Cys Val
                885                 890                 895

Ala Tyr Gly Asp Gly His Phe Ile Thr Phe Asp Gly Asp Arg Tyr Ser
            900                 905                 910

Phe Glu Gly Ser Cys Glu Tyr Ile Leu Ala Gln Asp Tyr Cys Gly Asp
        915                 920                 925

Asn Thr Thr His Gly Thr Phe Arg Ile Val Thr Glu Asn Ile Pro Cys
    930                 935                 940

Gly Thr Thr Gly Thr Thr Cys Ser Lys Ala Ile Lys Leu Phe Val Glu
945                 950                 955                 960

Ser Tyr Glu Leu Ile Leu Gln Glu Gly Thr Phe Lys Ala Val Ala Arg
                965                 970                 975

Gly Pro Gly Gly Asp Pro Pro Tyr Lys Ile Arg Tyr Met Gly Ile Phe
            980                 985                 990

Leu Val Ile Glu Thr His Gly Met Ala Val Ser Trp Asp Arg Lys Thr
        995                 1000                1005

Ser Val Phe Ile Arg Leu His Gln Asp Tyr Lys Gly Arg Val Cys
    1010                1015                1020

Gly Leu Cys Gly Asn Phe Asp Asp Asn Ala Ile Asn Asp Phe Ala
    1025                1030                1035

Thr Arg Ser Arg Ser Val Val Gly Asp Ala Leu Glu Phe Gly Asn
    1040                1045                1050

Ser Trp Lys Leu Ser Pro Ser Cys Pro Asp Ala Leu Ala Pro Lys
    1055                1060                1065

Asp Pro Cys Thr Ala Asn Pro Phe Arg Lys Ser Trp Ala Gln Lys
    1070                1075                1080

Gln Cys Ser Ile Leu His Gly Pro Thr Phe Ala Ala Cys Arg Ser
    1085                1090                1095

Gln Val Asp Ser Thr Lys Tyr Tyr Glu Ala Cys Val Asn Asp Ala
    1100                1105                1110

Cys Ala Cys Asp Ser Gly Gly Asp Cys Glu Cys Phe Cys Thr Ala
    1115                1120                1125

Val Ala Ala Tyr Ala Gln Ala Cys His Asp Ala Gly Leu Cys Val
    1130                1135                1140

Ser Trp Arg Thr Pro Asp Thr Cys Pro Leu Phe Cys Asp Phe Tyr
    1145                1150                1155

Asn Pro His Gly Gly Cys Glu Trp His Tyr Gln Pro Cys Gly Ala
    1160                1165                1170

Pro Cys Leu Lys Thr Cys Arg Asn Pro Ser Gly His Cys Leu Val
    1175                1180                1185

Asp Leu Pro Gly Leu Glu Gly Cys Tyr Pro Lys Cys Pro Pro Ser
    1190                1195                1200

Gln Pro Phe Phe Asn Glu Asp Gln Met Lys Cys Val Ala Gln Cys
    1205                1210                1215

Gly Cys Tyr Asp Lys Asp Gly Asn Tyr Tyr Asp Val Gly Ala Arg
    1220                1225                1230

Val Pro Thr Ala Glu Asn Cys Gln Ser Cys Asn Cys Thr Pro Ser
    1235                1240                1245

Gly Ile Gln Cys Ala His Ser Leu Glu Ala Cys Thr Cys Thr Tyr
    1250                1255                1260

Glu Asp Arg Thr Tyr Ser Tyr Gln Asp Val Ile Tyr Asn Thr Thr
    1265                1270                1275

Asp Gly Leu Gly Ala Cys Leu Ile Ala Ile Cys Gly Ser Asn Gly
    1280                1285                1290

Thr Ile Ile Arg Lys Ala Val Ala Cys Pro Gly Thr Pro Ala Thr
    1295                1300                1305

Thr Pro Phe Thr Phe Thr Thr Ala Trp Val Pro His Ser Thr Thr
    1310                1315                1320

Ser Pro Ala Leu Pro Val Ser Thr Val Cys Val Arg Glu Val Cys
    1325                1330                1335

Arg Trp Ser Ser Trp Tyr Asn Gly His Arg Pro Glu Pro Gly Leu
    1340                1345                1350

Gly Gly Gly Asp Phe Glu Thr Phe Glu Asn Leu Arg Gln Arg Gly
    1355                1360                1365

Tyr Gln Val Cys Pro Val Leu Ala Asp Ile Glu Cys Arg Ala Ala
    1370                1375                1380

Gln Leu Pro Asp Met Pro Leu Glu Glu Leu Gly Gln Gln Val Asp
    1385                1390                1395

Cys Asp Arg Met Arg Gly Leu Met Cys Ala Asn Ser Gln Gln Ser
    1400                1405                1410

Pro Pro Leu Cys His Asp Tyr Glu Leu Arg Val Leu Cys Cys Glu
    1415                1420                1425

Tyr Val Pro Cys Gly Pro Ser Pro Ala Pro Gly Thr Ser Pro Gln
    1430                1435                1440

Pro Ser Leu Ser Ala Ser Thr Glu Pro Ala Val Pro Thr Pro Thr
    1445                1450                1455

Gln Thr Thr Ala Thr Glu Lys Thr Thr Leu Trp Val Thr Pro Ser

1460                1465                1470

Ile Arg Ser Thr Ala Ala Leu Thr Ser Gln Thr Gly Ser Ser Ser
    1475                1480                1485

Gly Pro Val Thr Val Thr Pro Ser Ala Pro Gly Thr Thr Thr Cys
    1490                1495                1500

Gln Pro Arg Cys Gln Trp Thr Glu Trp Phe Asp Glu Asp Tyr Pro
    1505                1510                1515

Lys Ser Glu Gln Leu Gly Gly Asp Val Glu Ser Tyr Asp Lys Ile
    1520                1525                1530

Arg Ala Ala Gly Gly His Leu Cys Gln Gln Pro Lys Asp Ile Glu
    1535                1540                1545

Cys Gln Ala Glu Ser Phe Pro Asn Trp Thr Leu Ala Gln Val Gly
    1550                1555                1560

Gln Lys Val His Cys Asp Val His Phe Gly Leu Val Cys Arg Asn
    1565                1570                1575

Trp Glu Gln Glu Gly Val Phe Lys Met Cys Tyr Asn Tyr Arg Ile
    1580                1585                1590

Arg Val Leu Cys Cys Ser Asp Asp His Cys Arg Gly Arg Ala Thr
    1595                1600                1605

Thr Pro Pro Pro Thr Thr Glu Leu Glu Thr Ala Thr Thr Thr Thr
    1610                1615                1620

Thr Gln Ala Leu Phe Ser Thr Pro Gln Pro Thr Ser Ser Pro Gly
    1625                1630                1635

Leu Thr Arg Ala Pro Pro Ala Ser Thr Thr Ala Val Pro Thr Leu
    1640                1645                1650

Ser Glu Gly Leu Thr Ser Pro Arg Tyr Thr Ser Thr Leu Gly Thr
    1655                1660                1665

Ala Thr Thr Gly Gly Pro Thr Thr Pro Ala Gly Ser Thr Glu Pro
    1670                1675                1680

Thr Val Pro Gly Val Ala Thr Ser Thr Leu Pro Thr Arg Ser Ala
    1685                1690                1695

Leu Pro Gly Thr Thr Gly Ser Leu Gly Thr Trp Arg Pro Ser Gln
    1700                1705                1710

Pro Pro Thr Leu Ala Pro Thr Thr Met Ala Thr Ser Arg Ala Arg
    1715                1720                1725

Pro Thr Gly Thr Ala Ser Thr Ala Ser Lys Glu Pro Leu Thr Thr
    1730                1735                1740

Ser Leu Ala Pro Thr Leu Thr Ser Glu Leu Ser Thr Ser Gln Ala
    1745                1750                1755

Glu Thr Ser Thr Pro Arg Thr Glu Thr Thr Met Ser Pro Leu Thr
    1760                1765                1770

Asn Thr Thr Thr Ser Gln Gly Thr Thr Arg Cys Gln Pro Lys Cys
    1775                1780                1785

Glu Trp Thr Glu Trp Phe Asp Val Asp Phe Pro Thr Ser Gly Val
    1790                1795                1800

Ala Gly Gly Asp Met Glu Thr Phe Glu Asn Ile Arg Ala Ala Gly
    1805                1810                1815

Gly Lys Met Cys Trp Ala Pro Lys Ser Ile Glu Cys Arg Ala Glu
    1820                1825                1830

Asn Tyr Pro Glu Val Ser Ile Asp Gln Val Gly Gln Val Leu Thr
    1835                1840                1845

Cys Ser Leu Glu Thr Gly Leu Thr Cys Lys Asn Glu Asp Gln Thr
    1850                1855                1860

Gly Arg Phe Asn Met Cys Phe Asn Tyr Asn Val Arg Val Leu Cys
    1865                1870                1875

Cys Asp Asp Tyr Ser His Cys Pro Ser Thr Pro Ala Thr Ser Ser
    1880                1885                1890

Thr Ala Thr Pro Ser Ser Thr Pro Gly Thr Thr Trp Ile Leu Thr
    1895                1900                1905

Lys Pro Thr Thr Thr Ala Thr Thr Thr Ala Ser Thr Gly Ser Thr
    1910                1915                1920

Ala Thr Pro Thr Ser Thr Leu Arg Thr Ala Pro Pro Pro Lys Val
    1925                1930                1935

Leu Thr Thr Thr Ala Thr Thr Pro Thr Val Thr Ser Ser Lys Ala
    1940                1945                1950

Thr Pro Ser Ser Ser Pro Gly Thr Ala Thr Ala Leu Pro Ala Leu
    1955                1960                1965

Arg Ser Thr Ala Thr Thr Pro Thr Ala Thr Ser Val Thr Pro Ile
    1970                1975                1980

Pro Ser Ser Ser Leu Gly Thr Thr Trp Thr Arg Leu Ser Gln Thr
    1985                1990                1995

Thr Thr Pro Thr Ala Thr Met Ser Thr Ala Thr Pro Ser Ser Thr
    2000                2005                2010

Pro Glu Thr Ala His Thr Ser Thr Val Leu Thr Ala Thr Ala Thr
    2015                2020                2025

Thr Thr Gly Ala Thr Gly Ser Val Ala Thr Pro Ser Ser Thr Pro
    2030                2035                2040

Gly Thr Ala His Thr Thr Lys Val Pro Thr Thr Thr Thr Thr Gly
    2045                2050                2055

Phe Thr Ala Thr Pro Ser Ser Ser Pro Gly Thr Ala Leu Thr Pro
    2060                2065                2070

Pro Val Trp Ile Ser Thr Thr Thr Thr Pro Thr Thr Arg Gly Ser
    2075                2080                2085

Thr Val Thr Pro Ser Ser Ile Pro Gly Thr Thr His Thr Ala Thr
    2090                2095                2100

Val Leu Thr Thr Thr Thr Thr Thr Val Ala Thr Gly Ser Met Ala
    2105                2110                2115

Thr Pro Ser Ser Ser Thr Gln Thr Ser Gly Thr Pro Pro Ser Leu
    2120                2125                2130

Thr Thr Thr Ala Thr Thr Ile Thr Ala Thr Gly Ser Thr Thr Asn
    2135                2140                2145

Pro Ser Ser Thr Pro Gly Thr Thr Pro Ile Pro Pro Val Leu Thr
    2150                2155                2160

Thr Thr Ala Thr Thr Pro Ala Ala Thr Ser Asn Thr Val Thr Pro
    2165                2170                2175

Ser Ser Ala Leu Gly Thr Thr His Thr Pro Pro Val Pro Asn Thr
    2180                2185                2190

Met Ala Thr Thr His Gly Arg Ser Leu Pro Pro Ser Ser Pro His
    2195                2200                2205

Thr Val Arg Thr Ala Trp Thr Ser Ala Thr Ser Gly Ile Leu Gly
    2210                2215                2220

Thr Thr His Ile Thr Glu Pro Ser Thr Val Thr Ser His Thr Leu
    2225                2230                2235

Ala Ala Thr Thr Gly Thr Thr Gln His Ser Thr Pro Ala Leu Ser
    2240                2245                2250

Ser Pro His Pro Ser Ser Arg Thr Thr Glu Ser Pro Pro Ser Pro
    2255                2260                2265

Gly Thr Thr Thr Pro Gly His Thr Thr Ala Thr Ser Arg Thr Thr
    2270                2275                2280

Ala Thr Ala Thr Pro Ser Lys Thr Arg Thr Ser Thr Leu Leu Pro
    2285                2290                2295

Ser Ser Pro Thr Ser Ala Pro Ile Thr Thr Val Val Thr Met Gly
    2300                2305                2310

Cys Glu Pro Gln Cys Ala Trp Ser Glu Trp Leu Asp Tyr Ser Tyr
    2315                2320                2325

Pro Met Pro Gly Pro Ser Gly Gly Asp Phe Asp Thr Tyr Ser Asn
    2330                2335                2340

Ile Arg Ala Ala Gly Gly Ala Val Cys Glu Gln Pro Leu Gly Leu
    2345                2350                2355

Glu Cys Arg Ala Gln Ala Gln Pro Gly Val Pro Leu Arg Glu Leu
    2360                2365                2370

Gly Gln Val Val Glu Cys Ser Leu Asp Phe Gly Leu Val Cys Arg
    2375                2380                2385

Asn Arg Glu Gln Val Gly Lys Phe Lys Met Cys Phe Asn Tyr Glu
    2390                2395                2400

Ile Arg Val Phe Cys Cys Asn Tyr Gly His Cys Pro Ser Thr Pro

2405                2410                2415

Ala Thr Ser Ser Thr Ala Met Pro Ser Ser Thr Pro Gly Thr Thr
    2420                2425                2430

Trp Ile Leu Thr Glu Leu Thr Thr Thr Ala Thr Thr Thr Glu Ser
    2435                2440                2445

Thr Gly Ser Thr Ala Thr Pro Ser Ser Thr Pro Gly Thr Thr Trp
    2450                2455                2460

Ile Leu Thr Glu Pro Ser Thr Thr Ala Thr Val Thr Val Pro Thr
    2465                2470                2475

Gly Ser Thr Ala Thr Ala Ser Ser Thr Gln Ala Thr Ala Gly Thr
    2480                2485                2490

Pro His Val Ser Thr Thr Ala Thr Thr Pro Thr Val Thr Ser Ser
    2495                2500                2505

Lys Ala Thr Pro Phe Ser Ser Pro Gly Thr Ala Thr Ala Leu Pro
    2510                2515                2520

Ala Leu Arg Ser Thr Ala Thr Thr Pro Thr Ala Thr Ser Phe Thr
    2525                2530                2535

Ala Ile Pro Ser Ser Ser Leu Gly Thr Thr Trp Thr Arg Leu Ser
    2540                2545                2550

Gln Thr Thr Thr Pro Thr Ala Thr Met Ser Thr Ala Thr Pro Ser
    2555                2560                2565

Ser Thr Pro Glu Thr Val His Thr Ser Thr Val Leu Thr Thr Thr
    2570                2575                2580

Ala Thr Thr Thr Gly Ala Thr Gly Ser Val Ala Thr Pro Ser Ser
    2585                2590                2595

Thr Pro Gly Thr Ala His Thr Thr Lys Val Leu Thr Thr Thr Thr
    2600                2605                2610

Thr Gly Phe Thr Ala Thr Pro Ser Ser Ser Pro Gly Thr Ala Arg
    2615                2620                2625

Thr Leu Pro Val Trp Ile Ser Thr Thr Thr Thr Pro Thr Thr Arg
    2630                2635                2640

Gly Ser Thr Val Thr Pro Ser Ser Ile Pro Gly Thr Thr His Thr
    2645                2650                2655

Pro Thr Val Leu Thr Thr Thr Thr Thr Thr Val Ala Thr Gly Ser
    2660                2665                2670

Met Ala Thr Pro Ser Ser Ser Thr Gln Thr Ser Gly Thr Pro Pro
    2675                2680                2685

Ser Leu Thr Thr Thr Ala Thr Thr Ile Thr Ala Thr Gly Ser Thr
    2690                2695                2700

Thr Asn Pro Ser Ser Thr Pro Gly Thr Thr Pro Ile Pro Pro Val
    2705                2710                2715

Leu Thr Thr Thr Ala Thr Thr Pro Ala Ala Thr Ser Ser Thr Val
    2720                2725                2730

Thr Pro Ser Ser Ala Leu Gly Thr Thr His Thr Pro Pro Val Pro
    2735                2740                2745

Asn Thr Thr Ala Thr Thr His Gly Arg Ser Leu Ser Pro Ser Ser
    2750                2755                2760

Pro His Thr Val Arg Thr Ala Trp Thr Ser Ala Thr Ser Gly Thr
    2765                2770                2775

Leu Gly Thr Thr His Ile Thr Glu Pro Ser Thr Gly Thr Ser His
    2780                2785                2790

Thr Pro Ala Ala Thr Thr Gly Thr Thr Gln His Ser Thr Pro Ala
    2795                2800                2805

Leu Ser Ser Pro His Pro Ser Ser Arg Thr Thr Glu Ser Pro Pro
    2810                2815                2820

Ser Pro Gly Thr Thr Thr Pro Gly His Thr Arg Ala Thr Ser Arg
    2825                2830                2835

Thr Thr Ala Thr Ala Thr Pro Ser Lys Thr Arg Thr Ser Thr Leu
    2840                2845                2850

Leu Pro Ser Ser Pro Thr Ser Ala Pro Ile Thr Thr Val Val Thr
    2855                2860                2865

Met Gly Cys Glu Pro Gln Cys Ala Trp Ser Glu Trp Leu Asp Tyr
    2870                2875                2880

Ser Tyr Pro Met Pro Gly Pro Ser Gly Gly Asp Phe Asp Thr Tyr
    2885                2890                2895

Ser Asn Ile Arg Ala Ala Gly Gly Ala Val Cys Glu Gln Pro Leu
    2900                2905                2910

Gly Leu Glu Cys Arg Ala Gln Ala Gln Pro Gly Val Pro Leu Arg
    2915                2920                2925

Glu Leu Gly Gln Val Val Glu Cys Ser Leu Asp Phe Gly Leu Val
    2930                2935                2940

Cys Arg Asn Arg Glu Gln Val Gly Lys Phe Lys Met Cys Phe Asn
    2945                2950                2955

Tyr Glu Ile Arg Val Phe Cys Cys Asn Tyr Gly His Cys Pro Ser
    2960                2965                2970

Thr Pro Ala Thr Ser Ser Thr Ala Thr Pro Ser Ser Thr Pro Gly
    2975                2980                2985

Thr Thr Trp Ile Leu Thr Glu Gln Thr Thr Ala Ala Thr Thr Thr
    2990                2995                3000

Ala Thr Thr Gly Ser Thr Ala Ile Pro Ser Ser Thr Pro Gly Thr
    3005                3010                3015

Ala Pro Pro Pro Lys Val Leu Thr Ser Thr Ala Thr Thr Pro Thr
    3020                3025                3030

Ala Thr Ser Ser Lys Ala Thr Ser Ser Ser Ser Pro Arg Thr Ala
    3035                3040                3045

Thr Thr Leu Pro Val Leu Thr Ser Thr Ala Thr Lys Ser Thr Ala
    3050                3055                3060

Thr Ser Phe Thr Pro Ile Pro Ser Phe Thr Leu Gly Thr Thr Gly
    3065                3070                3075

Thr Leu Pro Glu Gln Thr Thr Thr Pro Met Ala Thr Met Ser Thr
    3080                3085                3090

Ile His Pro Ser Ser Thr Pro Glu Thr Thr His Thr Ser Thr Val
    3095                3100                3105

Leu Thr Thr Lys Ala Thr Thr Thr Arg Ala Thr Ser Ser Met Ser
    3110                3115                3120

Thr Pro Ser Ser Thr Pro Gly Thr Thr Trp Ile Leu Thr Glu Leu
    3125                3130                3135

Thr Thr Ala Ala Thr Thr Thr Ala Ala Thr Gly Pro Thr Ala Thr
    3140                3145                3150

Pro Ser Ser Thr Pro Gly Thr Thr Trp Ile Leu Thr Glu Pro Ser
    3155                3160                3165

Thr Thr Ala Thr Val Thr Val Pro Thr Gly Ser Thr Ala Thr Ala
    3170                3175                3180

Ser Ser Thr Arg Ala Thr Ala Gly Thr Leu Lys Val Leu Thr Ser
    3185                3190                3195

Thr Ala Thr Thr Pro Thr Val Ile Ser Ser Arg Ala Thr Pro Ser
    3200                3205                3210

Ser Ser Pro Gly Thr Ala Thr Ala Leu Pro Ala Leu Arg Ser Thr
    3215                3220                3225

Ala Thr Thr Pro Thr Ala Thr Ser Val Thr Ala Ile Pro Ser Ser
    3230                3235                3240

Ser Leu Gly Thr Ala Trp Thr Arg Leu Ser Gln Thr Thr Thr Pro
    3245                3250                3255

Thr Ala Thr Met Ser Thr Ala Thr Pro Ser Ser Thr Pro Glu Thr
    3260                3265                3270

Val His Thr Ser Thr Val Leu Thr Thr Thr Thr Thr Thr Thr Arg
    3275                3280                3285

Ala Thr Gly Ser Val Ala Thr Pro Ser Ser Thr Pro Gly Thr Ala
    3290                3295                3300

His Thr Thr Lys Val Pro Thr Thr Thr Thr Thr Gly Phe Thr Ala
    3305                3310                3315

Thr Pro Ser Ser Ser Pro Gly Thr Ala Leu Thr Pro Pro Val Trp
    3320                3325                3330

Ile Ser Thr Thr Thr Thr Pro Thr Thr Arg Gly Ser Thr Val Thr
    3335                3340                3345

Pro Ser Ser Ile Pro Gly Thr Thr His Thr Ala Thr Val Leu Thr

3350                3355                3360

Thr Thr Thr Thr Thr Val Ala Thr Gly Ser Met Ala Thr Pro Ser
    3365                3370                3375

Ser Ser Thr Gln Thr Ser Gly Thr Pro Pro Ser Leu Thr Thr Thr
    3380                3385                3390

Ala Thr Thr Ile Thr Ala Thr Gly Ser Thr Thr Asn Pro Ser Ser
    3395                3400                3405

Thr Pro Gly Thr Thr Pro Ile Pro Pro Val Leu Thr Thr Thr Ala
    3410                3415                3420

Thr Thr Pro Ala Ala Thr Ser Ser Thr Val Thr Pro Ser Ser Ala
    3425                3430                3435

Leu Gly Thr Thr His Thr Pro Pro Val Pro Asn Thr Thr Ala Thr
    3440                3445                3450

Thr His Gly Arg Ser Leu Pro Pro Ser Ser Pro His Thr Val Arg
    3455                3460                3465

Thr Ala Trp Thr Ser Ala Thr Ser Gly Ile Leu Gly Thr Thr His
    3470                3475                3480

Ile Thr Glu Pro Ser Thr Val Thr Ser His Thr Pro Ala Ala Thr
    3485                3490                3495

Thr Ser Thr Thr Gln His Ser Thr Pro Ala Leu Ser Ser Pro His
    3500                3505                3510

Pro Ser Ser Arg Thr Thr Glu Ser Pro Pro Ser Pro Gly Thr Thr
    3515                3520                3525

Thr Pro Gly His Thr Arg Gly Thr Ser Arg Thr Thr Ala Thr Ala
    3530                3535                3540

Thr Pro Ser Lys Thr Arg Thr Ser Thr Leu Leu Pro Ser Ser Pro
    3545                3550                3555

Thr Ser Ala Pro Ile Thr Thr Val Val Thr Thr Gly Cys Glu Pro
    3560                3565                3570

Gln Cys Ala Trp Ser Glu Trp Leu Asp Tyr Ser Tyr Pro Met Pro
    3575                3580                3585

Gly Pro Ser Gly Gly Asp Phe Asp Thr Tyr Ser Asn Ile Arg Ala
    3590                3595                3600

Ala Gly Gly Ala Val Cys Glu Gln Pro Leu Gly Leu Glu Cys Arg
    3605                3610                3615

Ala Gln Ala Gln Pro Gly Val Pro Leu Arg Glu Leu Gly Gln Val
    3620                3625                3630

Val Glu Cys Ser Leu Asp Phe Gly Leu Val Cys Arg Asn Arg Glu
    3635                3640                3645

Gln Val Gly Lys Phe Lys Met Cys Phe Asn Tyr Glu Ile Arg Val
    3650                3655                3660

Phe Cys Cys Asn Tyr Gly His Cys Pro Ser Thr Pro Ala Thr Ser
    3665                3670                3675

Ser Thr Ala Thr Pro Ser Ser Thr Pro Gly Thr Thr Trp Ile Leu
    3680                3685                3690

Thr Lys Leu Thr Thr Thr Ala Thr Thr Thr Glu Ser Thr Gly Ser
    3695                3700                3705

Thr Ala Thr Pro Ser Ser Thr Pro Gly Thr Thr Trp Ile Leu Thr
    3710                3715                3720

Glu Pro Ser Thr Thr Ala Thr Val Thr Val Pro Thr Gly Ser Thr
    3725                3730                3735

Ala Thr Ala Ser Ser Thr Gln Ala Thr Ala Gly Thr Pro His Val
    3740                3745                3750

Ser Thr Thr Ala Thr Thr Pro Thr Val Thr Ser Ser Lys Ala Thr
    3755                3760                3765

Pro Phe Ser Ser Pro Gly Thr Ala Thr Ala Leu Pro Ala Leu Arg
    3770                3775                3780

Ser Thr Ala Thr Thr Pro Thr Ala Thr Ser Phe Thr Ala Ile Pro
    3785                3790                3795

Ser Ser Ser Leu Gly Thr Thr Trp Thr Arg Leu Ser Gln Thr Thr
    3800                3805                3810

Thr Pro Thr Ala Thr Met Ser Thr Ala Thr Pro Ser Ser Thr Pro
    3815                3820                3825

Glu Thr Ala His Thr Ser Thr Val Leu Thr Thr Thr Ala Thr Thr
    3830                3835                3840

Thr Arg Ala Thr Gly Ser Val Ala Thr Pro Ser Ser Thr Pro Gly
    3845                3850                3855

Thr Ala His Thr Thr Lys Val Pro Thr Thr Thr Thr Thr Gly Phe
    3860                3865                3870

Thr Val Thr Pro Ser Ser Ser Pro Gly Thr Ala Arg Thr Pro Pro
    3875                3880                3885

Val Trp Ile Ser Thr Thr Thr Thr Pro Thr Thr Ser Gly Ser Thr
    3890                3895                3900

Val Thr Pro Ser Ser Val Pro Gly Thr Thr His Thr Pro Thr Val
    3905                3910                3915

Leu Thr Thr Thr Thr Thr Thr Val Ala Thr Gly Ser Met Ala Thr
    3920                3925                3930

Pro Ser Ser Ser Thr Gln Thr Ser Gly Thr Pro Pro Ser Leu Ile
    3935                3940                3945

Thr Thr Ala Thr Thr Ile Thr Ala Thr Gly Ser Thr Thr Asn Pro
    3950                3955                3960

Ser Ser Thr Pro Gly Thr Thr Pro Ile Pro Pro Val Leu Thr Thr
    3965                3970                3975

Thr Ala Thr Thr Pro Ala Ala Thr Ser Ser Thr Val Thr Pro Ser
    3980                3985                3990

Ser Ala Leu Gly Thr Thr His Thr Pro Pro Val Pro Asn Thr Thr
    3995                4000                4005

Ala Thr Thr His Gly Arg Ser Leu Ser Pro Ser Ser Pro His Thr
    4010                4015                4020

Val Arg Thr Ala Trp Thr Ser Ala Thr Ser Gly Thr Leu Gly Thr
    4025                4030                4035

Thr His Ile Thr Glu Pro Ser Thr Gly Thr Ser His Thr Pro Ala
    4040                4045                4050

Ala Thr Thr Gly Thr Thr Gln His Ser Thr Pro Ala Leu Ser Ser
    4055                4060                4065

Pro His Pro Ser Ser Arg Thr Thr Glu Ser Pro Pro Ser Pro Gly
    4070                4075                4080

Thr Thr Thr Pro Gly His Thr Thr Ala Thr Ser Arg Thr Thr Ala
    4085                4090                4095

Thr Ala Thr Pro Ser Lys Thr Arg Thr Ser Thr Leu Leu Pro Ser
    4100                4105                4110

Ser Pro Thr Ser Ala Pro Ile Thr Thr Val Val Thr Thr Gly Cys
    4115                4120                4125

Glu Pro Gln Cys Ala Trp Ser Glu Trp Leu Asp Tyr Ser Tyr Pro
    4130                4135                4140

Met Pro Gly Pro Ser Gly Gly Asp Phe Asp Thr Tyr Ser Asn Ile
    4145                4150                4155

Arg Ala Ala Gly Gly Ala Val Cys Glu Gln Pro Leu Gly Leu Glu
    4160                4165                4170

Cys Arg Ala Gln Ala Gln Pro Gly Val Pro Leu Gly Glu Leu Gly
    4175                4180                4185

Gln Val Val Glu Cys Ser Leu Asp Phe Gly Leu Val Cys Arg Asn
    4190                4195                4200

Arg Glu Gln Val Gly Lys Phe Lys Met Cys Phe Asn Tyr Glu Ile
    4205                4210                4215

Arg Val Phe Cys Cys Asn Tyr Gly His Cys Pro Ser Thr Pro Ala
    4220                4225                4230

Thr Ser Ser Thr Ala Met Pro Ser Ser Thr Pro Gly Thr Thr Trp
    4235                4240                4245

Ile Leu Thr Glu Leu Thr Thr Thr Ala Thr Thr Thr Ala Ser Thr
    4250                4255                4260

Gly Ser Thr Ala Thr Pro Ser Ser Thr Pro Gly Thr Ala Pro Pro
    4265                4270                4275

Pro Lys Val Leu Thr Ser Pro Ala Thr Thr Pro Thr Ala Thr Ser
    4280                4285                4290

Ser Lys Ala Thr Ser Ser Ser Ser Pro Arg Thr Ala Thr Thr Leu

4295                4300                4305

Pro Val Leu Thr Ser Thr Ala Thr Lys Ser Thr Ala Thr Ser Val
    4310                4315                4320

Thr Pro Ile Pro Ser Ser Thr Leu Gly Thr Thr Gly Thr Leu Pro
    4325                4330                4335

Glu Gln Thr Thr Thr Pro Val Ala Thr Met Ser Thr Ile His Pro
    4340                4345                4350

Ser Ser Thr Pro Glu Thr Thr His Thr Ser Thr Val Leu Thr Thr
    4355                4360                4365

Lys Ala Thr Thr Thr Arg Ala Thr Ser Ser Thr Ser Thr Pro Ser
    4370                4375                4380

Ser Thr Pro Gly Thr Thr Trp Ile Leu Thr Glu Leu Thr Thr Ala
    4385                4390                4395

Ala Thr Thr Thr Ala Ala Thr Gly Pro Thr Ala Thr Pro Ser Ser
    4400                4405                4410

Thr Pro Gly Thr Thr Trp Ile Leu Thr Glu Leu Thr Thr Thr Ala
    4415                4420                4425

Thr Thr Thr Ala Ser Thr Gly Ser Thr Ala Thr Pro Ser Ser Thr
    4430                4435                4440

Pro Gly Thr Thr Trp Ile Leu Thr Glu Pro Ser Thr Thr Ala Thr
    4445                4450                4455

Val Thr Val Pro Thr Gly Ser Thr Ala Thr Ala Ser Ser Thr Gln
    4460                4465                4470

Ala Thr Ala Gly Thr Pro His Val Ser Thr Thr Ala Thr Thr Pro
    4475                4480                4485

Thr Val Thr Ser Ser Lys Ala Thr Pro Ser Ser Ser Pro Gly Thr
    4490                4495                4500

Ala Thr Ala Leu Pro Ala Leu Arg Ser Thr Ala Thr Thr Pro Thr
    4505                4510                4515

Ala Thr Ser Phe Thr Ala Ile Pro Ser Ser Ser Leu Gly Thr Thr
    4520                4525                4530

Trp Thr Arg Leu Ser Gln Thr Thr Thr Pro Thr Ala Thr Met Ser
    4535                4540                4545

Thr Ala Thr Pro Ser Ser Thr Pro Glu Thr Val His Thr Ser Thr
    4550                4555                4560

Val Leu Thr Ala Thr Ala Thr Thr Thr Gly Ala Thr Gly Ser Val
    4565                4570                4575

Ala Thr Pro Ser Ser Thr Pro Gly Thr Ala His Thr Thr Lys Val
    4580                4585                4590

Pro Thr Thr Thr Thr Thr Gly Phe Thr Ala Thr Pro Ser Ser Ser
    4595                4600                4605

Pro Gly Thr Ala Leu Thr Pro Pro Val Trp Ile Ser Thr Thr Thr
    4610                4615                4620

Thr Pro Thr Thr Thr Thr Pro Thr Thr Ser Gly Ser Thr Val Thr
    4625                4630                4635

Pro Ser Ser Ile Pro Gly Thr Thr His Thr Ala Arg Val Leu Thr
    4640                4645                4650

Thr Thr Thr Thr Thr Val Ala Thr Gly Ser Met Ala Thr Pro Ser
    4655                4660                4665

Ser Ser Thr Gln Thr Ser Gly Thr Pro Pro Ser Leu Thr Thr Thr
    4670                4675                4680

Ala Thr Thr Ile Thr Ala Thr Gly Ser Thr Thr Asn Pro Ser Ser
    4685                4690                4695

Thr Pro Gly Thr Thr Pro Ile Thr Pro Val Leu Thr Ser Thr Ala
    4700                4705                4710

Thr Thr Pro Ala Ala Thr Ser Ser Lys Ala Thr Ser Ser Ser Ser
    4715                4720                4725

Pro Arg Thr Ala Thr Thr Leu Pro Val Leu Thr Ser Thr Ala Thr
    4730                4735                4740

Lys Ser Thr Ala Thr Ser Phe Thr Pro Ile Pro Ser Ser Thr Leu
    4745                4750                4755

Trp Thr Thr Trp Thr Val Pro Ala Gln Thr Thr Thr Pro Met Ser
    4760                4765                4770

Thr Met Ser Thr Ile His Thr Ser Ser Thr Pro Glu Thr Thr His
    4775                4780                4785

Thr Ser Thr Val Leu Thr Thr Thr Ala Thr Met Thr Arg Ala Thr
    4790                4795                4800

Asn Ser Thr Ala Thr Pro Ser Ser Thr Leu Gly Thr Thr Arg Ile
    4805                4810                4815

Leu Thr Glu Leu Thr Thr Thr Ala Thr Thr Thr Ala Ala Thr Gly
    4820                4825                4830

Ser Thr Ala Thr Leu Ser Ser Thr Pro Gly Thr Thr Trp Ile Leu
    4835                4840                4845

Thr Glu Pro Ser Thr Ile Ala Thr Val Met Val Pro Thr Gly Ser
    4850                4855                4860

Thr Ala Thr Ala Ser Ser Thr Leu Gly Thr Ala His Thr Pro Lys
    4865                4870                4875

Val Val Thr Thr Met Ala Thr Met Pro Thr Ala Thr Ala Ser Thr
    4880                4885                4890

Val Pro Ser Ser Ser Thr Val Gly Thr Thr Arg Thr Pro Ala Val
    4895                4900                4905

Leu Pro Ser Ser Leu Pro Thr Phe Ser Val Ser Thr Val Ser Ser
    4910                4915                4920

Ser Val Leu Thr Thr Leu Arg Pro Thr Gly Phe Pro Ser Ser His
    4925                4930                4935

Phe Ser Thr Pro Cys Phe Cys Arg Ala Phe Gly Gln Phe Phe Ser
    4940                4945                4950

Pro Gly Glu Val Ile Tyr Asn Lys Thr Asp Arg Ala Gly Cys His
    4955                4960                4965

Phe Tyr Ala Val Cys Asn Gln His Cys Asp Ile Asp Arg Phe Gln
    4970                4975                4980

Gly Ala Cys Pro Thr Ser Pro Pro Pro Val Ser Ser Ala Pro Leu
    4985                4990                4995

Ser Ser Pro Ser Pro Ala Pro Gly Cys Asp Asn Ala Ile Pro Leu
5000                5005                5010

Arg Gln Val Asn Glu Thr Trp Thr Leu Glu Asn Cys Thr Val Ala
    5015                5020                5025

Arg Cys Val Gly Asp Asn Arg Val Val Leu Leu Asp Pro Lys Pro
    5030                5035                5040

Val Ala Asn Val Thr Cys Val Asn Lys His Leu Pro Ile Lys Val
    5045                5050                5055

Ser Asp Pro Ser Gln Pro Cys Asp Phe His Tyr Glu Cys Glu Cys
    5060                5065                5070

Ile Cys Ser Met Trp Gly Gly Ser His Tyr Ser Thr Phe Asp Gly
    5075                5080                5085

Thr Ser Tyr Thr Phe Arg Gly Asn Cys Thr Tyr Val Leu Met Arg
    5090                5095                5100

Glu Ile His Ala Arg Phe Gly Asn Leu Ser Leu Tyr Leu Asp Asn
    5105                5110                5115

His Tyr Cys Thr Ala Ser Ala Thr Ala Ala Ala Ala Arg Cys Pro
    5120                5125                5130

Arg Ala Leu Ser Ile His Tyr Lys Ser Met Asp Ile Val Leu Thr
    5135                5140                5145

Val Thr Met Val His Gly Lys Glu Glu Gly Leu Ile Leu Phe Asp
    5150                5155                5160

Gln Ile Pro Val Ser Ser Gly Phe Ser Lys Asn Gly Val Leu Val
    5165                5170                5175

Ser Val Leu Gly Thr Thr Thr Met Arg Val Asp Ile Pro Ala Leu
    5180                5185                5190

Gly Val Ser Val Thr Phe Asn Gly Gln Val Phe Gln Ala Arg Leu
    5195                5200                5205

Pro Tyr Ser Leu Phe His Asn Asn Thr Glu Gly Gln Cys Gly Thr
    5210                5215                5220

Cys Thr Asn Asn Gln Arg Asp Asp Cys Leu Gln Arg Asp Gly Thr
    5225                5230                5235

Thr Ala Ala Ser Cys Lys Asp Met Ala Lys Thr Trp Leu Val Pro

5240                5245                5250

Asp Ser Arg Lys Asp Gly Cys Trp Ala Pro Thr Gly Thr Pro Pro
    5255                5260                5265

Thr Ala Ser Pro Ala Ala Pro Val Ser Ser Thr Pro Thr Pro Thr
    5270                5275                5280

Pro Cys Pro Pro Gln Pro Leu Cys Asp Leu Met Leu Ser Gln Val
    5285                5290                5295

Phe Ala Glu Cys His Asn Leu Val Pro Pro Gly Pro Phe Phe Asn
    5300                5305                5310

Ala Cys Ile Ser Asp His Cys Arg Gly Arg Leu Glu Val Pro Cys
    5315                5320                5325

Gln Ser Leu Glu Ala Tyr Ala Glu Leu Cys Arg Ala Arg Gly Val
    5330                5335                5340

Cys Ser Asp Trp Arg Gly Ala Thr Gly Gly Leu Cys Asp Leu Thr
    5345                5350                5355

Cys Pro Pro Thr Lys Val Tyr Lys Pro Cys Gly Pro Ile Gln Pro
    5360                5365                5370

Ala Thr Cys Asn Ser Arg Asn Gln Ser Pro Gln Leu Glu Gly Met
    5375                5380                5385

Ala Glu Gly Cys Phe Cys Pro Glu Asp Gln Ile Leu Phe Asn Ala
    5390                5395                5400

His Met Gly Ile Cys Val Gln Ala Cys Pro Cys Val Gly Pro Asp
    5405                5410                5415

Gly Phe Pro Lys Phe Pro Gly Glu Arg Trp Val Ser Asn Cys Gln
    5420                5425                5430

Ser Cys Val Cys Asp Glu Gly Ser Val Ser Val Gln Cys Lys Pro
    5435                5440                5445

Leu Pro Cys Asp Ala Gln Gly Gln Pro Pro Pro Cys Asn Arg Pro
    5450                5455                5460

Gly Phe Val Thr Val Thr Arg Pro Arg Ala Glu Asn Pro Cys Cys
    5465                5470                5475

Pro Glu Thr Val Cys Val Cys Asn Thr Thr Thr Cys Pro Gln Ser
    5480                5485                5490

Leu Pro Val Cys Pro Pro Gly Gln Glu Ser Ile Cys Thr Gln Glu
    5495                5500                5505

Glu Gly Asp Cys Cys Pro Thr Phe Arg Cys Arg Pro Gln Leu Cys
    5510                5515                5520

Ser Tyr Asn Gly Thr Phe Tyr Gly Val Gly Ala Thr Phe Pro Gly
    5525                5530                5535

Ala Leu Pro Cys His Met Cys Thr Cys Leu Ser Gly Asp Thr Gln
    5540                5545                5550

Asp Pro Thr Val Gln Cys Gln Glu Asp Ala Cys Asn Asn Thr Thr
    5555                5560                5565

Cys Pro Gln Gly Phe Glu Tyr Lys Arg Val Ala Gly Gln Cys Cys
    5570                5575                5580

Gly Glu Cys Val Gln Thr Ala Cys Leu Thr Pro Asp Gly Gln Pro
    5585                5590                5595

Val Gln Leu Asn Glu Thr Trp Val Asn Ser His Val Asp Asn Cys
    5600                5605                5610

Thr Val Tyr Leu Cys Glu Ala Glu Gly Gly Val His Leu Leu Thr
    5615                5620                5625

Pro Gln Pro Ala Ser Cys Pro Asp Val Ser Ser Cys Arg Gly Ser
    5630                5635                5640

Leu Arg Lys Thr Gly Cys Cys Tyr Ser Cys Glu Glu Asp Ser Cys
    5645                5650                5655

Gln Val Arg Ile Asn Thr Thr Ile Leu Trp His Gln Gly Cys Glu
    5660                5665                5670

Thr Glu Val Asn Ile Thr Phe Cys Glu Gly Ser Cys Pro Gly Ala
    5675                5680                5685

Ser Lys Tyr Ser Ala Glu Ala Gln Ala Met Gln His Gln Cys Thr
    5690                5695                5700

Cys Cys Gln Glu Arg Arg Val His Glu Glu Thr Val Pro Leu His
    5705                5710                5715

Cys Pro Asn Gly Ser Ala Ile Leu His Thr Tyr Thr His Val Asp
    5720                5725                5730

Glu Cys Gly Cys Thr Pro Phe Cys Val Pro Ala Pro Met Ala Pro
    5735                5740                5745

Pro His Thr Arg Gly Phe Pro Ala Gln Glu Ala Thr Ala Val
    5750                5755                5760

<210> 15
<211> 217
<212> PRT
<213> Homo sapiens

<400> 15

<210>  16
<211>  320
<212>  PRT
<213>  Homo sapiens

<400>  16

Met Ala Gln Val Leu Arg Gly Thr Val Thr Asp Phe Pro Gly Phe Asp
1               5                   10                  15

Glu Arg Ala Asp Ala Glu Thr Leu Arg Lys Ala Met Lys Gly Leu Gly
            20                  25                  30

Thr Asp Glu Glu Ser Ile Leu Thr Leu Leu Thr Ser Arg Ser Asn Ala
        35                  40                  45

Gln Arg Gln Glu Ile Ser Ala Ala Phe Lys Thr Leu Phe Gly Arg Asp
    50                  55                  60

Leu Leu Asp Asp Leu Lys Ser Glu Leu Thr Gly Lys Phe Glu Lys Leu
65                  70                  75                  80

Ile Val Ala Leu Met Lys Pro Ser Arg Leu Tyr Asp Ala Tyr Glu Leu
                85                  90                  95

Lys His Ala Leu Lys Gly Ala Gly Thr Asn Glu Lys Val Leu Thr Glu
            100                 105                 110

Ile Ile Ala Ser Arg Thr Pro Glu Glu Leu Arg Ala Ile Lys Gln Val
        115                 120                 125

Tyr Glu Glu Glu Tyr Gly Ser Ser Leu Glu Asp Asp Val Val Gly Asp
    130                 135                 140

Thr Ser Gly Tyr Tyr Gln Arg Met Leu Val Val Leu Leu Gln Ala Asn
145                 150                 155                 160

Arg Asp Pro Asp Ala Gly Ile Asp Glu Ala Gln Val Glu Gln Asp Ala
                165                 170                 175

Gln Ala Leu Phe Gln Ala Gly Glu Leu Lys Trp Gly Thr Asp Glu Glu
            180                 185                 190

Lys Phe Ile Thr Ile Phe Gly Thr Arg Ser Val Ser His Leu Arg Lys
        195                 200                 205

Val Phe Asp Lys Tyr Met Thr Ile Ser Gly Phe Gln Ile Glu Glu Thr
    210                 215                 220

Ile Asp Arg Glu Thr Ser Gly Asn Leu Glu Gln Leu Leu Leu Ala Val
225                 230                 235                 240

Val Lys Ser Ile Arg Ser Ile Pro Ala Tyr Leu Ala Glu Thr Leu Tyr
                245                 250                 255

Tyr Ala Met Lys Gly Ala Gly Thr Asp Asp His Thr Leu Ile Arg Val
            260                 265                 270

Met Val Ser Arg Ser Glu Ile Asp Leu Phe Asn Ile Arg Lys Glu Phe
        275                 280                 285

Arg Lys Asn Phe Ala Thr Ser Leu Tyr Ser Met Ile Lys Gly Asp Thr
    290                 295                 300

Ser Gly Asp Tyr Lys Lys Ala Leu Leu Leu Leu Cys Gly Glu Asp Asp
305                 310                 315                 320

<210> 17
<211> 510
<212> PRT
<213> Homo sapiens

<400> 17

Met Arg Pro Gly Leu Ser Phe Leu Leu Ala Leu Leu Phe Phe Leu Gly
1               5                   10                  15

Gln Ala Ala Gly Asp Leu Gly Asp Val Gly Pro Pro Ile Pro Ser Pro
            20                  25                  30

Gly Phe Ser Ser Phe Pro Gly Val Asp Ser Ser Ser Ser Phe Ser Ser
        35                  40                  45

Ser Ser Arg Ser Gly Ser Ser Ser Ser Arg Ser Leu Gly Ser Gly Gly
    50                  55                  60

Ser Val Ser Gln Leu Phe Ser Asn Phe Thr Gly Ser Val Asp Asp Arg
65                  70                  75                  80

Gly Thr Cys Gln Cys Ser Val Ser Leu Pro Asp Thr Thr Phe Pro Val
                85                  90                  95

Asp Arg Val Glu Arg Leu Glu Phe Thr Ala His Val Leu Ser Gln Lys
            100                 105                 110

Phe Glu Lys Glu Leu Ser Lys Val Arg Glu Tyr Val Gln Leu Ile Ser
        115                 120                 125

Val Tyr Glu Lys Lys Leu Leu Asn Leu Thr Val Arg Ile Asp Ile Met
    130                 135                 140

Glu Lys Asp Thr Ile Ser Tyr Thr Glu Leu Asp Phe Glu Leu Ile Lys
145                 150                 155                 160

Val Glu Val Lys Glu Met Glu Lys Leu Val Ile Gln Leu Lys Glu Ser
                165                 170                 175

Phe Gly Gly Ser Ser Glu Ile Val Asp Gln Leu Glu Val Glu Ile Arg
            180                 185                 190

Asn Met Thr Leu Leu Val Glu Lys Leu Glu Thr Leu Asp Lys Asn Asn
        195                 200                 205

Val Leu Ala Ile Arg Arg Glu Ile Val Ala Leu Lys Thr Lys Leu Lys
    210                 215                 220

Glu Cys Glu Ala Ser Lys Asp Gln Asn Thr Pro Val Val His Pro Pro
225                 230                 235                 240

Pro Thr Pro Gly Ser Cys Gly His Gly Gly Val Val Asn Ile Ser Lys
                245                 250                 255

Pro Ser Val Val Gln Leu Asn Trp Arg Gly Phe Ser Tyr Leu Tyr Gly
            260                 265                 270

Ala Trp Gly Arg Asp Tyr Ser Pro Gln His Pro Asn Lys Gly Leu Tyr
        275                 280                 285

Trp Val Ala Pro Leu Asn Thr Asp Gly Arg Leu Leu Glu Tyr Tyr Arg
    290                 295                 300

Leu Tyr Asn Thr Leu Asp Asp Leu Leu Leu Tyr Ile Asn Ala Arg Glu
305                 310                 315                 320

Leu Arg Ile Thr Tyr Gly Gln Gly Ser Gly Thr Ala Val Tyr Asn Asn
                325                 330                 335

Asn Met Tyr Val Asn Met Tyr Asn Thr Gly Asn Ile Ala Arg Val Asn
                340                 345                 350

Leu Thr Thr Asn Thr Ile Ala Val Thr Gln Thr Leu Pro Asn Ala Ala
            355                 360                 365

Tyr Asn Asn Arg Phe Ser Tyr Ala Asn Val Ala Trp Gln Asp Ile Asp
        370                 375                 380

Phe Ala Val Asp Glu Asn Gly Leu Trp Val Ile Tyr Ser Thr Glu Ala
385                 390                 395                 400

Ser Thr Gly Asn Met Val Ile Ser Lys Leu Asn Asp Thr Thr Leu Gln
                405                 410                 415

Val Leu Asn Thr Trp Tyr Thr Lys Gln Tyr Lys Pro Ser Ala Ser Asn
            420                 425                 430

Ala Phe Met Val Cys Gly Val Leu Tyr Ala Thr Arg Thr Met Asn Thr
        435                 440                 445

Arg Thr Glu Glu Ile Phe Tyr Tyr Tyr Asp Thr Asn Thr Gly Lys Glu
    450                 455                 460

Gly Lys Leu Asp Ile Val Met His Lys Met Gln Glu Lys Val Gln Ser
465                 470                 475                 480

Ile Asn Tyr Asn Pro Phe Asp Gln Lys Leu Tyr Val Tyr Asn Asp Gly
                485                 490                 495

Tyr Leu Leu Asn Tyr Asp Leu Ser Val Leu Gln Lys Pro Gln
            500                 505                 510

<210>  18
<211>  541
<212>  PRT
<213>  Homo sapiens

<400>  18

Met Val Ala Asp Pro Pro Arg Asp Ser Lys Gly Leu Ala Ala Ala Glu
1               5                   10                  15

Pro Thr Ala Asn Gly Gly Leu Ala Leu Ala Ser Ile Glu Asp Gln Gly
            20                  25                  30

Ala Ala Ala Gly Gly Tyr Cys Gly Ser Arg Asp Gln Val Arg Arg Cys
        35                  40                  45

Leu Arg Ala Asn Leu Leu Val Leu Leu Thr Val Val Ala Val Val Ala
    50                  55                  60

Gly Val Ala Leu Gly Leu Gly Val Ser Gly Ala Gly Gly Ala Leu Ala
65                  70                  75                  80

Leu Gly Pro Glu Arg Leu Ser Ala Phe Val Phe Pro Gly Glu Leu Leu
                85                  90                  95

Leu Arg Leu Leu Arg Met Ile Ile Leu Pro Leu Val Val Cys Ser Leu
            100                 105                 110

Ile Gly Gly Ala Ala Ser Leu Asp Pro Gly Ala Leu Gly Arg Leu Gly
        115                 120                 125

Ala Trp Ala Leu Leu Phe Phe Leu Val Thr Thr Leu Leu Ala Ser Ala
    130                 135                 140

Leu Gly Val Gly Leu Ala Leu Ala Leu Gln Pro Gly Ala Ala Ser Ala
145                 150                 155                 160

Ala Ile Asn Ala Ser Val Gly Ala Ala Gly Ser Ala Glu Asn Ala Pro
                165                 170                 175

Ser Lys Glu Val Leu Asp Ser Phe Leu Asp Leu Ala Arg Asn Ile Phe
            180                 185                 190

Pro Ser Asn Leu Val Ser Ala Ala Phe Arg Ser Tyr Ser Thr Thr Tyr
        195                 200                 205

Glu Glu Arg Asn Ile Thr Gly Thr Arg Val Lys Val Pro Val Gly Gln
    210                 215                 220

Glu Val Glu Gly Met Asn Ile Leu Gly Leu Val Val Phe Ala Ile Val
225                 230                 235                 240

Phe Gly Val Ala Leu Arg Lys Leu Gly Pro Glu Gly Glu Leu Leu Ile
                245                 250                 255

Arg Phe Phe Asn Ser Phe Asn Glu Ala Thr Met Val Leu Val Ser Trp
            260                 265                 270

Ile Met Trp Tyr Ala Pro Val Gly Ile Met Phe Leu Val Ala Gly Lys
        275                 280                 285

Ile Val Glu Met Glu Asp Val Gly Leu Leu Phe Ala Arg Leu Gly Lys

290                 295                 300

Tyr Ile Leu Cys Cys Leu Leu Gly His Ala Ile His Gly Leu Leu Val
305                 310                 315                 320

Leu Pro Leu Ile Tyr Phe Leu Phe Thr Arg Lys Asn Pro Tyr Arg Phe
                325                 330                 335

Leu Trp Gly Ile Val Thr Pro Leu Ala Thr Ala Phe Gly Thr Ser Ser
            340                 345                 350

Ser Ser Ala Thr Leu Pro Leu Met Met Lys Cys Val Glu Glu Asn Asn
        355                 360                 365

Gly Val Ala Lys His Ile Ser Arg Phe Ile Leu Pro Ile Gly Ala Thr
    370                 375                 380

Val Asn Met Asp Gly Ala Ala Leu Phe Gln Cys Val Ala Ala Val Phe
385                 390                 395                 400

Ile Ala Gln Leu Ser Gln Gln Ser Leu Asp Phe Val Lys Ile Ile Thr
                405                 410                 415

Ile Leu Val Thr Ala Thr Ala Ser Ser Val Gly Ala Ala Gly Ile Pro
            420                 425                 430

Ala Gly Gly Val Leu Thr Leu Ala Ile Ile Leu Glu Ala Val Asn Leu
        435                 440                 445

Pro Val Asp His Ile Ser Leu Ile Leu Ala Val Asp Trp Leu Val Asp
    450                 455                 460

Arg Ser Cys Thr Val Leu Asn Val Glu Gly Asp Ala Leu Gly Ala Gly
465                 470                 475                 480

Leu Leu Gln Asn Tyr Val Asp Arg Thr Glu Ser Arg Ser Thr Glu Pro
                485                 490                 495

Glu Leu Ile Gln Val Lys Ser Glu Leu Pro Leu Asp Pro Leu Pro Val
            500                 505                 510

Pro Thr Glu Glu Gly Asn Pro Leu Leu Lys His Tyr Arg Gly Pro Ala
        515                 520                 525

Gly Asp Ala Thr Val Ala Ser Glu Lys Glu Ser Val Met
    530                 535                 540

<210>  19
<211>  563
<212>  PRT
<213>  Homo sapiens

<400>  19

<210>  20
<211>  639
<212>  PRT
<213>  Homo sapiens

<400>  20

Met Ser Ala Arg Gly Pro Ala Ile Gly Ile Asp Leu Gly Thr Thr Tyr
1               5                   10                  15

Ser Cys Val Gly Val Phe Gln His Gly Lys Val Glu Ile Ile Ala Asn
            20                  25                  30

Asp Gln Gly Asn Arg Thr Thr Pro Ser Tyr Val Ala Phe Thr Asp Thr
        35                  40                  45

Glu Arg Leu Ile Gly Asp Ala Ala Lys Asn Gln Val Ala Met Asn Pro
    50                  55                  60

Thr Asn Thr Ile Phe Asp Ala Lys Arg Leu Ile Gly Arg Lys Phe Glu
65                  70                  75                  80

Asp Ala Thr Val Gln Ser Asp Met Lys His Trp Pro Phe Arg Val Val
                85                  90                  95

Ser Glu Gly Gly Lys Pro Lys Val Gln Val Glu Tyr Lys Gly Glu Thr
            100                 105                 110

Lys Thr Phe Phe Pro Glu Glu Ile Ser Ser Met Val Leu Thr Lys Met
        115                 120                 125

Lys Glu Ile Ala Glu Ala Tyr Leu Gly Gly Lys Val His Ser Ala Val
    130                 135                 140

Ile Thr Val Pro Ala Tyr Phe Asn Asp Ser Gln Arg Gln Ala Thr Lys
145                 150                 155                 160

Asp Ala Gly Thr Ile Thr Gly Leu Asn Val Leu Arg Ile Ile Asn Glu
                165                 170                 175

Pro Thr Ala Ala Ala Ile Ala Tyr Gly Leu Asp Lys Lys Gly Cys Ala
            180                 185                 190

Gly Gly Glu Lys Asn Val Leu Ile Phe Asp Leu Gly Gly Gly Thr Phe
        195                 200                 205

Asp Val Ser Ile Leu Thr Ile Glu Asp Gly Ile Phe Glu Val Lys Ser
    210                 215                 220

Thr Ala Gly Asp Thr His Leu Gly Gly Glu Asp Phe Asp Asn Arg Met
225                 230                 235                 240

Val Ser His Leu Ala Glu Glu Phe Lys Arg Lys His Lys Lys Asp Ile
                245                 250                 255

Gly Pro Asn Lys Arg Ala Val Arg Arg Leu Arg Thr Ala Cys Glu Arg
            260                 265                 270

Ala Lys Arg Thr Leu Ser Ser Ser Thr Gln Ala Ser Ile Glu Ile Asp
        275                 280                 285

Ser Leu Tyr Glu Gly Val Asp Phe Tyr Thr Ser Ile Thr Arg Ala Arg
    290                 295                 300

Phe Glu Glu Leu Asn Ala Asp Leu Phe Arg Gly Thr Leu Glu Pro Val
305                 310                 315                 320

Glu Lys Ala Leu Arg Asp Ala Lys Leu Asp Lys Gly Gln Ile Gln Glu
                325                 330                 335

Ile Val Leu Val Gly Gly Ser Thr Arg Ile Pro Lys Ile Gln Lys Leu
            340                 345                 350

Leu Gln Asp Phe Phe Asn Gly Lys Glu Leu Asn Lys Ser Ile Asn Pro
        355                 360                 365

Asp Glu Ala Val Ala Tyr Gly Ala Ala Val Gln Ala Ala Ile Leu Ile
    370                 375                 380

Gly Asp Lys Ser Glu Asn Val Gln Asp Leu Leu Leu Leu Asp Val Thr
385                 390                 395                 400

Pro Leu Ser Leu Gly Ile Glu Thr Ala Gly Gly Val Met Thr Pro Leu
                405                 410                 415

Ile Lys Arg Asn Thr Thr Ile Pro Thr Lys Gln Thr Gln Thr Phe Thr
            420                 425                 430

Thr Tyr Ser Asp Asn Gln Ser Ser Val Leu Val Gln Val Tyr Glu Gly
        435                 440                 445

Glu Arg Ala Met Thr Lys Asp Asn Asn Leu Leu Gly Lys Phe Asp Leu
    450                 455                 460

Thr Gly Ile Pro Pro Ala Pro Arg Gly Val Pro Gln Ile Glu Val Thr
465                 470                 475                 480

Phe Asp Ile Asp Ala Asn Gly Ile Leu Asn Val Thr Ala Ala Asp Lys
                485                 490                 495

Ser Thr Gly Lys Glu Asn Lys Ile Thr Ile Thr Asn Asp Lys Gly Arg
            500                 505                 510

Leu Ser Lys Asp Asp Ile Asp Arg Met Val Gln Glu Ala Glu Arg Tyr
        515                 520                 525

Lys Ser Glu Asp Glu Ala Asn Arg Asp Arg Val Ala Ala Lys Asn Ala
    530                 535                 540

Leu Glu Ser Tyr Thr Tyr Asn Ile Lys Gln Thr Val Glu Asp Glu Lys
545                 550                 555                 560

Leu Arg Gly Lys Ile Ser Glu Gln Asp Lys Asn Lys Ile Leu Asp Lys
                565                 570                 575

Cys Gln Glu Val Ile Asn Trp Leu Asp Arg Asn Gln Met Ala Glu Lys
            580                 585                 590

Asp Glu Tyr Glu His Lys Gln Lys Glu Leu Glu Arg Val Cys Asn Pro
        595                 600                 605

Ile Ile Ser Lys Leu Tyr Gln Gly Gly Pro Gly Gly Gly Ser Gly Gly
    610                 615                 620

Gly Gly Ser Gly Ala Ser Gly Gly Pro Thr Ile Glu Glu Val Asp

625                 630                 635

<210>  21
<211>  241
<212>  PRT
<213>  Homo sapiens

<400>  21

Met Phe Leu Thr Arg Ser Glu Tyr Asp Arg Gly Val Asn Thr Phe Ser
1               5                   10                  15

Pro Glu Gly Arg Leu Phe Gln Val Glu Tyr Ala Ile Glu Ala Ile Lys
            20                  25                  30

Leu Gly Ser Thr Ala Ile Gly Ile Gln Thr Ser Glu Gly Val Cys Leu
        35                  40                  45

Ala Val Glu Lys Arg Ile Thr Ser Pro Leu Met Glu Pro Ser Ser Ile
    50                  55                  60

Glu Lys Ile Val Glu Ile Asp Ala His Ile Gly Cys Ala Met Ser Gly
65                  70                  75                  80

Leu Ile Ala Asp Ala Lys Thr Leu Ile Asp Lys Ala Arg Val Glu Thr
                85                  90                  95

Gln Asn His Trp Phe Thr Tyr Asn Glu Thr Met Thr Val Glu Ser Val
            100                 105                 110

Thr Gln Ala Val Ser Asn Leu Ala Leu Gln Phe Gly Glu Glu Asp Ala
        115                 120                 125

Asp Pro Gly Ala Met Ser Arg Pro Phe Gly Val Ala Leu Leu Phe Gly
    130                 135                 140

Gly Val Asp Glu Lys Gly Pro Gln Leu Phe His Met Asp Pro Ser Gly
145                 150                 155                 160

Thr Phe Val Gln Cys Asp Ala Arg Ala Ile Gly Ser Ala Ser Glu Gly
                165                 170                 175

Ala Gln Ser Ser Leu Gln Glu Val Tyr His Lys Ser Met Thr Leu Lys
            180                 185                 190

Glu Ala Ile Lys Ser Ser Leu Ile Ile Leu Lys Gln Val Met Glu Glu
        195                 200                 205

Lys Leu Asn Ala Thr Asn Ile Glu Leu Ala Thr Val Gln Pro Gly Gln

210                 215                 220

Asn Phe His Met Phe Thr Lys Glu Glu Leu Glu Glu Val Ile Lys Asp
225                 230                 235                 240

Ile

<210>  22
<211>  198
<212>  PRT
<213>  Homo sapiens

<400>  22

Met Pro Leu Gly Leu Leu Trp Leu Gly Leu Ala Leu Leu Gly Ala Leu
1               5                   10                  15

His Ala Gln Ala Gln Asp Ser Thr Ser Asp Leu Ile Pro Ala Pro Pro
            20                  25                  30

Leu Ser Lys Val Pro Leu Gln Gln Asn Phe Gln Asp Asn Gln Phe Gln
        35                  40                  45

Gly Lys Trp Tyr Val Val Gly Leu Ala Gly Asn Ala Ile Leu Arg Glu
    50                  55                  60

Asp Lys Asp Pro Gln Lys Met Tyr Ala Thr Ile Tyr Glu Leu Lys Glu
65                  70                  75                  80

Asp Lys Ser Tyr Asn Val Thr Ser Val Leu Phe Arg Lys Lys Lys Cys
                85                  90                  95

Asp Tyr Trp Ile Arg Thr Phe Val Pro Gly Cys Gln Pro Gly Glu Phe
            100                 105                 110

Thr Leu Gly Asn Ile Lys Ser Tyr Pro Gly Leu Thr Ser Tyr Leu Val
        115                 120                 125

Arg Val Val Ser Thr Asn Tyr Asn Gln His Ala Met Val Phe Phe Lys
    130                 135                 140

Lys Val Ser Gln Asn Arg Glu Tyr Phe Lys Ile Thr Leu Tyr Gly Arg
145                 150                 155                 160

Thr Lys Glu Leu Thr Ser Glu Leu Lys Glu Asn Phe Ile Arg Phe Ser
                165                 170                 175

Lys Ser Leu Gly Leu Pro Glu Asn His Ile Val Phe Pro Val Pro Ile

                180                 185                 190

Asp Gln Cys Ile Asp Gly
        195



<210>  23
<211>  13
<212>  PRT
<213>  Artificial

<220>
<223>  Synthetic

<400>  23

Gly Thr Ile Thr Asp Ala Pro Gly Phe Asp Pro Leu Arg
1               5                   10



<210>  24
<211>  9
<212>  PRT
<213>  Artificial

<220>
<223>  Synthetic

<400>  24

Ser Glu Thr Asp Leu Leu Asp Ile Arg
1               5



<210>  25
<211>  16
<212>  PRT
<213>  Artificial

<220>
<223>  Synthetic

<400>  25

Gly Ala Gly Thr Asn Glu Asp Ala Leu Ile Glu Ile Leu Thr Thr Arg
1               5                   10                  15



<210>  26
<211>  13
<212>  PRT
<213>  Artificial

<220>
<223>  Synthetic

<400>  26

Ser Asp Thr Ser Gly Asp Tyr Glu Ile Thr Leu Leu Lys
1               5                   10

<210>  27
<211>  16
<212>  PRT
<213>  Artificial

<220>
<223>  Synthetic

<400>  27

Gly Ala Gly Thr Asp Glu Gly Cys Leu Ile Glu Ile Leu Ala Ser Arg
1               5                   10                  15


<210>  28
<211>  12
<212>  PRT
<213>  Artificial

<220>
<223>  Synthetic

<400>  28

Asp Glu Gly Asn Tyr Leu Asp Asp Ala Leu Val Arg
1               5                   10


<210>  29
<211>  8
<212>  PRT
<213>  Artificial

<220>
<223>  Synthetic

<400>  29

Glu Glu Gln Asn Ile Ile Phe Arg
1               5


<210>  30
<211>  9
<212>  PRT
<213>  Artificial

<220>
<223>  Synthetic

<400>  30

Ala Gln Thr Glu Ile Asp Gly Pro Lys
1               5


<210>  31
<211>  10
<212>  PRT
<213>  Artificial

<220>
<223>  Synthetic

<400>  31

Leu Leu Asn Glu Asn Ser Tyr Val Pro Arg
1               5                   10


<210>  32
<211>  13
<212>  PRT
<213>  Artificial

<220>
<223>  Synthetic

<400>  32

Val Ser Ala Ser Pro Leu Leu Tyr Thr Leu Ile Glu Lys
1               5                   10


<210>  33
<211>  8
<212>  PRT
<213>  Artificial

<220>
<223>  Synthetic

<400>  33

Val Thr Ile Leu Glu Leu Phe Arg
1               5


<210>  34
<211>  10
<212>  PRT
<213>  Artificial

<220>
<223>  Synthetic

<400>  34

Thr Phe Asp Glu Ile Ala Ser Gly Phe Arg
1               5                   10


<210>  35
<211>  11
<212>  PRT
<213>  Artificial

<220>
<223>  Synthetic

<400>  35

Leu Ser Pro Ile Tyr Asn Leu Val Pro Val Lys
1               5                   10

<210>  36
<211>  12
<212>  PRT
<213>  Artificial

<220>
<223>  Synthetic

<400>  36

Ala Ile Glu Asp Tyr Ile Asn Glu Phe Ser Val Arg
1               5                   10


<210>  37
<211>  7
<212>  PRT
<213>  Artificial

<220>
<223>  Synthetic

<400>  37

Ser Leu Pro Ser Leu Leu Arg
1               5


<210>  38
<211>  10
<212>  PRT
<213>  Artificial

<220>
<223>  Synthetic

<400>  38

Asn Val Leu Thr Glu Glu Ser Val Val Arg
1               5                   10


<210>  39
<211>  8
<212>  PRT
<213>  Artificial

<220>
<223>  Synthetic

<400>  39

Val Leu Glu Asp Ser Asp Leu Lys
1               5


<210>  40
<211>  14
<212>  PRT
<213>  Artificial

<220>
<223>  Synthetic

<400>  40

Ser Asp Ile Asp Glu Ile Val Leu Val Gly Gly Ser Thr Arg
1               5                   10


<210>  41
<211>  15
<212>  PRT
<213>  Artificial

<220>
<223>  Synthetic

<400>  41

Tyr Val Gly Gly Gln Glu His Phe Ala His Leu Leu Ile Leu Arg
1               5                   10                  15


<210>  42
<211>  14
<212>  PRT
<213>  Artificial

<220>
<223>  Synthetic

<400>  42

Glu Gln Leu Gly Glu Phe Tyr Glu Ala Leu Asp Cys Leu Arg
1               5                   10


<210>  43
<211>  12
<212>  PRT
<213>  Artificial

<220>
<223>  Synthetic

<400>  43

Gln Ser Thr Leu Val Leu Phe Pro Gly Asp Leu Arg
1               5                   10


<210>  44
<211>  9
<212>  PRT
<213>  Artificial

<220>
<223>  Synthetic

<400>  44

Phe Gln Thr Phe Glu Gly Asp Leu Lys
1               5

<210>  45
<211>  14
<212>  PRT
<213>  Artificial

<220>
<223>  Synthetic

<400>  45

Leu Glu Ile Gln Leu Leu Glu Asn Ser Leu Ser Thr Tyr Lys
1               5                   10


<210>  46
<211>  9
<212>  PRT
<213>  Artificial

<220>
<223>  Synthetic

<400>  46

Glu Asn Leu Gln Gly Leu Val Thr Arg
1               5


<210>  47
<211>  14
<212>  PRT
<213>  Artificial

<220>
<223>  Synthetic

<400>  47

Glu Val Leu Leu Leu Val His Asn Leu Pro Gln Asp Pro Arg
1               5                   10


<210>  48
<211>  9
<212>  PRT
<213>  Artificial

<220>
<223>  Synthetic

<400>  48

Leu Phe Ile Pro Asn Ile Thr Thr Lys
1               5


<210>  49
<211>  16
<212>  PRT
<213>  Artificial

<220>
<223>  Synthetic

<400>  49

Ala Ala Gly Gly Ala Val Cys Glu Gln Pro Leu Gly Leu Glu Cys Arg
1               5                   10                  15


<210>  50
<211>  19
<212>  PRT
<213>  Artificial

<220>
<223>  Synthetic

<400>  50

Val Cys Gly Leu Cys Gly Asn Phe Asp Asp Asn Ala Ile Asn Asp Phe
1               5                   10                  15


Ala Thr Arg



<210>  51
<211>  7
<212>  PRT
<213>  Artificial

<220>
<223>  Synthetic

<400>  51

Tyr Phe Leu Trp Val Val Lys
1               5


<210>  52
<211>  7
<212>  PRT
<213>  Artificial

<220>
<223>  Synthetic

<400>  52

Asn Gln Gln Ile Phe Leu Arg
1               5


<210>  53
<211>  9
<212>  PRT
<213>  Artificial

<220>
<223>  Synthetic

<400>  53

Ser Glu Ile Asp Leu Phe Asn Ile Arg
1               5

<210>  54
<211>  12
<212>  PRT
<213>  Artificial

<220>
<223>  Synthetic

<400>  54

Val Gln Ser Ile Asn Tyr Asn Pro Phe Asp Gln Lys
1               5                   10

<210>  55
<211>  12
<212>  PRT
<213>  Artificial

<220>
<223>  Synthetic

<400>  55

Gly Pro Ala Gly Asp Ala Thr Val Ala Ser Glu Lys
1               5                   10

<210>  56
<211>  14
<212>  PRT
<213>  Artificial

<220>
<223>  Synthetic

<400>  56

Leu Phe Gly Gly Asn Phe Ala His Gln Ala Ser Val Ala Arg
1               5                   10

<210>  57
<211>  11
<212>  PRT
<213>  Artificial

<220>
<223>  Synthetic

<400>  57

Asn Ala Leu Glu Ser Tyr Thr Tyr Asn Ile Lys
1               5                   10

<210>  58
<211>  14
<212>  PRT

<213>  Artificial

<220>
<223>  Synthetic

<400>  58

Pro Phe Gly Val Ala Leu Leu Phe Gly Gly Val Asp Glu Lys
1               5                   10


<210>  59
<211>  7
<212>  PRT
<213>  Artificial

<220>
<223>  Synthetic

<400>  59

Glu Leu Thr Ser Glu Leu Lys
1               5